# EUROPEAN PATENT APPLICATION

(11) **EP 2 020 404 A1**
(43) Date of publication of application: **04.02.2009**
(21) Application number: 07075662.2
(22) Date of filing: 01.08.2007
(51) Int. Cl.: C07D 209/16, C07D 209/14, C07D 401/12, C07D 405/12, C07D 409/12, A61K 31/4045, A61P 5/06, A61P 19/10

(54) **Cyanomethyl substituted N-Acyl Tryptamines**

(71) Applicant: Bayer Schering Pharma Aktiengesellschaft, 13353 Berlin (DE)
(72) Inventor: Wortmann, Lars, Dr., 10435 Berlin (DE); Koppitz, Marcus, Dr., 10115 Berlin (DE); Muhn, Peter, Dr., 13465 Berlin (DE); Frenzel, Thomas, Dr., 65719 Hofheim (Taunus) (DE); Liesener, Florian Peter, Dr., 83308 Trostberg (DE); Schrey, Anna Katharina, Dr., 10179 Berlin (DE); Kühne, Ronald, Dr., 12683 Berlin (DE)

(57) **Abstract**

The present invention relates to cyanomethyl substituted N-acyl tryptamines of the formula I 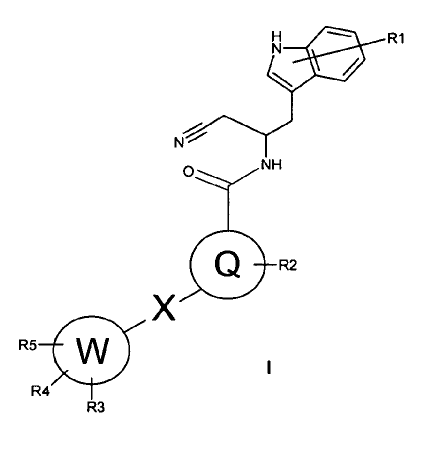 in which
R1, R2, R3, R4, R5, Q, X and W have the meaning as defined in the description.

The compounds according to the invention are effective FSH antagonists and can be used for example for fertility control in men or in women, or for the prevention and/or treatment of osteoporosis.

## Description

The present invention relates to novel cyanomethyl substituted N-acyl tryptamines with FSH-receptor antagonist activity. The present invention also relates to a process for their preparation, pharmaceutical compositions comprising the compounds according to the invention, and the use thereof for fertility control in men or women, for the treatment and/or prevention of osteoporosis.

Follicle-stimulating hormone (FSH) and luteinizing hormone (LH) are together responsible for the control of male and female fertility and of the production of sex steroids.

In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.

In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.

FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).

The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled person expects advantages from non-peptidergic FSH agonists when used to promote fertility in women (stimulation of follicle ripening). There are no reports of experience on the use of FSH or FSH agonists in male infertility, but specific indications are also conceivable in this connection.

New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).

FSH receptors have been detected in osteoclasts but not in osteoblasts. In vitro, FSH stimulates bone resorption by mouse osteoclasts ( Li Sun et al. Cell 2006; 125: 247-60). A clinical correlation between the serum FSH level and low bone density has been observed in postmenopausal women (Devleta et al, J. Bone Miner. Metab. 2004, 22: 360-4).

These findings among others suggest that FSH stimulates loss of bone mass, and consequently FSH antagonists will display an antiresorptive effect on bone and are therefore suitable for the therapy and/or prevention of peri- and postmenopausal loss of bone mass and osteoporosis.

FSH receptor modulators are compounds that have a mixed profile of both FSH receptor antagonistic and FSH receptor agonistic properties. FSH receptor modulators of various compound classes of low molecular weight, have been reported on recently. FSH receptor modulators are disclosed in WO 2004/056779, WO 2004/056780; J. Med. Chem. 2005, 48, 1697 [tetrahydroquinolines]; WO 02/70493, Bioorg. Med. Chem. Lett. 2004, 14, 1713 and 1717 [diketopiperazines]; and WO 01/47875 [sulphonamides]. FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d]pyrimidines)}; WO 01/87287 [pyrazoles]; WO 00/08015 [carbazoles]; WO 06/117023, WO 06/117368, WO 06/117370, WO 06/117371, [hexahydroquino-lines].

FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645, US 2006/0287522 [pyrrolobenzodiazepines], WO 2007/017289 [acyltryptophanols], EP06090223.6 [1,2-diarylacetylene derivatives of acyltryptophanols], EP06077263.9 [bicyclic acyltryptophanols], EP07090034.5 [sulfonyltryptophanols] and EP07090059.2 [tetrahydrocarbazoles].

WO 2007/017289 is considered to be the closest prior art.

In view of the prior art, the objective technical problem to be solved according to the present invention may therefore be seen in providing alternative compounds having a FSH receptor antagonistic activity.

The technical problem has been solved according to the present invention by the provision of novel compounds of the formula I 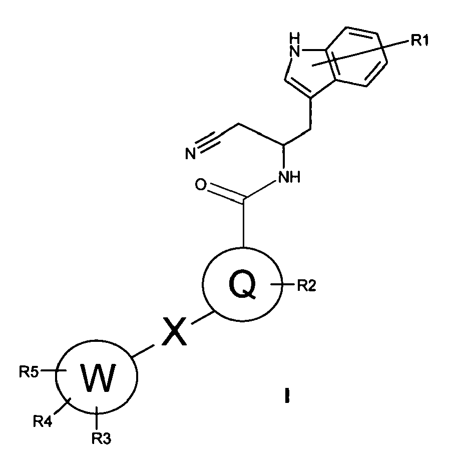 in which
- R1: is hydrogen, fluorine or C₁-C₆-alkyl; where the hydrocarbon chains therein may optionally be substi- tuted one or more times by fluorine;
- R2: is hydrogen, halogen, nitro, amino, cyano, C₁-C₆-alkyl, C₂-C₆-alkenyl or C₂-C₆-alkynyl, C₃-C₇-cydoalkyl, hydroxy-C₁-C₆-alkylene, hydroxy-C₃- C₆-alkenylene, hydroxy-C₃-C₆-alkynylene, C₁-C₆-alkyloxy, C₁-C₆- alkyloxy-C₁-C₆-alkylene, C₃-C₇-cycloalkyloxy, C₃-C₇-cycloalkyl-C₁-C₆- alkylenoxy, C₃-C₇-cycloalkyloxy-C₁-C₆-alkylene, C₁-C₆-alkyloxy-C₃-C₆- alkenylene, C₁-C₆-alkyloxy-C₃-C₆-alkynylene, C₁-C₆-alkylamino-C₁-C₆- alkylene, di(C₁-C₆-alkyl)amino-C₁-C₆-alkylene; where the hydrocarbon chains therein may optionally be substituted one or more times by fluorine, cyano or hydroxy;
- R3, R4, R5: may be independently of one another hydrogen, hydroxy, halogen, nitro, amino, cyano, phenyl, C₁-C₆-alkyl, C₂-C₆-alkenyl or C₂-C₆-alkynyl, C₃- C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the radicals: 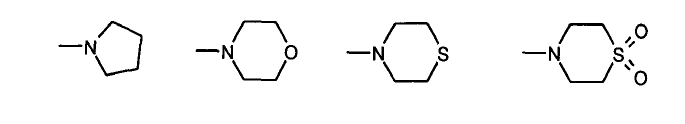 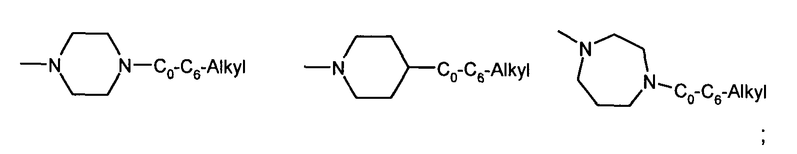 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cydoalkyl-C₁-C₆-alkyleneamino-carbonyl,
C₁-C₆-alkylcarbonyl, C₃-C₇-cycloalkylcarbonyl, carboxy, carboxamido [-C(O)NH₂], C₁-C₆-alkyloxycarbonyl,
C₁-C₃-alkylsulphanyl, C₁-C₆-alkysulphonyl, C₃-C₇cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido,
-N(C₀-C₆-alkyl)-C(O)-C₁-C₆-alkyl, -N(C₀-C₆-alkyl)-C(O)-C₃-C₇-cycloalkyl,
-N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇cycloalkyl,
-N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycoalkyl-C₁-C₆-alkyl)amine,
-S(O₂)-N(H)-C₂-C₆-alkylene-(C1-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
(C₁-C₆-alkyl)oxy-C₂-C₆-alkylene-aminocarbonyl, carboxy-C₁-C₆-alkylene-carbonylamido, (C₁-C₆-alkyl)sulfonylmido,
or the radicals: 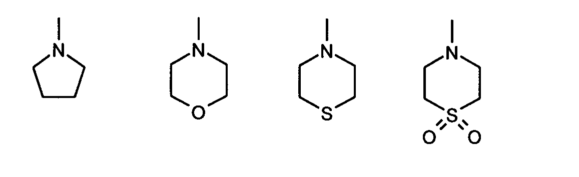 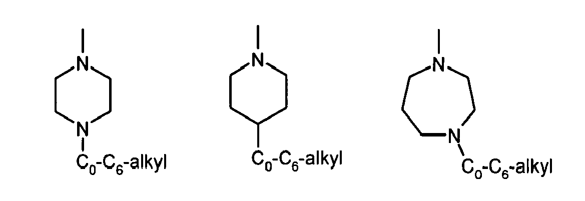 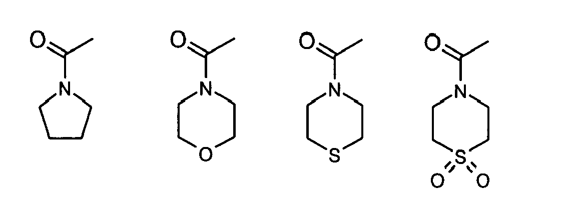 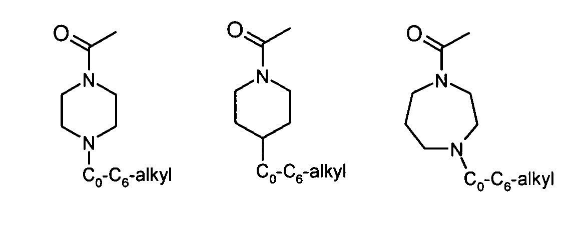 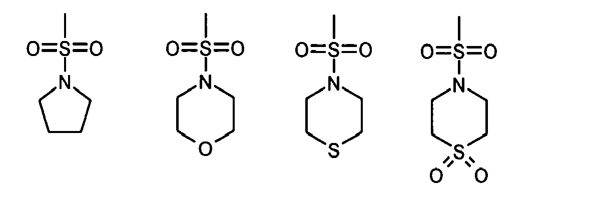 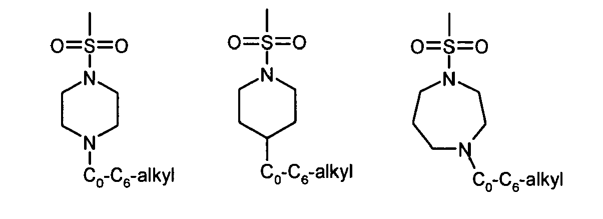 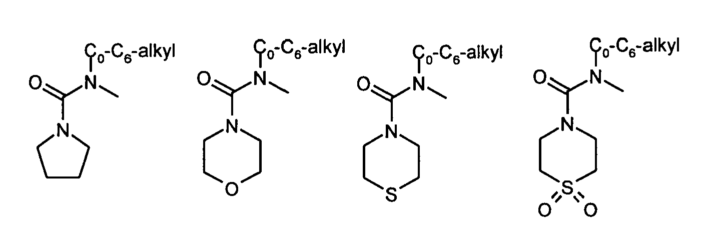 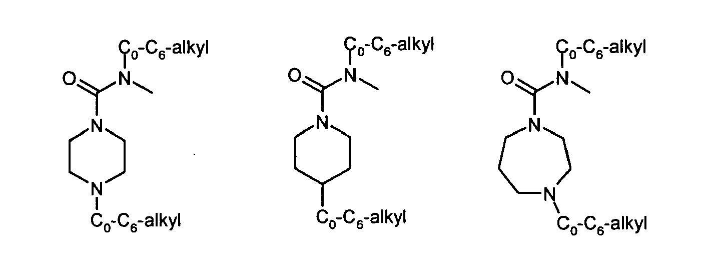 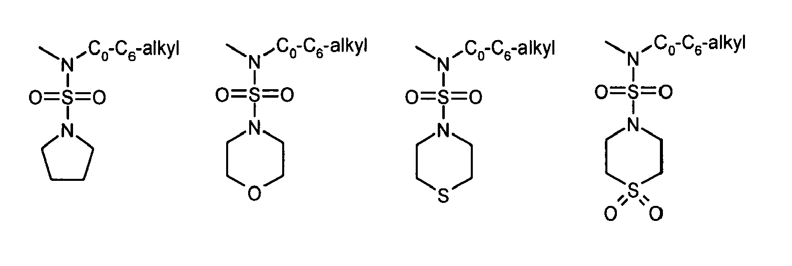 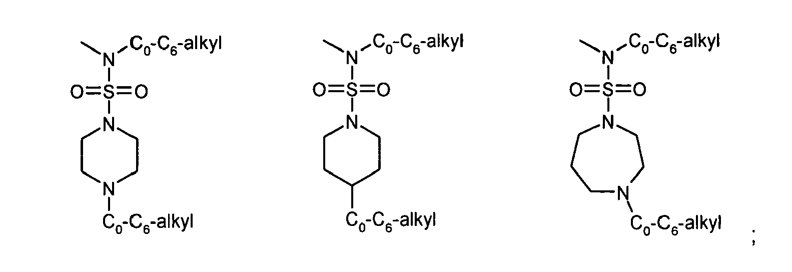
- R3 and R4: may together form heterocycloalkyl, cycloalkyl;
- Q: is an aryl or heteroaryl group or the group 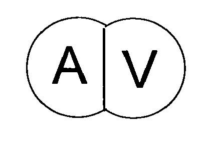 in which
A is a monocyclic aryl or a monocyclic heteroaryl group;
V is a cycloalkylen, cycloalkenylen, heterocycloalkylen or heterocycloalkenylen group;
- X: is a bond or an ethinylen group;
- W: is an aryl or heteroaryl group;
where
- R1: substitutes one or more positions of the aryl or heteroaryl ring in the indole residue;
- R2: substitutes one or more positions of the aryl or heteroaryl ring in the radical Q and or the radical V.

The present invention relates to both possible enantiomeric forms at the stereocentre of the tryptamine residue.

The unbranched C₁-C₆-alkyl groups for the radicals R1 to R5 may be for example a methyl, ethyl, propyl, butyl, pentyl or a hexyl group; and the branched C₃-C₆-alkyl groups for the radicals R1 to R6 may be an isopropyl, isobutyl, sec-butyl, *tert*-butyl, isopentyl, 2-methylbutyl, 1-methylbutyl, 1-ethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.

The branched or unbranched C₂-C₆-alkenyl groups for the radical R2 to R5 may be for example a vinyl, allyl, (*E*)-2-methylvinyl, (*Z*)-2-methylvinyl, homoallyl, (*E*)-but-2-enyl, (Z)-but-2-enyl, (*E*)-but-l-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methyl-pent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (Z)-2-methyl-pent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methyl-pent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethyl-but-2-enyl, (*E*)-2-ethylbut-2-enyl, (Z)-2-ethylbut-2-enyl, (*E*)-1-ethylbut-2-enyl, (*Z*)-1-ethyl-but-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropyl-prop-1-enyl, (*E*)-1-isopropylprop-1-enyl, (*Z*)-1-isopropylprop-1-enyl, (*E*)-3,3-dimethyl-prop-1-enyl, (Z)-3,3-dimethylprop-1-enyl- or a 1-(1,1-dimethylethyl)ethenyl group.

The C₃-C₆-alkynyl groups for the radicals R2 to R5 may be for example an ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

The C₁-C₆-alkyloxy groups for the radicals R2 to R5 may be for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, *ne*opentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy group.

The halogens for the radicals R1 to R5 are fluorine, chlorine, bromine or iodine.

The C₁-C₃-alkylsulphanyl groups for the radicals R3 to R5 may be for example a methylsulphanyl (CH₃S-), ethylsulphanyl (CH₃CH₂S-), propylsulphanyl, isopropyl-sulphanyl group.

The C₁-C₆-alkylaminocarbonyl groups for the radicals R3 to R5 may be for example a methylaminocarbonyl-, ethylaminocarbonyl-, propylaminocarbonyl-, isopropylaminocarbonyl-, butylaminocarbonyl-, isobutylaminocarbonyl-, sec-butylaminocarbonyl-, *tert-*butylaminocarbonyl-, pentylaminocarbonyl-, isopentylaminocarbonyl-, (2-methylbutyl)-aminocarbonyl-, (1-methylbutyl)aminocarbonyl-, (1-ethylpropyl)aminocarbonyl-, neo-*pent*ylaminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.

The hydroxy-C₁-C₆-alkylene groups for the radicals R3 to R5 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-1-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The heterocycloalkyl groups which may form the radicals R3 and R4 together may be for example the following groups:

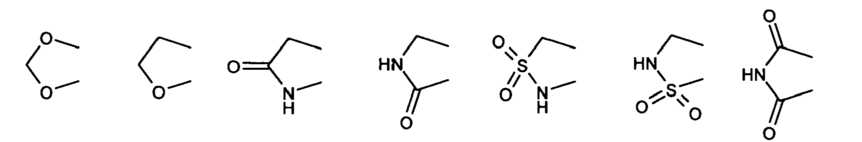

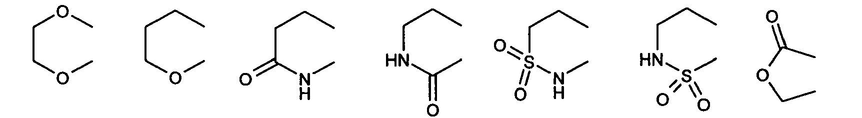

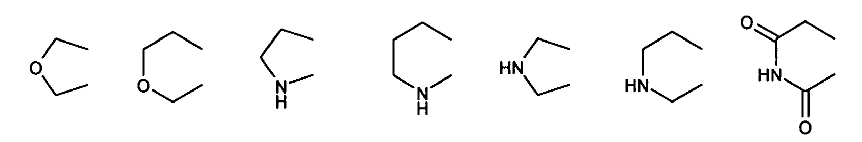

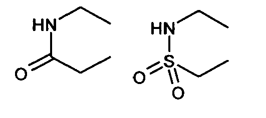

The cycloalkyl groups which may form the radicals R3 and R4 together may be for example the following groups:

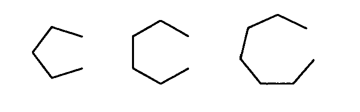

The C₃-C₇-cycloalkyl groups for the radicals R2 to R5 may be for example a cyclopropyl, cyclobutyl, cyclopentyl, cyclohexyl, cycloheptyl group.

The C₃-C₇-heterocycloalkyl groups for the radicals R1 to R5 may be for example a cyclopropyl, cyclobutyl, cycopentyl, cyclohexyl, cycloheptyl group in which one or two carbon atoms of the ring are replaced independently of one another by an oxygen, nitrogen or sulphur atom.

The monocyclic aryl group for A may be for example a phenyl group which is linked via substitutable positions.

The aryl group for W or Q may be for example a phenyl, naphthyl group which is linked via substitutable positions.

The monocylic heteroaryl group for A may be for example a pyridinyl, pyrimidinyl, furanyl, thienyl, oxazolyl, isoxazolyl, thiazolyl, pyrrolyl, pyrazolyl or an imidazolyl group which is linked via substitutable positions.

The heteroaryl group for W or Q may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzodioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.

The heterocycloalkylen groups for V may be for example the following groups:

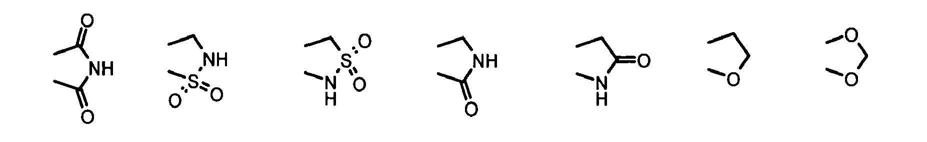

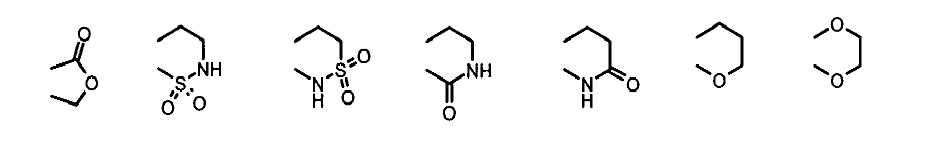

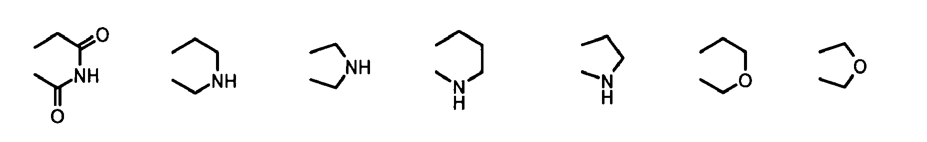

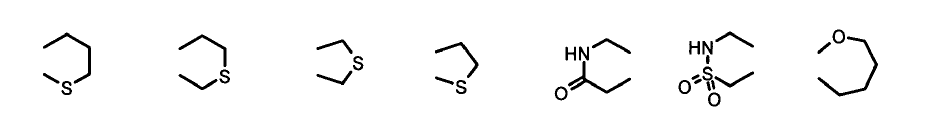

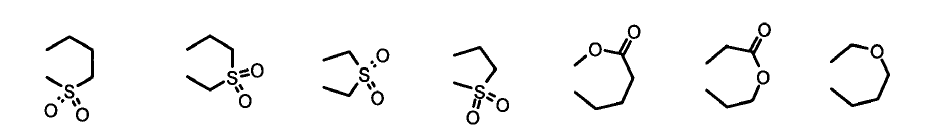

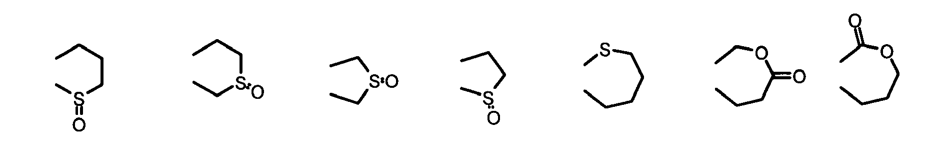

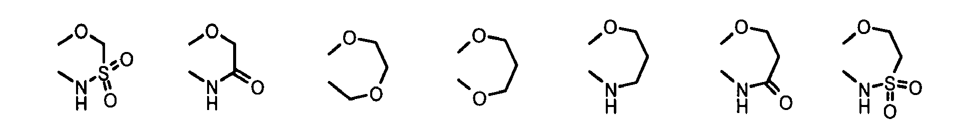

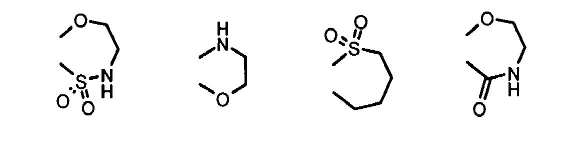

The heterocycloalkenylen groups for V may be for example the following groups:

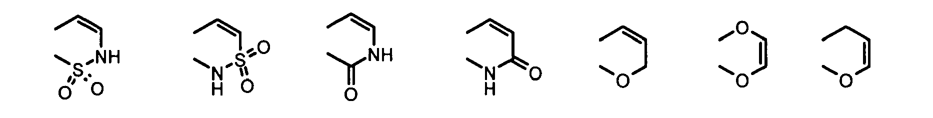

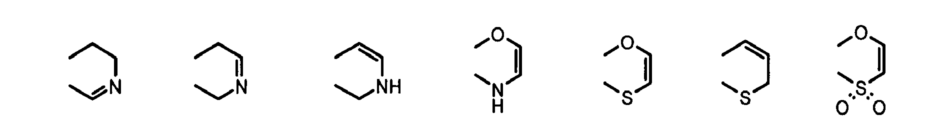

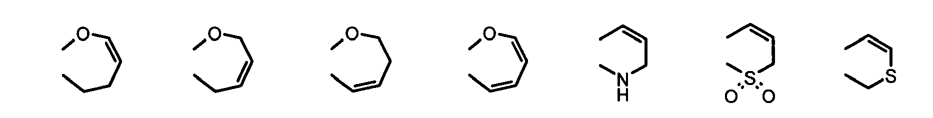

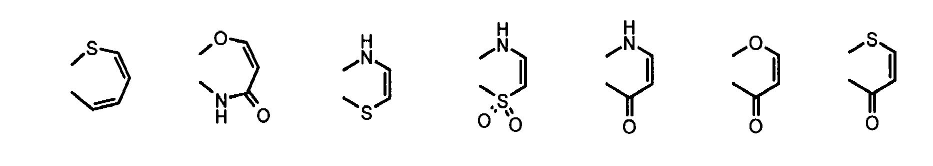

The cycloalkylen groups for V may be for example the following groups:

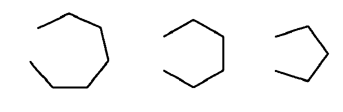

The cycloalkenylen groups for V may be for example the following groups:

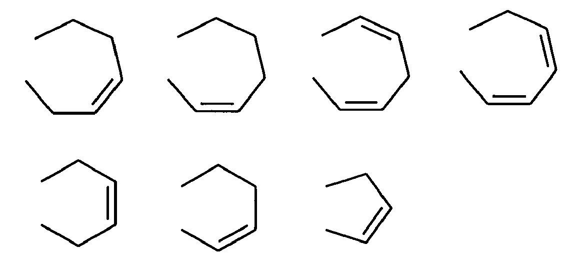

The C₃-C₇-cycloalkyloxy groups for the radicals R2 to R5 may be for example a cyclo-propyloxy, cyclobutyloxy, cyclopentyloxy, cyclohexyloxy, cycloheptyloxy group.

The C₁-C₆-alkylamino groups for the radicals R1 to R6 may be for example methyl-amino, ethylamino, propylamino, isopropylamino, butylamino, isobutylamino, sec-butylamino, *tert*-butylamino, pentylamino, isopentylamino, (2-methylbutyl)amino, (1-methylbutyl)amino, (1-ethylpropyl)amino, *ne*opentylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)-amino, (2,2-dimethylbutyl)amino, (1,1-dimethylbutyl)amino, (2,3-dimethylbutyl)amino, (1,3-dimethylbutyl)amino or a (1,2-dimethylbutyl)amino group.

In the di(C₁-C₆-alkyl)amino groups for the radicals R3 to R5, each of the two radicals on the nitrogen atom of the dialkylamino group may be chosen independently of one another from the following radicals: possible examples are a methyl, ethyl, propyl, isopropyl, butyl, isobutyl, sec-butyl, *tert*-butyl, pentyl, isopentyl, (2-methylbutyl), (1-methylbutyl), (1-ethylpropyl), *neo*pentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the C₃-C₇-cydoalkyl-C₁-C₆-alkyleneoxy groups for the radicals R2 to R5 it is possible to combine each of the C₃-C₇-cycloalkyl groups of the C₃-C₇-cycloalkyl-C₁-C₆-alkyleneoxy group, for example of a cyclopropyl, cyclobutyl, cyclopentyl, cyclohexyl or cycloheptyl group, independently of one another with each C0-C6-alkyleneoxy group, for example with a methyleneoxy, ethyleneoxy, propyleneoxy, butyleneoxy, pentylene-oxy, hexyleneoxy group.

In the hydroxy-C₃-C₆-alkenylene groups for the radicals R2 to R5 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (Z)-but-2-enyl, (*E*)-but-1-enyl, (*Z*)-but-1-eny), pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-l-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methyl-pent-3-enyl, (*Z*)-1-methylpent-3-enyl, (*E*)-4-methylpent-2-enyl, (Z)-4-methylpent-2-enyl, (*E*)-3-methylpent-2-enyl, (*Z*)-3-methylpent-2-enyl, (*E*)-2-methylpent-2-enyl, (*Z*)-2-methyl-pent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methyl-pent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethyl-but-2-enyl, (*E*)-2-ethylbut-2-enyl, (Z)-2-ethylbut-2-enyl, (*E*)-1-ethylbut-2-enyl, (*Z*)-1-ethyl-but-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isoprop-ylprop-1-enyl, (*E*)-1-isopropylprop-1-enyl, (*Z*)-1-isopropylprop-1-enyl, (*E*)-3,3-dimethyl-prop-1-enyl, (*Z*)-3,3-dimethylprop-1-enyl or a 1-(1,1-dimethylethyl)ethenyl group, and to be combined independently of one another.

In the hydroxy-C₃-C₆-alkynyl groups for the radicals R2 to R5 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-di-methylbut-3-ynyl, 1,1-dimethylbut-3-ynyl, 1,1-dimethylbut-2-ynyl or a 3,3-dimethylbut-1-ynyl group.

In the C₁-C₆-alkyloxy-C₃-C₆-alkenylene groups for the radicals R2 to R5 it is possible for the C₁-C₆-alkyloxy group, for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (Z)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methytprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (Z)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methyl-pent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methyl-pent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methyl-pent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (Z)-3-methyl-pent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (Z)-2-ethylbut-2-enyl, (*E*)-1-ethylbut-2-enyl, (*Z*)-1-ethylbut-2-enyl, (*E*)-3-ethylbut-l-enyl, (Z*)*-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another.

In the C₁-C₆-alkyloxy-C₃-C₆-alkynylene groups for the radicals R2 to R5 it is possible for the C₁-C₆-alkyloxy group, for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located at any desired position of the C3-C6-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group, and to be combined independently of one another.

In the C₃-C₇-cycloalkyl-(C₀-C₆)-alkyleneamino groups of the radicals R2 to R5 it is possible for each of the C₃-C₇-cycloalkyl groups of the C₃-C₇-cycloalkyl-(C₀-C₆)-alkylene-amino group, for example of a cyclopropyl, cyclobutyl, cyclopentyl, cyclohexyl or cycloheptyl group, to be combined independently of one another with each C₀-C₆-alkylene group, for example with a bond, a methylene, ethylene, propylene, butylene, pentylene, hexylene group.

In the C₁-C₆-alkyloxy-C₁-C₆-alkylene groups for the radical R2 to R5, it is possible for the C₁-C₆-alkyloxy group to be selected independently for example from methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.

In the di(C₁-C₆-alkyl)amino-C₁-C₆-alkylene group for the radicals R3 to R5 it is possible for each of the two radicals on the nitrogen atom of the amino group to be selected independently for example from methyl, ethyl, propyl, isopropyl, butyl, isobutyl, *sec*-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, and to be combined with C₁-C₆-alkylene groups such as, for example, methylene, ethylene, propylene, butylene, pentylene, hexylene.

The C₃-C₇-cycloalkyl-C₁-C₆-alkylene groups for the radicals R2 to R5 may be for example a cyclopropyloxymethylene, cyclopropyloxyethylene, cyclopropyloxypropylene, cyclopropyloxybutylene, cyclopropyloxypentylene, cyclopropyloxyhexylene, cyclobuty-loxymethylene, cyclobutyloxyethylene, cyclobutyloxypropylene, cyclobutyloxybutylene, cyclobutyloxypentylene, cyclobutyloxyhexylene, cyclopentyloxymethylene, cyclopenty-loxyethylene, cyclopentyloxypropylene, cyclopentyloxybutylene, cyclopentyloxypenty-lene, cyclopentyloxyhexylene, cyclohexyloxymethylene, cyclohexyloxyethylene, cyclo-hexyloxypropylene, cyclohexyloxybutylene, cyclohexyloxypentylene, cyclohexyloxy-hexylene, cycloheptyloxymethylene, cycloheptyloxyethylene, cycloheptyloxypropylene, cycloheptyloxybutylene, cycloheptyloxypentylene, cycloheptyloxyhexylen group.

In the C₁-C₆-alkylamino-C₁-C₆-alkylene groups for the radicals R2 to R5 it is possible for the C₁-C₆-alkylamino group to be selected independently for example from methyl-amino, ethylamino, propylamino, isopropylamino, butylamino, isobutylamino, sec-butylamino, *tert*-butylamino, pentylamino, isopentylamino, (2-methylbutyl)amino, (1-methylbutyl)amino, (1-ethylpropyl)amino, *neopent*ylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.

In the C₁-C₆-acyl-(C₀-C₆-alkyl)amido groups for the radicals R3 to R5, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethyl-butyryl, 2-ethylbutyryl, pentanoyl, 2-methylpentanoyl, 3-methylpentanoyl, 4-methylpentanoyl or a hexanoyl group, to be combined independently of one another with each (C₀-C₆-alkyl)amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)amido, (1-ethylpropyl)amido, neopentylamido, (1,1-dimethylpropyl)amido, hexylamido, (4-methylpentyl)amido, (3-methylpentyl)amido, (2-methylpentyl)amido, (1-methylpentyl)amido, (1-ethylbutyl)amido, (2-ethylbutyl)amido, (3,3-dimethylbutyl)amido, (2,2-dimethylbutyl)-amido, (1,1-dimethylbutyl)amido, (2,3-dimethylbutyl)amido, (1,3-dimethylbutyl)amido or a (1,2-dimethylbutyl)amido group.

The C₁-C₆-alkylaminocarbonyl groups for the radicals R3 to R5 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopen-tylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)aminocarbonyl, (1,1-dimethylbutyl)aminocarbonyl, (2,3-dimethylbutyl)aminocarbonyl, (1,3-dimethylbutyl)-aminocarbonyl or a (1,2-dimethylbutyl)aminocarbonyl group.

In the di(C₁-C₆-alkyl)aminocarbonyl groups for the radicals R3 to R5, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminocarbonyl groups for the radicals R3 to R5 may be for example a cyclopropylaminocarbonyl, cyclobutylaminocarbonyl, cyclopentylaminocar-bonyl, cyclohexylaminocarbonyl or cycloheptylaminocarbonyl group.

In the di(C₃-C₇-cycloalkyl)aminocarbonyl groups for the radicals R3 to R5, each of the two C₃-C₇-cycloalkyl radicals on the nitrogen atom of the di(C₃-C₇-cycloalkyl)aminocarbonyl group may be independently of one another for example a cyclopropyl, cyclobutyl, cyclopentyl, cyclohexyl or cycloheptyl group.

In the C₃-C₇-cycloalkyl-C₁-C₆-alkyleneaminocarbonyl groups of the radicals R3 to R5 it is possible for each of the C₃-C₇-cydoalkyl groups of the C₃-C₇-cydoalkyl-C₁-C₆-alkyl-eneaminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethyl-eneaminocarbonyl, propyleneaminocarbonyl, butyleneaminocarbonyl, pentylene-aminocarbonyl, hexyleneaminocarbonyl group.

The C₁-C₆-alkylcarbonyl groups for the radicals R3 to R5 may be for example a methyl-carbonyl, ethylcarbonyl, propylcarbonyl, isopropylcarbonyl, butylcarbonyl, isobutylcar-bonyl, sec-butylcarbonyl, tert-butylcarbonyl, pentylcarbonyl, isopentylcarbonyl, (2-methylbutyl)carbonyl, (1-methylbutyl)carbonyl, (1-ethylpropyl)carbonyl, neopentylcarbonyl, (1,1-dimethylpropyl)carbonyl, hexylcarbonyl, (4-methylpentyl)carbonyl, (3-methylpentyl)carbonyl, (2-methylpentyl)carbonyl, (1-methylpentyl)carbonyl, (1-ethylbutyl)carbonyl, (2-ethylbutyl)carbonyl, (3,3-dimethylbutyl)carbonyl, (2,2-dimethylbutyl)carbonyl, (1,1-dimethylbutyl)carbonyl, (2,3-dimethylbutyl)carbonyl, (1,3-dimethylbutyl)carbonyl or a (1,2-dimethylbutyl)carbonyl group.

The C₃-C₇-cycloalkylcarbonyl groups for the radicals R3 to R5 may be for example a cyclopropylcarbonyl, cyclobutylcarbonyl, cyclopentylcarbonyl, cyclohexylcarbonyl or cycloheptylcarbonyl group.

The C₁-C₆-alkyloxycarbonyl groups for the radicals R3 to R5 may be for example a me-thyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)oxycarbonyl, (1-ethylpropyl)oxycarbonyl, neopentyloxycarbonyl, (1,1-dimethylpropyl)oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radicals R3 to R5 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)-sulphonyl, (3-methylpentyl)sulphonyl, (2-methylpentyl)sulphonyl, (1-methylpentyl)sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sutphonyl, (2,3-dimethylbutyl)sulphonyl, (1,3-dimethylbutyl)sulphonyl or a (1,2-dimethylbutyl)sulphonyl group.

The C₃-C₇-cycloalkylsulphonyl groups for the radicals R3 to R5 may be for example a cyclopropylsulphonyl, cyclobutylsulphonyl, cyclopentylsulphonyl, cyclohexylsulphonyl or cycloheptylsulphonyl group.

In the C₃-C₇-cydoalkyl-C₁-C₆-alkylenesulphonyl groups of the radicals R3 to R5 it is possible for each of the C₃-C₇-cycloalkyl groups of the C₃-C₇-cycloalkyl-C₁-C₆-alkylene-sulphonyl groups, for example of a cyclopropyl, cyclobutyl, cyclopentyl, cyclohexyl or cycloheptyl group, to be combined independently of one another with each C₁-C₆-alkyl-enesulphonyl group, for example with a methylenesulphonyl, ethylenesulphonyl, propyl-enesulphonyl, butylenesulphonyl, pentylenesulphonyl, hexylenesulphonyl group.

The C₁-C₆-alkylaminosulphonyl groups for the radicals R3 to R5 may be for example a methylaminosulphonyl, ethylaminosulphonyl, propylaminosulphonyl, isopropylaminosul-phonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)-aminosulphonyl, (1-methylbutyl)aminosulphonyl, (1-ethylpropyl)aminosulphonyl, neopentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)-aminosulphonyl, (1-methylpentyl)aminosulphonyl, (1-ethylbutyl)aminosulphonyl, (2-ethylbutyl)aminosulphonyl, (3,3-dimethylbutyl)aminosulphonyl, (2,2-dimethylbutyl)-aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group.

In the di(C₁-C₆-alkyl)aminosulphonyl groups for the radicals R4 to R6, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminosulphonyl groups for the radicals R3 to R5 may be for example a cyclopropylaminosulphonyl, cyclobutylaminosulphonyl, cyclopentylaminosul-phonyl, cyclohexylaminosulphonyl or cycloheptylaminosulphonyl group.

In the di(C₃-C₇-cycloalkyl)aminosulphonyl groups for the radicals R3 to R5, each of the two C₃-C₇-cycloalkyl radicals on the nitrogen atom of the di(C₃-C₇-cycloalkyl)amino-sulphonyl group may be independently of one another for example a cyclopropyl, cyclobutyl, cyclopentyl, cyclohexyl or cycloheptyl group.

In the C₃-C₇-cycloalkyl-C₁-C₆-alkyleneaminosulphonyl groups of the radicals R3 to R5, each of the C₃-C₇-cycloalkyl groups of the C₃-C₇-cycloalkyl-C₁-C₆-alkyleneaminosul-phonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyl-eneaminosulphonyl, propyleneaminosulphonyl, butyleneaminosulphonyl, pentyleneami-nosulphonyl, hexyleneaminosulphonyl group.

The C₁-C₆-alkylsulphonylamido groups for the radicals R3 to R5 may be for example a methylsulphonylamido, ethylsulphonylamido, propylsulphonylamido, isopropylsulphon-ylamido, butylsulphonylamido, isobutylsulphonylamido, sec-butylsulphonylamido, tert-butylsulphonylamido, pentylsulphonylamido, isopentylsulphonylamido, (2-methylbutyl)-sulphonylamido, (1-methylbutyl)sulphonylamido, (1-ethylpropyl)sulphonylamido, neo-pentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.

In the -N(C₀-C₆-alkyl)-C(O)-C₁-C₆-alkyl groups of the radicals R3 to R5, each of the (_{C}0-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, iso-propyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N-(C₀-C₆-alkyl)-C(O)-C₃-C₇-cycloalkyl groups of the radicals R3 to R5, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.

In the -N(C₀-C₆-alkyl)-C(O)-N-di(C₀-C₆-alkyl) groups of the radicals R3 to R5, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radicals R3 to R5, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-NH-(C₃-C₇-cycloalkyl) groups of the radicals R3 to R5, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.

In the -N(C₀-C₆-alkyl)-SO₂-(C₁-C₆-alkyl) groups of the radicals R3 to R5, each of the (C₀-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1 ,2-dimethylbutyl) group.

In the -N(C₀-C₆-alkyl)-SO₂-C₃-C₇-cycloalkyl groups of the radicals R3 to R5, each of the (C₀-C₆-alkyl) groups on the nitrogen atom of the -N(C₀-C₆-alkyl)-SO₂-C₃-C₇-cydoalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the sulphonyl group, for example with a cyclopropyl, cyclobutyl, cyclopentyl, cyclohexyl or cycloheptyl group.

In the -N(C₀-C₆-alkyl)-SO₂-N-di(C₀-C₆-alkyl) groups of the radicals R3 to R5, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-NH-(C₃-C₇)-cycloalkyl groups of the radicals R3 to R5, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.

In the -C(O)-N(H)-C₂-C₆-alkylene-(C₁-C₆-alkyl)amine groups of the radicals R3 to R5, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-[di(C₁-C₆-alkyl)]amine groups of the radicals R3 to R5, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-(C₃-C₇-cycloalkyl)amine groups of the radicals R3 to R5, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.

In the -C(O)-N(H)-C₂-C₆-alkylene-(C₃-C₇-cycloalkyl-C₁-C₆-alkylene)amine groups of the radicals R3 to R5, each of the (C₂-C₆-alkylene) groups of the -C(O)-N(H)-C₂-C₆-alkylene-(C₃-C₆-cydoalky)-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radicals R3 to R5, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-[di(C₁-C₆-alkyl)]amine groups of the radicals R4 to R6, the C₂-C₆-alkylene group of the -S(O₂)-N(H)-C₂-C₆-alkylene-[di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-(C₃-C₇-cycloalkyl)amine groups of the radicals R3 to R5, the C₂-C₆-alkylene group of the -S(O₂)-N(H)-C₂-C₆-alkylene-(C₃-C₇-cycloalkyl)-amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.

In the -S(O₂)-N(H)-C₂-C₆-alkylene-(C₃-C₇cycloalkyl-C₁-C₆-alkylene)amine groups of the radicals R3 to R5, each C₂-C₆-alkylene group of the -S(O₂)-N(H)-C₂-C₆-alkylene-(C₃-C₇-cycloalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.

In the -O-C₂-C₆-alkylene-(C₁-C₆-alkyl)amine groups of the radicals R3 to R5, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -O-C₂-C₆-alkylene-[di(C₁-C₆-alkyl)]amine groups of the radicals R3 to R5, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Compounds preferred according to the present invention are those of the formulae II and III 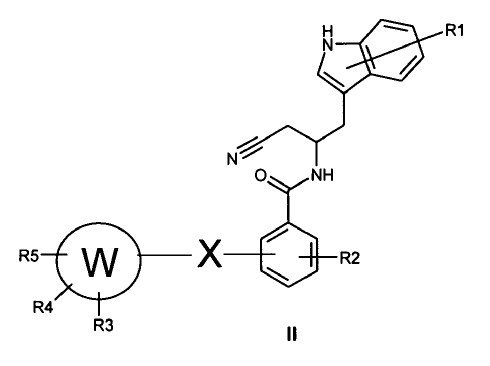 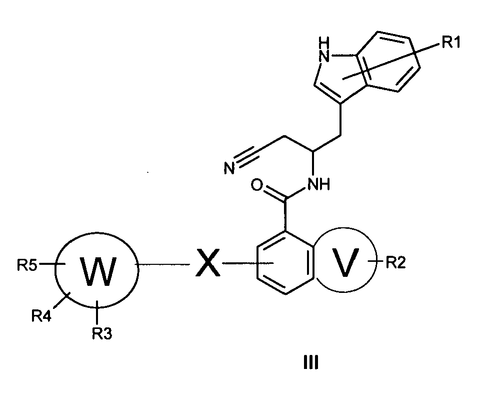 in which the radicals R1 to R5, X, W and V have the same meaning as defined in formula I.

Compounds particularly preferred according to the present invention are those of the formula IV and V 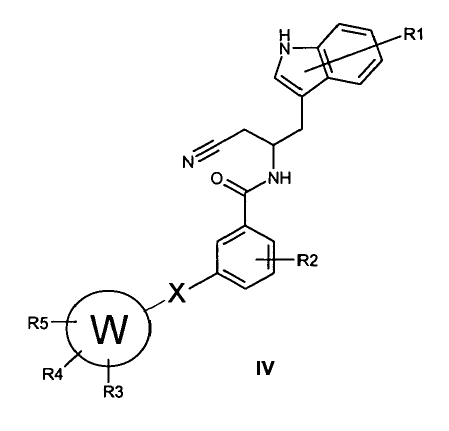 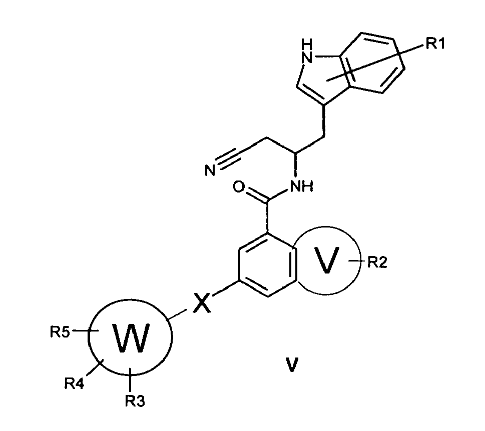 in which the radicals R1 to R5, X, W and V have the same meaning as defined in formula I.

The following compounds are very particularly preferred:

| | |
|---|---|
| **1** | 5'-Fluoro-4-isopropoxy-biphenyl-3,3'-dicarboxylic acid 3-{[2-cyano-1-(1H-indol-3-yl-methyl)-ethyl]-amide}3'-methylamide; |
| **2** | N-[2-Cyano-1-(1H-indol-3-ylmethyl)-ethyl]-5-(4-methylcarbamoyl-phenylethynyl)-2-trifluoromethoxy-benzamide; |
| **3** | 3'-Chloro-4-isopropoxy-biphenyl-3,4'-dicarboxylic acid 3-{[2-cyano-1-(1H-indol-3-yl-methyl)-ethyl]-amide}4'-methylamide; |
| **4** | 5-(4-Carbamoyl-2-methylphenylethynyl)-N-[2-cyano-1-(1H-indol-3-ylmethyl)-ethyl]-2-trifluoromethoxy-benzamide; |
| **5** | 3'-Chloro-4-isopropoxy-biphenyl-3,4'-dicarboxylic acid 4'-amide 3-{[2-cyano-1-(1H-indol-3-ylmethyl)-ethyl]-amide}; |
| **6** | 3'-Chloro-4-isopropoxy-4'-(morpholine-4-carbonyl)-biphenyl-3-carboxylic acid [2-cyano-1-(1H-indol-3-ylmethyl)-ethyl]-amide; |
| **7** | 4-Isopropoxy-3',4'-dimethoxy-biphenyl-3-carboxylic acid [2-cyano-1-(1H-indol-3-yl-methyl)-ethyl]-amide; |
| **8** | 5-(4-Carbamoyl-phenylethynyl)-N-[2-cyano-1-(1H-indol-3-ylmethyl)-ethyl]-2-isoprop-oxy-benzamide |
| **9** | N-[2-Cyano-1-(1H-indol-3-ylmethyl)-ethyl]-2-isopropoxy-5-(4-methylcarbamoyl-phenyl-ethynyl)-benzamide; |
| **10** | N-[2-Cyano-1-(1H-indol-3-ylmethyl)-ethyl]-2-isopropoxy-5-(3-methylcarbamoyl-phenyl-ethynyl)-benzamide; |
| **11** | N-[2-Cyano-1-(1H-indol-3-ylmethyl)-ethyl]-2-isopropoxy-5-(2-methylcarbamoyl-phenyl-ethynyl)-benzamide; |
| **12** | 4'-Morpholin-4-yl-4-trifluoromethoxy-biphenyl-3-carboxylic acid [2-cyano-1-(1H-indol-3-ylmethyl)-ethyl]-amide |
| **13** | 4'-Methanesulfonylamino-methyl)-4-trifluoromethoxy-biphenyl-3-carboxylic acid [2-cyano-1-(1H-indol-3-ylmethyl)-ethyl]-amide; |
| **14** | N-{3'-[2-Cyano-1-(1H-indol-3-ylmethyl)-ethylcarbamoyl]-4'-trifluoromethoxy-biphenyl- |
| | 3-yl}-succinamic acid; |
| **15** | 4'-(Pyrrolidine-1-carbonyl)-4-trifluoromethoxy-biphenyl-3-carboxylic acid [2-cyano-1-(1 H-indol-3-ylmethyl)-ethyl]-amide; |
| **16** | N-[2-Cyano-1-(1H-indol-3-ylmethyl)-ethyl]-5-(1-methyl-1H-indol-5-yl)-2-trifluoromethoxy-benzam ide; |
| **17** | 4-Trifluoromethoxy-biphenyl-3,4'-dicarboxylic acid 3-{[2-cyano-1-(1H-indol-3-yl-methyl)-ethyl]-amide}4'-[(2-methoxy-ethyl)-amide]; |
| **18** | 3'-(Pyrrolidine-1-carbonyl)-4-trifluoromethoxy-biphenyl-3-carboxylic acid [2-cyano-1-(1 H-indol-3-ylmethyl)-ethyl]-amide; |
| **19** | 4-Trifluoromethoxy-biphenyl-3,3'-dicarboxylic acid 3-{[2-cyano-1-(1H-indol-3-yl-methyl)-ethyl]-amide}3'-ethylamide; |
| **20** | 4-Trifluoromethoxy-biphenyl-3,3'-dicarboxylic acid 3-{[2-cyano-1-(1H-indol-3-yl-methyl)-ethyl]-amide}3'-(isobutyl-amide); |
| **21** | 4-Trifluoromethoxy-biphenyl-3,3'-dicarboxylic acid 3-{[2-cyano-1-(1H-indol-3-yl-methyl)-ethyl]-amide}3'-cyclopentylamide; |
| **22** | 3'-(Methanesulfonylamino-methyl)-4-trifluoromethoxy-biphenyl-3-carboxylic acid [2-cyano-1-(1H-indol-3-ylmethyl)-ethyl]-amide; |
| **23** | 4'-(Acetylamino-methyl)-4-trifluoromethoxy-biphenyl-3-carboxylic acid [2-cyano-1-(1H-indol-3-ylmethyl)-ethyl]-amide; |
| **24** | 3'-(Acetylamino-methyl)-4-trifluoromethoxy-biphenyl-3-carboxylic acid [2-cyano-1-(1H-indol-3-ylmethyl)-ethyl]-amide; |
| **25** | 4'-Methylsulfamoyl-4-trifluoromethoxy-biphenyl-3-carboxylic acid [2-cyano-1-(1H-indol-3-ylmethyl)-ethyl]-amide; |
| **26** | 3-Chloro-3'-[2-cyano-1-(1H-indol-3-ylmethyl)-ethylcarbamoyl]-4'-trifluoromethoxy-biphenyl-4-carboxylic acid methyl ester; |
| **27** | 4'-Ethylsulfamoyl-4-trifluoromethoxy-biphenyl-3-carboxylic acid [2-cyano-1-(1H-indol-3-ylmethyl)-ethyl]-amide; |
| **28** | 3'-Chloro-4-trifluoromethoxy-biphenyl-3,4'-dicarboxylic acid 3-{[2-cyano-1-(1H-indol-3-ylmethyl)-ethyl]-amide}4'-isopropylamide; |
| **29** | 3'-Chloro-4-trifluoromethoxy-biphenyl-3,4'-dicarboxylic acid 3-{[2-cyano-1-(1H-indol-3-ylmethyl)-ethyl]-amide}4'-cyclopropylamide; |
| **30** | 3'-Chloro-4-trifluoromethoxy-biphenyl-3,4'-dicarboxylic acid 4'-tert-butylamide 3-{[2-cyano-1-(1H-indol-3-ylmethyl)-ethyl]-amide}; |
| **31** | 3'-Chloro-4-trifluoromethoxy-biphenyl-3,4'-dicarboxylic acid 3-{[2-cyano-1-(1H-indol-3-ylmethyl)-ethyl]-amide}4'-dimethylamide; |
| **32** | 3'-Chloro-4-trifluoromethoxy-biphenyl-3,4'-dicarboxylic acid 3-{[2-cyano-1-(1H-indol-3-ylmethyl)-ethyl]-amide}4'-diethylamide; |
| **33** | 3'-Chloro-4'-(morpholine-4-carbonyl)-4-trifluoromethoxy-biphenyl-3-carboxylic acid [2-cyano-1-(1H-indol-3-ylmethyl)-ethyl]-amide; |
| **34** | 3'-Chloro-4'-(piperidine-1-carbonyl)-4-trifluoromethoxy-biphenyl-3-carboxylic acid [2-cyano-1-(1H-indol-3-ylmethyl)-ethyl]-amide; |
| **35** | 3'-Chloro-4'-(pyrrolidine-1-carbonyl)-4-trifluoromethoxy-biphenyl-3-carboxylic acid [2-cyano-1-(1H-indol-3-ylmethyl)-ethyl]-amide; |
| **36** | 5'-Ethoxy-3'-fluoro-4-trifluoromethoxy-biphenyl-3-carboxylic acid [2-cyano-1-(1H-indol-3-ylmethyl)-ethyl]-amide; |
| **37** | 5'-Fluoro-4-trifluoromethoxy-biphenyl-3,3'-dicarboxylic acid 3-{[2-cyano-1-(1H-indol-3-ylmethyl)-ethyl]-amide}3'-methylamide; |
| **38** | 3'-[2-Cyano-1-(1H-indol-3-ylmethyl)-ethylcarbamoyl]-3-methoxy-4'-trifluoromethoxy-biphenyl-4-carboxylic acid methyl ester; |
| **39** | 3'-Fluoro-5'-methoxy-4-trifluoromethoxy-biphenyl-3-carboxylic acid [2-cyano-1-(1H-indol-3-ylmethyl)-ethyl]-amide; |
| **40** | 4'-Acetylamino-4-trifluoromethoxy-biphenyl-3-carboxylic acid [2-cyano-1-(1H-indol-3-ylmethyl)-ethyl]-amide; |
| **41** | N-[2-Cyano-1-(1H-indol-3-ylmethyl)-ethyl]-5-quinolin-3-yl-2-trifluoromethoxy-benzamide; |
| **42** | 3',5'-Dimethyl-4-trifluoromethoxy-biphenyl-3-carboxylic acid [2-cyano-1-(1H-indol-3-ylmethyl)-ethyl]-amide; |
| **43** | 4-Trifluoromethoxy-biphenyl-3,3'-dicarboxylic acid 3'-amide 3-{[2-cyano-1-(1H-indol-3-ylmethyl)-ethyl]-amide}; |
| **44** | 3'-Cyano-4-trifluoromethoxy-biphenyl-3-carboxylic acid [2-cyano-1-(1H-indol-3-yl-methyl)-ethyl]-amide; |
| **45** | 5-Benzo[1,3]dioxol-5-yl-N-[2-cyano-1-(1H-indol-3-ylmethyl)-ethyl]-2-trifluoromethoxy-benzamide; |
| **46** | 3'-Acetylamino-4-trifluoromethoxy-biphenyl-3-carboxylic acid [2-cyano-1-(1H-indol-3-ylmethyl)-ethyl]-amide; |
| **47** | 3'-Methanesulfonylamino-4-trifluoromethoxy-biphenyl-3-carboxylic acid [2-cyano-1-(1H-indol-3-ylmethyl)-ethyl]-amide; |
| **48** | 4'-Chloro-4-trifluoromethoxy-biphenyl-3-carboxylic acid [2-cyano-1-(1H-indol-3-yl-methyl)-ethyl]-amide; |
| **49** | 2'-Methyl-4-trifluoromethoxy-biphenyl-3-carboxylic acid [2-cyano-1-(1H-indol-3-yl-methyl)-ethyl]-amide; |
| **50** | 2'-Fluoro-4-trifluoromethoxy-biphenyl-3-carboxylic acid [2-cyano-1-(1H-indol-3-yl-methyl)-ethyl]-amide; |
| **51** | 4-Trifluoromethoxy-biphenyl-3,3'-dicarboxylic acid 3-{[2-cyano-1-(1H-indol-3-yl-methyl)-ethyl]-amide}3'-methylamide; |
| **52** | N-[2-Cyano-1-(1H-indol-3-ylmethyl)-ethyl]-5-quinolin-6-yl-2-trifluoromethoxy-benzamide; |
| **53** | 3'-Hydroxy-4-trifluoromethoxy-biphenyl-3-carboxylic acid [2-cyano-1-(1H-indol-3-ylmethyl)-ethyl]-amide; |
| **54** | 4-Trifluoromethoxy-4'-trifluoromethyl-biphenyl-3-carboxylic acid [2-cyano-1-(1H-indol-3-ylmethyl)-ethyl]-amide; |
| **55** | 3'-Hydroxymethyl-4-trifluoromethoxy-biphenyl-3-carboxylic acid [2-cyano-1-(1H-indol-3-ylmethyl)-ethyl]-amide; |
| **56** | 4-Trifluoromethoxy-[1,1';3',1"]terphenyl-3-carboxylic acid [2-cyano-1-(1H-indol-3-yl-methyl)-ethyl]-amide; |
| **57** | 3'-Fluoro-4-trifluoromethoxy-biphenyl-3-carboxylic acid [2-cyano-1-(1H-indol-3-yl-methyl)-ethyl]-amide; |
| **58** | 3'-Acetyl-4-trifluoromethoxy-biphenyl-3-carboxylic acid [2-cyano-1-(1H-indol-3-yl-methyl)-ethyl]-amide; |
| **59** | 3'-Amino-4-trifluoromethoxy-biphenyl-3-carboxylic acid [2-cyano-1-(1H-indol-3-yl-methyl)-ethyl]-amide; |
| **60** | 4'-Acetyl-4-trifluoromethoxy-biphenyl-3-carboxylic acid [2-cyano-1-(1H-indol-3-yl-methyl)-ethyl]-amide; |
| **61** | 4'-Methylsulfanyl-4-trifluoromethoxy-biphenyl-3-carboxylic acid [2-cyano-1-(1H-indol-3-ylmethyl)-ethyl]-amide; |
| **62** | 4-Trifluoromethoxy-3'-trifluoromethyl-biphenyl-3-carboxylic acid [2-cyano-1-(1H-indol-3-ylmethyl)-ethyl]-amide; |
| **63** | 4'-Hydroxy-4-trifluoromethoxy-biphenyl-3-carboxylic acid [2-cyano-1-(1H-indol-3-yl-methyl)-ethyl]-amide; |
| **64** | N-[2-Cyano-1-(1H-indol-3-ylmethyl)-ethyl]-5-naphthalen-1-yl-2-trifluoromethoxy-benzamide; |
| **65** | 4'-Methyl-4-trifluoromethoxy-biphenyl-3-carboxylic acid [2-cyano-1-(1H-indol-3-yl-methyl)-ethyl]-amide; |
| **66** | 3'-Chloro-4-trifluoromethoxy-biphenyl-3-carboxylic acid [2-cyano-1-(1H-indol-3-yl-methyl)-ethyl]-amide; |
| **67** | N-[2-Cyano-1-(1H-indol-3-ylmethyl)-ethyl]-5-naphthalen-2-yl-2-trifluoromethoxy-benzamide; |
| **68** | 3'-Methoxy-4-trifluoromethoxy-biphenyl-3-carboxylic acid [2-cyano-1-(1H-indol-3-yl-methyl)-ethyl]-amide; |
| **69** | 4'-Methoxy-4-trifluoromethoxy-biphenyl-3-carboxylic acid [2-cyano-1-(1H-indol-3-yl-methyl)-ethyl]-amide; |
| **70** | N-[2-Cyano-1-(1H-indol-3-ylmethyl)-ethyl]-5-thiophen-3-yl-2-trifluoromethoxy-benzamide. |

### Pharmacological Experiments

### HTRF assay for measuring cAMP in cells

The method is based on a competitive immunoassay between native cAMP, which has been produced by the cells, and cAMP which is labelled with cAMPD2 conjugate. The tracer binding was visualized by a monoclonal antibody, anti-cAMP labelled with cryptate [HTRF = homogeneous time-resolved fluorescence].

The specific signal is inversely proportional to the cAMP concentration of the samples employed.

The 665nm/ 620nm fluorescence ratio was evaluated.

*The following material was used: 96-well plates for the tissue culture, 96-well plates with black edge and black base (e.g. Fluotrac 600 from Greiner), 96-well plates for the substance dilutions of polypropylene and CAMP Femtomolar (4000wells Kit, CIS Bio International # 62AM5PEJ).*

The following reagents were used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.
Buffer 1 (washing and testing buffer) contained PBS, 1 mM CaCl2, 1 mM MgCl₂, 0.2% glucose; 0.1 % BSA, 1 mM IBMX.
Buffer 2 (2x lysis buffer) contained 1% Triton X-100 in PBS (without CaCl₂ and MgCl₂).
Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells.

The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min).

The test conjugates (cAMP-D2 and anti-cAMP cryptate, CIS Bio) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of cAMP-D2 conjugate were pipetted and, after 10 min, 25 µl of the anti-cAMP cryptate were added). This is followed by incubation at RT for 90 minutes. The measurement was carried out in a PheraStar (BMG).

**Tissue culture conditions**

| | |
|---|---|
| 1) hFSHr clone 16 | Ham's F12 |
| | PSG |
| | 10% FCS |
| | 700 µg/ml G 418 (Geneticin) from PAA. |

Dose-effect curve (hFSH) for the human receptor: 1 e-8, 3e-9, 1 e-9, 3e-10, 1e-10, 3e-11, 1e-11, 3e-12 mol/l.

The test substances were employed in suitable dilutions in the absence (test for agonism) and in the presence of 1e-9 mol/l hFSH.

### Evaluation

The values of the well ratio were averaged and then entered directly in SigmaPlot versus the concentrations. The maximum and minimum values were determined for each plate, and half the difference is to be regarded as IC₅₀.

The test results (Table 1) show that the compounds according to the invention have an FSH-antagonistic effect.

**Table 1. FSH antagonistic effect of selected compounds in the HTRF assay**

| **Compound [Ex. #]** | **IC₅₀** |
|---|---|
| **1** | **3.5 µM** |
| **4** | **250 nM** |
| **6** | **80 nM** |
| **8** | **200 nM** |
| **9** | **750 nM** |
| **10** | **3.5 µM** |
| **11** | **10 µM** |

Being antagonists of the FSH receptor, compounds of the general formula I and their pharmaceutically acceptable salts can thus be used for the fertility control in male and/or in a female animals, in particular in men and/or women; as well as for the treatment and/or prevention of osteoporosis.

### Pharmaceutical compositions

The invention further relates to compounds of the general formula I or pharmaceutically acceptable salts thereof as therapeutic active ingredients, and to pharmaceutical compositions comprising at least one compound of the general formula I or pharmaceutically acceptable salts thereof, where appropriate together with pharmaceutically suitable excipients and/or carriers.

Pharmaceutically acceptable salts of the compounds of the general formula I can be prepared by methods known to the skilled person, depending on the nature of the compound of formula I, either by using as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid;
or by using an appropriate base as inorganic base inter alia alkalimetal hydroxide, carbonate, or hydrogencarbonate, as organic base inter alia tertiary amines and N-heterocycles.

These pharmaceutical compositions and medicaments may be intended for oral, rectal, subcutaneous, transdermal, percutaneous, intravenous or intramuscular administration. They comprise besides conventional carriers and/or diluents at least one compound of the general formula I.

The medicaments of the invention are formulated using the customary solid or liquid carriers or diluents and the excipients customarily used in pharmaceutical technology, in accordance with the desired mode of administration with a suitable dosage in a known manner: i.e. by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980) and to Gennaro, A. R. et al., Remington: The Science and Practice of Pharmacy (20th Edition., Lippincott Williams & Wilkins 2000, see especially Part 5: Pharmaceutical Manufactoring).

Pharmaceutical compositions according to the present invention are preferably administered orally. Suitable for oral administration are in particular tablets, (film)-coated tablets, sugar-coated tablets capsules, pills, powders, granules, pastilles, suspensions, emulsions, solutions or depot forms.

Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.

Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.

Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as p-hydroxybenzoates.

Capsules comprising the compounds of the general formula I can be produced for example by the compound(s) of the general formula I being mixed with an inert carrier such as lactose or sorbitol and encapsulated in gelatine capsules.

Parenteral preparations such as solutions for injection are also suitable. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy.

Suitable suppositories can be produced for example by mixing with carriers intended for this purpose, such as neutral fats or polyethylene glycol or derivatives thereof.

Formulations suitable for topical application include gels, ointments, greasy ointments, creams, pastes, dusting powders, milk and tinctures. Topical use can also take place by means of a transdermal system, for example a patch. The concentration of the compounds of the general formula I in these preparations should typically be in the range of 0.01% - 20% in order to achieve an adequate pharmacological effect.

The invention further relates to pharmaceutical compositions in combination with packaging material suitable for said composition, wherein said packaging material including instructions for the use of the composition.

### Dose

Suitable doses for the compounds according to the present invention may vary from 0.005 mg to 50 mg per day per kg of body weight, depending on the age and constitution of the patient. It is possible to administer the necessary daily dose by single or multiple delivery. The preferred daily dose for larger mammals, for example humans, may vary in the range from 10 µg to 30 mg per kg of body weight.

The exact dose and regimen of administration of the drug substance (active ingredient, or a pharmaceutical composition thereof, may however vary with the particular compound, the route of administration, and the age, sex and condition of the individual to whom the medicament is administered. The dose, the dosage as well as the regimen of the administration may thus differ between a male and a female considerably.

The present invention also relates to a process for preparing the compounds according to the invention. Compounds of the general formula I can be prepared as shown in Scheme 1 by an amide formation reaction between the amine VI and the carboxylic acid VII. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N*'-ethylcarbodiimide hydrochloride) /HOBt (1-hydroxy-1*H*-benzotriazole). It is possible as alternative for the carboxylic acid to be converted, where appropriate in the presence of a base, into the carbonyl chloride and reacted with the amine VI to give the product of the general formula I.

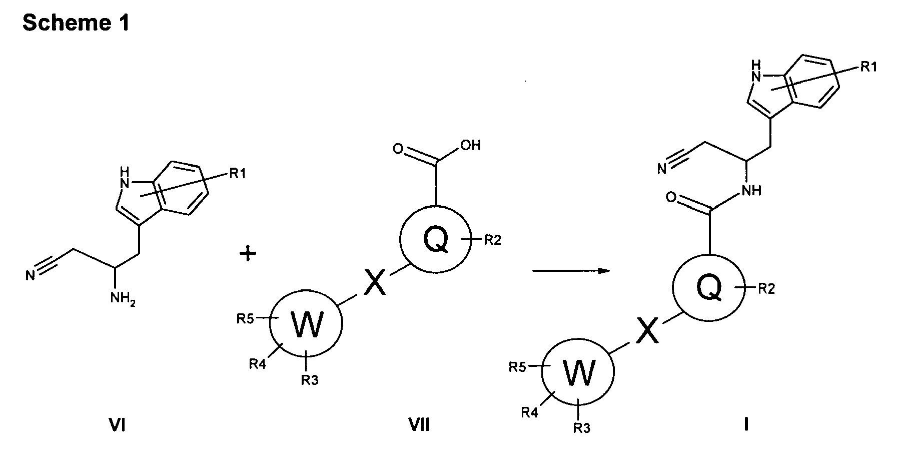

Compounds of the general formula II can be prepared as shown in Scheme 2 by an amide formation reaction between the amine VI and the carboxylic acid VIII. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzo-triazol-1-yl)oxy]tris(pyrrolidin-1-yl)phosphonium hexafluorophosphate), HATU (2-(7-aza-1H-benzotriazol-1-yl)-1,1,3,3-tetramethyluronium hexafluorophosphate), HBTU (2-(1*H-*benzotriazol-1-yl)-1,1,3,3-tetramethyluronium hexafluorophosphate), EDC (N-[3-(di-methylamino)propyl]-*N'*-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the amine VI to give the product of the general formula II.

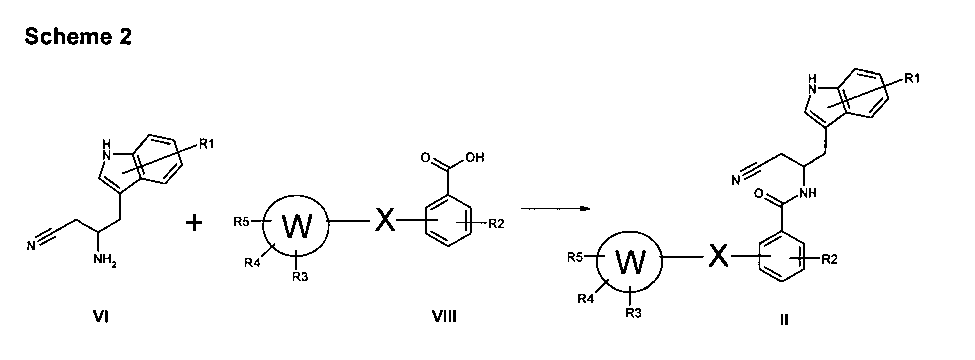

Compounds of the general formula III can be prepared as shown in Scheme 3 by an amide formation reaction between the amine VI and the carboxylic acid IX. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzo-triazol-1-yl)oxy]tris(pyrrolidin-1-yl)phosphonium hexafluorophosphate), HATU (2-(7-aza-1H-benzotriazol-1-yl)-1,1,3,3-tetramethyluronium hexafluorophosphate), HBTU (2-(1*H-*benzotriazol-1-yl)-1,1,3,3-tetramethyluronium hexafluorophosphate), EDC (N-[3-(di-methylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the amine VI to give the product of the general formula III.

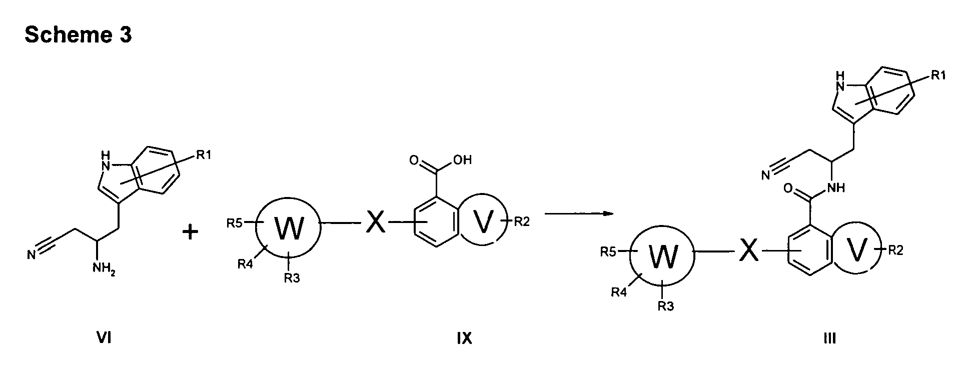

Compounds of the general formula IV can be prepared as shown in Scheme 4 by an amide formation reaction between the amine VI and the carboxylic acid X. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzo-triazol-1-yl)oxy]tris(pyrrolidin-1-yl)phosphonium hexafluorophosphate), HATU (2-(7-aza-1H-benzotriazol-1-yl)-1,1,3,3-tetramethyluronium hexafluorophosphate), HBTU (2-(1*H-*benzotriazol-1-yl)-1,1,3,3-tetramethyluronium hexafluorophosphate), EDC (*N*-[3-(di-methylamino)propyl]-*N'*-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the amine VI to give the product of the general formula IV.

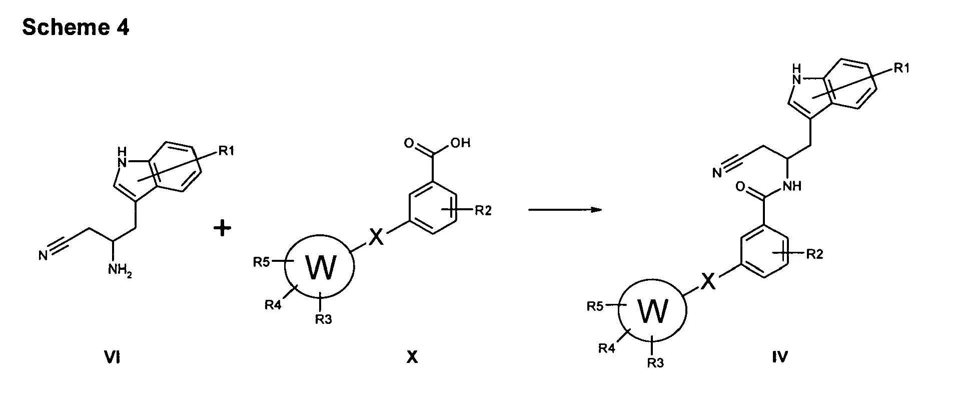

Compounds of the general formula V can be prepared as shown in Scheme 5 by an amide formation reaction between the amine VI and the carboxylic acid XI. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotri-azol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1H-benzotriazol-1-yl)-1,1,3,3-tetramethyluronium hexafluorophosphate), EDC (N-[3-(di-methylamino)propyl]-*N'*-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the amine VI to give the product of the general formula V.

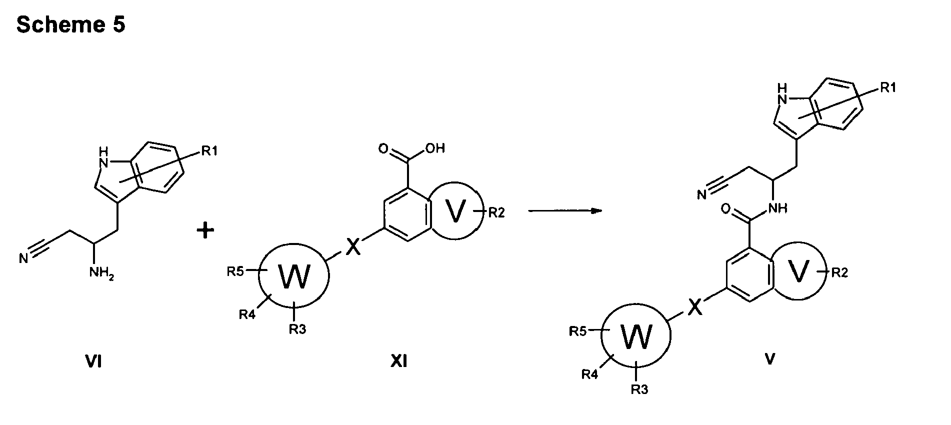

Alternatively, compounds of the general formula I can also be prepared as shown in Scheme 6 by a metal catalyzed cross coupling reaction between the aryl halide XIII (wherein Hal stands for chlorine, bromine or iodine) and the correspondingly functionalized building block XII known to the skilled person, for example: a Suzuki reaction (R = -B(OH)₂), a Sonogashira coupling (R = H, X = -C≡C-), a Negishi coupling (R = Zn-Hal) or a Stille coupling (R = Sn(alkyl)₃). Preferred metal catalysts used are typically those containing palladium or nickel, depending on the nature of the cross-coupling reaction. For a more detailed description of the metal catalyzed cross-coupling reactions applied and applicable for the synthesis of compounds of the formula I, see e.g. S. Takahashi, Y. Kuroyama, K. Sonogashira and N. Hagihara, Synthesis, 1980, 627; Metal catalyzed Cross-coupling Reactions, ed. F. Diederich and P. J. Stang, Wiley-VCH, Weinheim, 1998; K. Sonogashira, J. Organomet. Chem., 2002, 653 (1-2), 46.

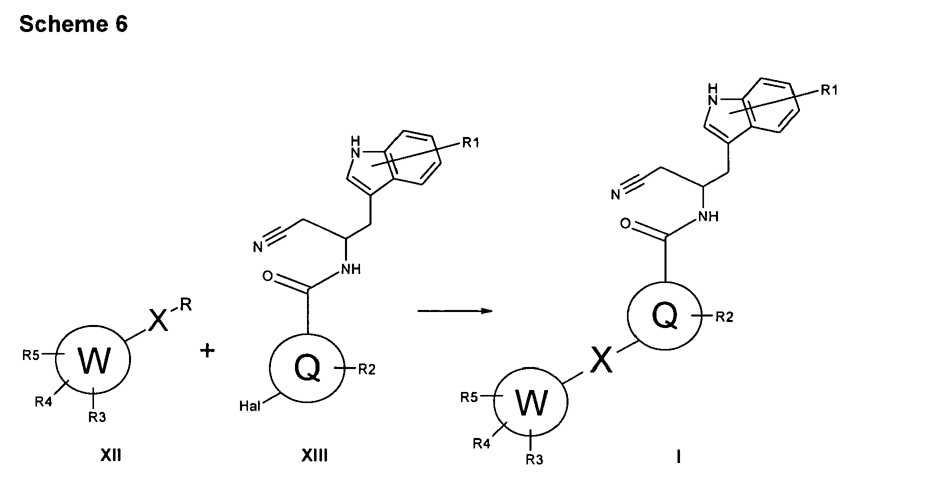

Likewise, compounds of the general formula II can be prepared as shown in Scheme 7 by a metal catalyzed cross coupling reaction between the aryl halide XIV and the correspondingly functionalized building block XII known to the skilled person.

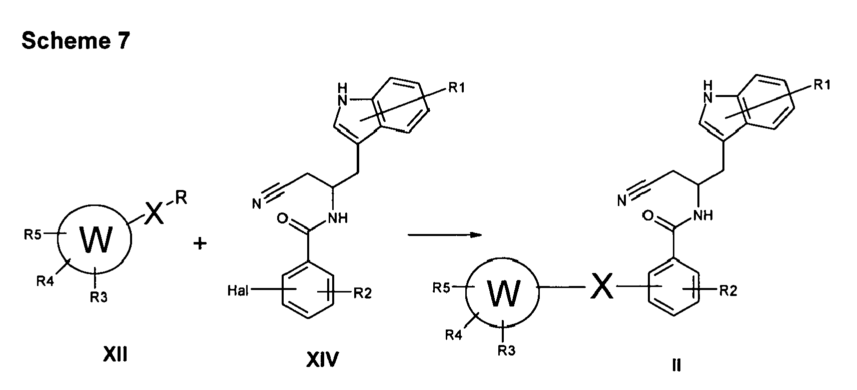

Likewise, compounds of the general formula III can be prepared as shown in Scheme 8 by a metal catalyzed cross coupling reaction between the aryl halide XV and the correspondingly functionalized building block XII known to the skilled person.

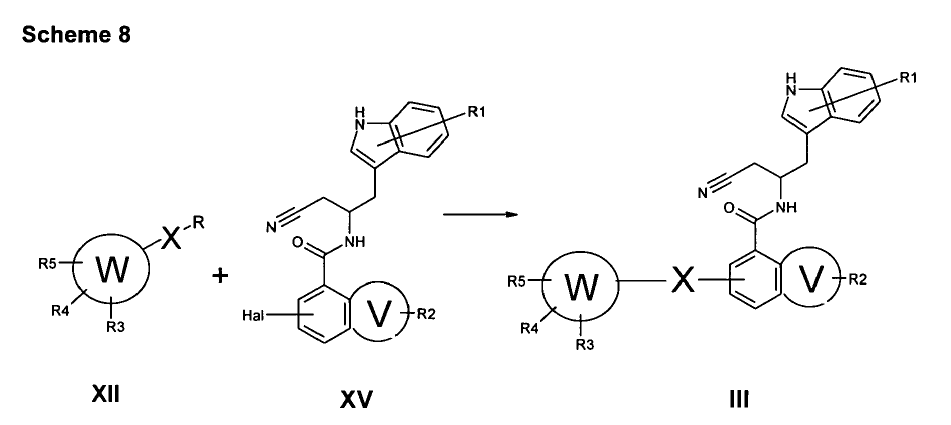

Likewise, compounds of the general formula IV can be prepared as shown in Scheme 9 by a metal catalyzed cross coupling reaction between the aryl halide XVI and the correspondingly functionalized building block XII known to the skilled person.

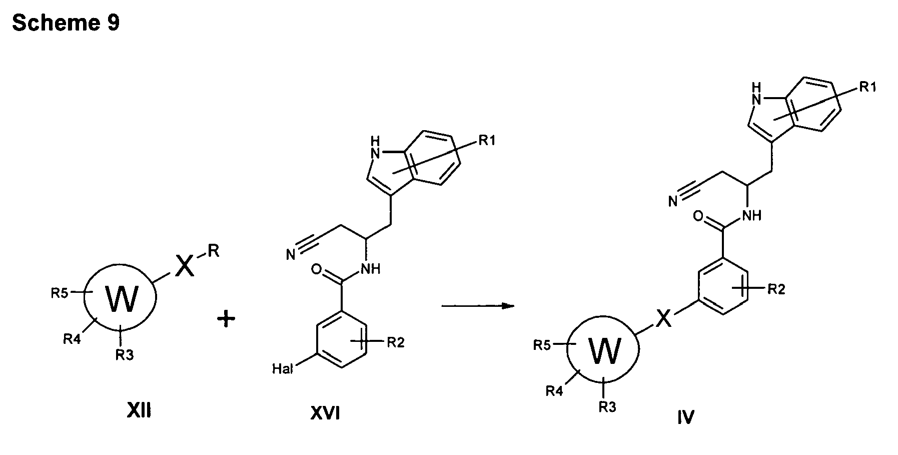

Likewise, compounds of the general formula V can be prepared as shown in Scheme 10 by a metal catalyzed cross coupling reaction between the aryl halide XVII and the correspondingly functionalized building block XII known to the skilled person.

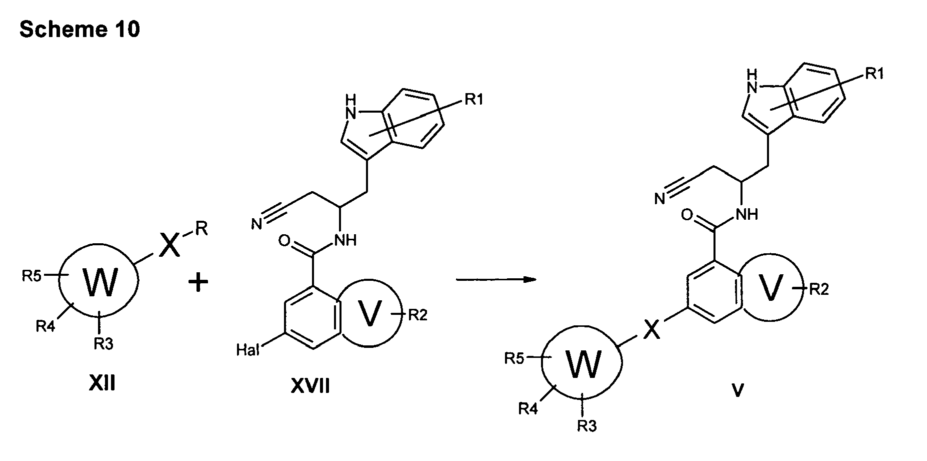

Compounds of the general formula I can in principle be prepared as shown in Scheme 11 by an amide formation reaction between an amine VI and a carboxylic acid VII. The reagents typically used for the coupling are EDCI and HOBt.

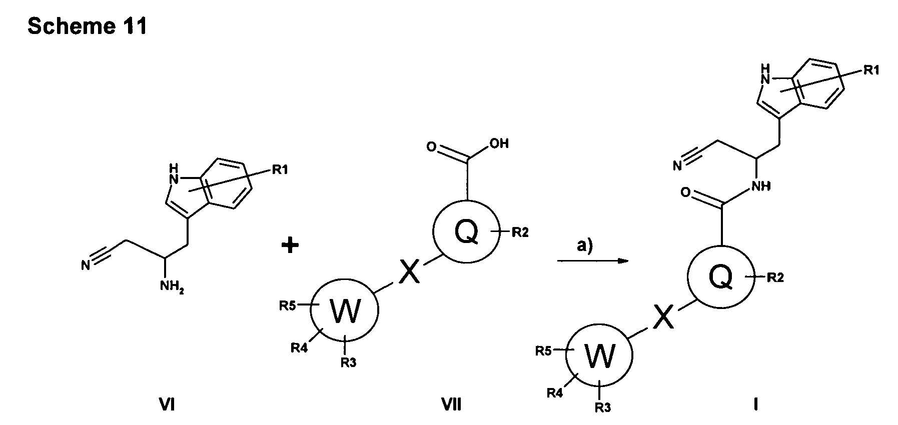

**Reagents:** a) EDCI, HOBt, triethylamine, DMF.

Compounds of the general formula VI can be prepared according to scheme 12 starting from commercially available or literature known tryptophan derivatives XVIII. Esterification and subsequent reduction of the tryptophan esters XIX to the corresponding tryptophanols XX and subsequent double tosylation gives rise to compounds of formula XXI. After displacement of one tosyl group by a cyano group and deprotection of the tosyl amide one obtains the nitriles of general formula VI.

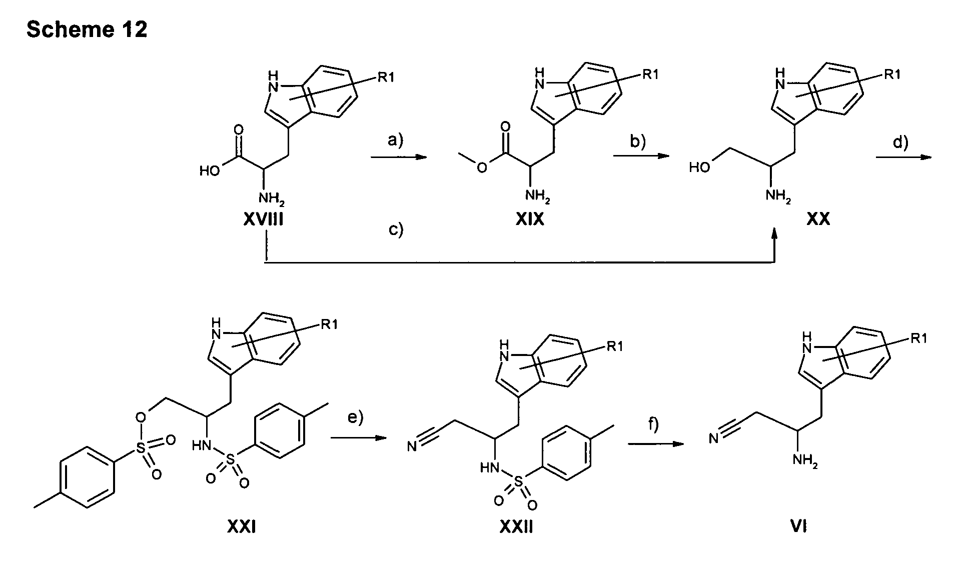

Reagents: a) SOCl₂, MeOH; b) LiBH₄, THF; c) LiAlH₄, THF; d) TsCl, Pyridine; e) KCN, MeOH; f) Na, NH₃.

The carboxylic acid derivatives of formula XXXVIII can in principle be prepared according to Scheme 13 via a Sonogashira type coupling of acetylenes XXIII or XXVII with their corresponding aryl halides XXIV or XXVI with subsequent hydrolysis of the resulting carboxylic esters XXV.

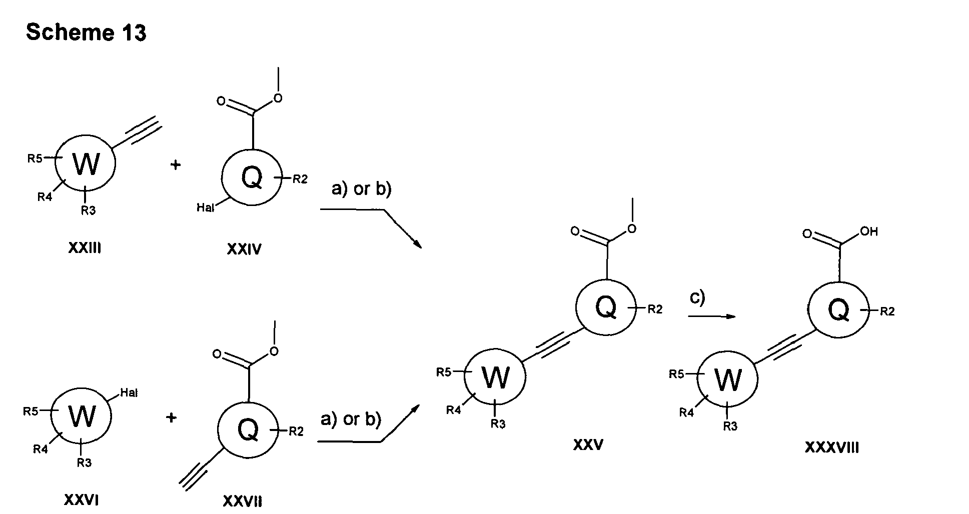

Reagents: a) Pd(PPh₃)₂Cl₂, TBAF, neat or THF; b) Pd(PPh₃)₂Cl₂, Cul, Et₂NH; c) KOH, MeOH.

The acetylene derivatives of formula XXVII can in principle be prepared according to Scheme 14 via a Sonogashira type coupling of aryl halide XXIV and trimethylsilyl protected acetylene with subsequent hydrolysis of the trimethylsilyl protecting group.

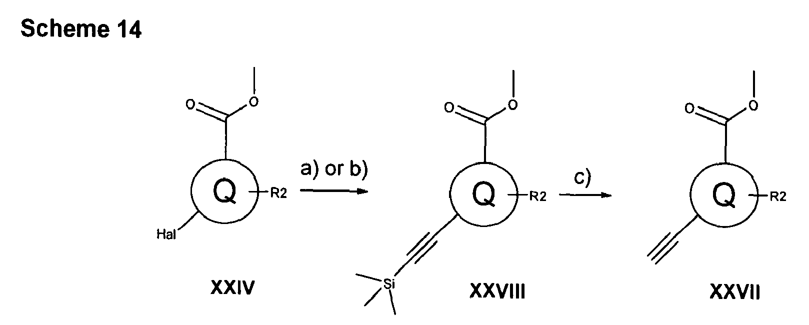

**Reagents:** a) Pd(PPh₃)₂Cl₂, TBAF, neat or THF; b) Pd(PPh₃)₂Cl₂, Cul, Et₂NH; c) TBAF

The acetylene derivatives of formula XXIII can in principle be prepared according to Scheme 15 via a Sonogashira type coupling of aryl halide XXVI and trimethylsilyl protected acetylene with subsequent hydrolysis of the trimethylsilyl protecting group.

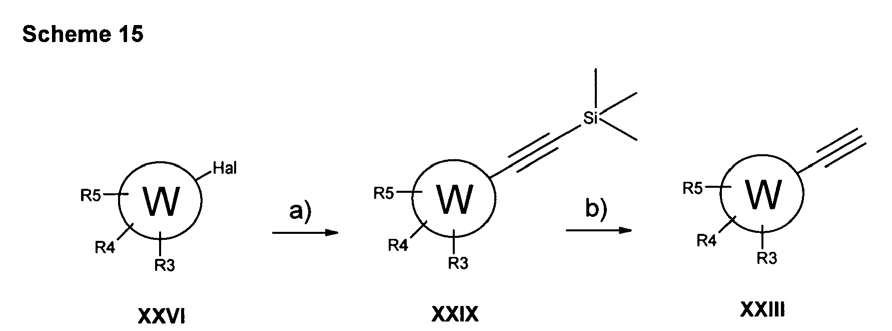

**Reagents:** a) Pd(PPh₃)₂Cl_{2,} TBAF, neat or THF; b) Pd(PPh₃)₂Cl₂, Cul, Et₂NH; c) TBAF

The carboxylic acid derivatives of formula XXXII can in principle be prepared according to Scheme 16 via a Suzuki type coupling of boronic acid XXX or XXXIII with their corresponding aryl halides XXIV or XXVI with subsequent hydrolysis of the resulting carboxylic esters XXXI.

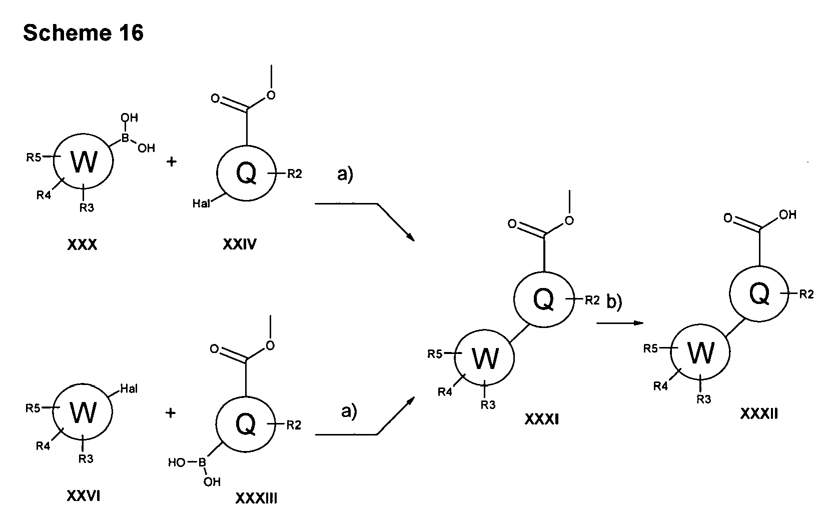

**Reagents:** a) Pd(PPh₃)₄, Na₂CO₃, H₂O, EtOH, toluene; b) KOH, MeOH.

Compounds of formula XXXV can in principle be prepared according to Scheme 17 via a Sonogashira type coupling of acetylene XXIII and aryl halide XXXIV.

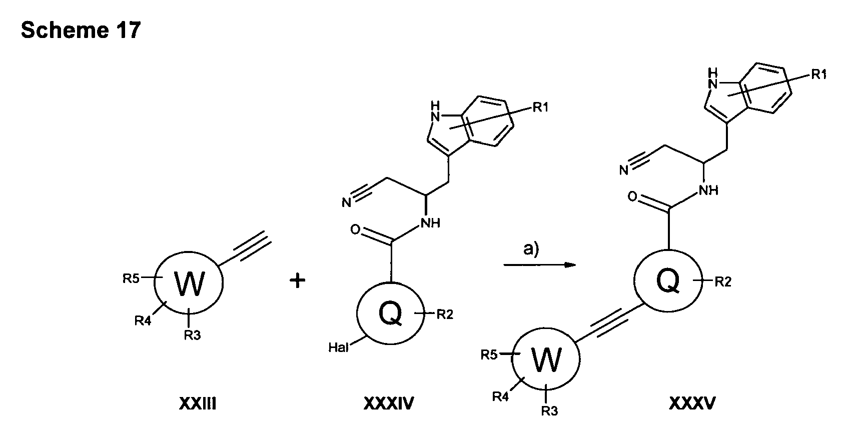

**Reagents:** a) Pd(PPh₃)₂Cl₂, TBAF, neat or THF; b) Pd(PPh₃)₂Cl₂, Cul, Et₂NH.

Compounds of formula XXXVI can in principle be prepared according to Scheme 18 via a Suzuki type coupling of boronic acid XXX and aryl halide XXXIV.

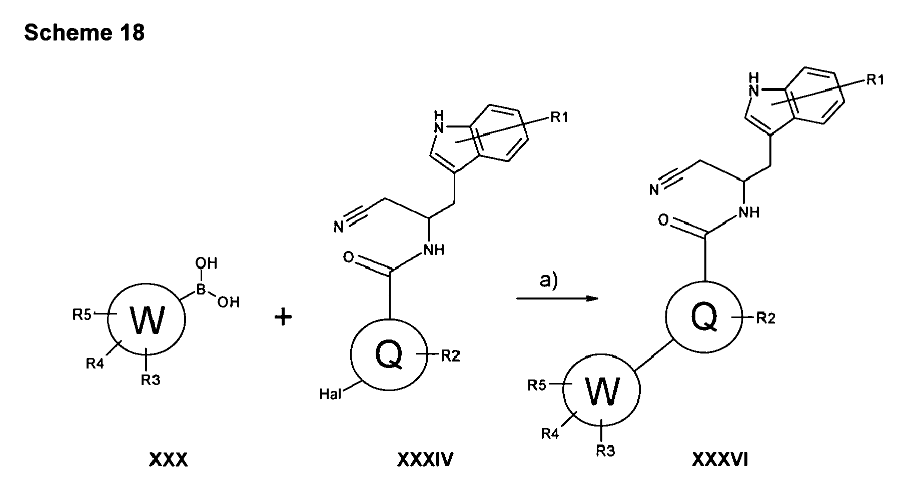

**Reagents:** a) Pd(PPh₃)₄, Na₂CO₃, H₂O, EtOH, toluene.

Compounds of the general formula XXXIV can in principle be prepared as shown in Scheme 19 by an amide formation reaction between an amine VI and a carboxylic acid XXXVII. The reagents typically used for the coupling are EDCI and HOBt.

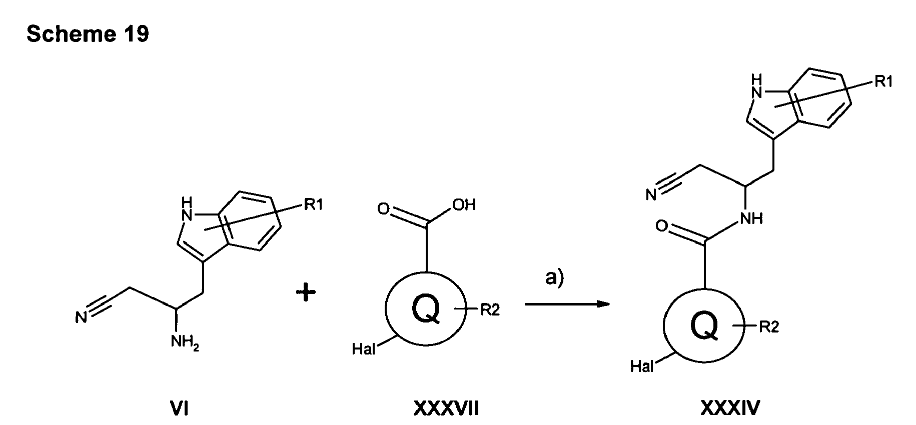

**Reagents:** a) Pd(PPh₃)₄, Na₂CO₃, H₂O, EtOH, toluene.

The compounds according to the invention of the general formula I can be prepared as described below.

### Abbreviations used:

| | |
|---|---|
| ACN | Acetonitrile |
| DIBAC | Diisobutylaluminium hydride |
| DMF | N,N-Dimethylformamide |
| EDC | *N*-Ethyl-*N*'-(3-dimethylaminopropyl)carbodiimide |
| EtOH | Ethanol |
| HATU | O-(7-Azabenzotriazol-1-yl)-N,N,N',N'-tetramethyluronium hexafluorophosphate |
| FMOC | (9H-Fluoren-9-ylmethoxy)carbonyl |
| HOBt | 1-Hydroxy-1*H*-benzotriazole |
| MeCN | Acetonitrile |
| MeOH | Methanol |
| MTBE | Methyl tert-butyl ether |
| NMM | 4-methylmorpholine |
| NMP | N-Methylpyrrolidinone |
| Rf | Reflux |
| RT | Room temperature |
| TBAF | Tetrabutylammonium fluoride |
| TFA | Trifluoroacetic acid |
| THF | Tetrahydrofuran |

### Synthesis of the compounds according to the invention

### Example 1

### 5'-Fluoro-4-isopropoxy-biphenyl-3,3'-dicarboxylic acid 3-{[2-cyano-1-(1H-indol-3-ylmethyl)-ethyl]-amide}3'-methylamide

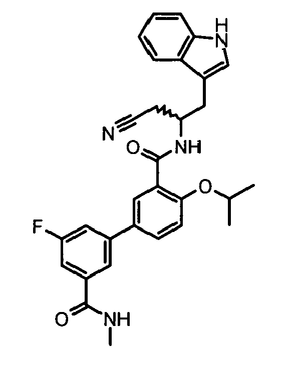

### 1a) 5-lodo-2-isopropoxy-benzoic acid methyl ester

A suspension of 5-lodo-2-hydroxy benzoic acid methyl ester (50 g), potassium carbonate (74,6 g) and iso-propyl iodide (89,9 ml) in acetone (500 ml) was stirred under reflux overnight. The reaction mixture was allowed to cool down to ambient temperature and the solid was removed by filtration. The filtrate was evaporated and the title compound was obtained in 96 % yield (55,1 g). **¹H-NMR (CDCl₃):** 8.02 d (*J* = 2.3 Hz, 1 H); 7.67 dd (*J* = 2.5 Hz / 8.9 Hz, 1 H); 6.74 d (*J* = 8.9 Hz, 1 H); 4.54 m (1 H); 3.87 s (3H); 1.36 m (6H).

### 1 b) 3'-Fluoro-4-isopropoxy-5'-methylcarbamoyl-biphenyl-3-carboxylic acid methyl ester

A solution of 5-lodo-2-isopropoxy-benzoic acid methyl ester (13.6 g), 3-Fluoro-5-methylcarbamoyl benzene boronic acid (9 g) and Pd(PPh₃)₄ (960 mg) in ethanol (150 mL), toluene (200 mL) and an aqueous sodium carbonate solution (2M, 45 mL) were stirred at reflux for 4 h. The solvent was evaporated and the title compound was obtained as crude product in 17g yield. The compound was used without further purification in the next step.

### 1 c) 3'-Fluoro-4-isopropoxy-5'-methylcarbamoyl-biphenyl-3-carboxylic acid

A solution of 3'-Fluoro-4-isopropoxy-5'-methylcarbamoyl-biphenyl-3-carboxylic acid methyl ester (2g), KOH (4 g) in methanol (30 mL) was stirred at ambient temperature overnight. The solvent was distilled off under reduced pressure and the residue was extracted with water / ether. The water phase was acidified by addition of HCl (4N) and the precipitate filtered off. The title compound was obtained in 70% yield. **¹H-NMR (CDCl₃):** 8.40 d (*J* = 2.5 Hz, 1 H); 7.77 dd (*J* = 2.5 Hz / 8.6 Hz, 1 H); 7.73 m (1 H); 7.47 m (1 H); 7.38 m (1H); 7.14 d (*J* = 8.6 Hz, 1 H); 6.49 s (1 H); 4.93 m (1 H); 3.04 d (*J* = 4.8 Hz, 3H); 1.52 d (*J* = 6.1 Hz, 6H).

### 1d) Toluene-4-sulfonic acid 3-(1H-indol-3-yl)-2-(toluene-4-sulfonylamino)-propyl ester

A solution of 2-Amino-3-(1H-indol-3-yl)-propan-1-ol (10g) in pryridin (50 ml) was cooled to 0°C and tosyl chloride (35 g) was added portionwise. The reaction mixture was allowed to warm to ambient temperature overnight and then poured into brine (100 ml). The mixture was filtrated, washed with dichloromethan and the layers of the filtrate were sepated. The aqueous phase was extracted with dichloromethane (3x) and the combined organic layer were washed with HCl (4x, 2N), brine (1x) and then dried over magnesium sulphate. The solvent was removed and the title compound was obtained in 92% yield. **¹H-NMR (DMSO-d₆):** 10.68 s (1 H); 7.93 d (*J* = 7.4 Hz, 1 H); 7.59 d (*J* = 8.5 Hz, 2H); 7.40 d (*J* = 8.3 Hz, 2H); 7.35 d (*J* = 7.9 Hz, 2H); 7.23 d (*J* = 7.9 Hz, 1 H); 7.09 d (*J* = 8.1 Hz, 2H); 6.97 m (2H); 6.84 m (2H); 3.84 m (2H); 2.74 m (1 H); 2.69 m (1 H); 2.37 s (3H); 2.27 s (3H).

### 1e) N-[2-Cyano-1-(1H-indol-3-ylmethyl)-ethyl]-4-methyl-benzenesulfonamide

A suspension of Toluene-4-sulfonic acid 3-(1H-indol-3-yl)-2-(toluene-4-sulfonylamino)-propyl ester (24.05 g) and KCN (6.28 g) in MeOH (350 ml) was stirred under reflux for 5 hours, cooled to ambient temperature and diluted with ethyl acetate (400 ml) and brine (200 ml). The phases were separated and the organic layer was extracted with brine (6x70 ml). The organic layer was dried over magnesium sulphate and the solvent was removed under reduced pressure to yield the title compound in 88% yield. The product was used without further purification in the next step. **¹H-NMR (DMSO-d₆):** 10.76 s (1H); 8.10 s (1H); 7.48 d (*J* = 8.3 Hz, 2H); 7.23 d (*J* = 8.1 Hz, 1 H); 7.14 d (*J* = 8.1 Hz, 2H); 7.04 m (3H); 6.83 m (1 H); 3.45 m (1 H); 2.72 m (2H); 2.61 m (1 H); 2.53 m (1 H); 2.28 s (3H).

### 1f) 3-Amino-4-(1H-indol-3-yl)-butyronitrile

Liquid ammonia (ca. 350 ml) was condensed onto a solution of -[2-Cyano-1-(1H-indol-3-ylmethyl)-ethyl]-4-methyl-benzenesulfonamide (15.5 g) in anhydrous THF (115 ml) at - 78 °C. Sodium metal (ca. 4 g) was added in small portions until a deep turquoise colour persists, and the reaction mix was stirred for 30 min. at -78 °C. The reaction mixture was then quenched by addition of MeOH (72 ml) and then ammonium chloride (7.2 g) and the ammonia was allowed to evaporate in the fume cupboard. The residual solvents were removed in vacuo. The residue was partitioned between ethyl acetate and 2M HCl. The aqueous phase was basified (to pH 8) with 2M NH₄OH, then extracted 3 times with ethyl acetate. Combined organics were washed (brine), dried (MgSO₄) and reduced in vacuo to give the title compound as an orange foam (5.94 g). **¹H-NMR (DMSO-d₆):** 10.87 s (1H); 7.50 d (*J* = 7.7 Hz, 1 H); 7.31 d (*J* = 8.9 Hz, 1 H); 7.14 d (*J* = 2.3 Hz, 1 H); 7.03 m (1 H); 6.94 m (1 H); 3.21 m (1H); 2.74 m (2H); 2.52 m (1 H); 2.40 m (1 H).

### 1g) 5'-Fluoro-4-isopropoxy-biphenyl-3,3'-dicarboxylic acid 3-{[2-cyano-1-(1H-indol-3-ylmethyl)-ethyl]-amide}3'-methylamide

A solution of 3'-Fluoro-4-isopropoxy-5'-methylcarbamoyl-biphenyl-3-carboxylic acid (69 mg), 3-Amino-4-(1H-indol-3-yl)-butyronitrile (50 mg), HOBt (34 mg) and EDCI (48 mg) in DMF (3 ml) was stirred overnight at ambient temperature. The reaction mixture was poured into water, extracted with ethyl acetate (3x). The combined organic layers were washed with brine and the solvent removed under vacuum. The title compound was obtained in 65 % yield after flash chromatography. **¹H-NMR (CDCl₃):** 8.60 d (*J* = 7.9 Hz, 1 H); 8.45 d (*J* = 2.5 Hz, 1 H); 8.22 s (1 H); 7.73 m (3H); 7.38 m (3H); 7.18 m (4H); 7.05 d (*J* = 8.7 Hz, 1 H); 4.80 m (2H); 3.33 m (1 H); 3.20 m (1 H); 3.05 d (*J* = 4.9 Hz, 1 H); 2.89 m (1 H); 2.57 m (1 H); 1.44 m (6H).

### Example 2

### N-[2-Cyano-1-(H-indol-3-ylmethyl)-ethyl]-5-(4-methylcarbamoyl-phenylethynyl)-2-trifluoromethoxy-benzamide

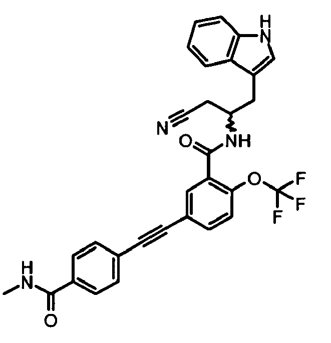

### 2a) 3-Amino-4-(1H-indol-3-yl)-butyronitrile

The title compound was prepared as described in example 1d-1f.

### 2b) 5-Bromo-2-trifluoromethoxy-benzoic acid

Under argon a solution of LDA (12.45 ml, 2M in THF) was cooled to -78°C and a solution of 1-Bromo-4-trifluoromethoxy-benzene (3.7 ml) in THF (50 ml) was added slowly. The reaction mixture was stirred at -78°C for two hours and dry ice was added in excess. The solvent was distilled off and the crude product was dissolved in aqueous NaOH (1 N), extracted with dichloromethane, then acidified with HCl and again extracted with dichloromethane. The organic layers were dried over sodium sulphate and the solvent was removed in vacuum. The title compound was obtained in 49% yield. **¹H-NMR (DMSO-d₆):** 13.78 s (1 H); 8.05 d (*J* = 2.6 Hz, 1 H); 7.92 dd (*J* = 2.6 Hz / 8.9 Hz, 1 H); 7.47 dd (*J* = 1.3 Hz / 8.9, 1 H).

### 2c) 5-Bromo-2-trifluoromethoxy-benzoic acid methyl ester

To a solution of 5-Bromo-2-trifluoromethoxy-benzoic acid (200 mg) in MeOH (2 ml) was added sulphuric acid (98%, 20µl) and the reaction was stirred under reflux overnight. The reaction was concentrated, then ethyl acetate (20 ml) was added. The organic phase was washed with brine (2x), dried over sodium sulphate and the solvent distilled off to yield 89% of the title compound. **¹H-NMR (DMSO-d₆):** 8.08 d (*J* = 2.6 Hz, 1 H); 7.97 dd (*J* = 2.6 Hz / 8.7 Hz, 1 H); 7.52 m (1 H); 3.88 s (3H).

### 2d) 4-iodo-N-methylbenzenamide

41.2 g (0.166 mole) of 4-iodobenzoic acid was put in a flask, 28 ml of redistilled thionyl chloride and 30 ml of benzene was added, and mixture was heated gently for 5-6 hr. than SOCl₂ and toluene was removed by the way of distillation. Hot residue was added with stirring to the mixture of a 10% aqueous sodium hydroxide, containing 7 g NaOH and 62 ml of 20% aqueous methylamine. The solution was stirred for 30 min, than it was cooled down with ice and the product was collected by filtration, washed with water, dried and crystallized from a water/ethanol (1:1). Yield 85 %.

### 2e) N-Methyl-4-trimethylsilanylethynyl-benzamide

A mixture of 4-iodo-N-methylbenzenamide (26.1 g, 0.1 mole), 2.5 g of PdCl₂(PPh₃)₂ (3,5%) and 1.6 g Cul (8%) in 200 ml of toluene was stirred for 30 min under argon. Than 1-ethynyl(trimethyl)silane (10 g, 0.102 mole) and Et₃N (40.4 g, 0.4 mole) was added. This mixture was stirred for 2-3 hours under argon at r.t. Sub products were filtered off. The solvent was evaporated product was purified by flash chromatography with CHCl₃. Chloroform was evaporated and product was crystallized from hexane. Yield - 17.65 g (75.7%)

### 2f) 4-Ethynyl-N-methyl-benzamide

17.65 g of N-methyl-4-[2-(1,1,1-trimethylsilyl)-1-ethynyl]benzamide (0.076 mole) was treated with 36 g of Bu₄NF*H₂O (0.115 mole) in 200 ml of THF at r.t. for 2 hours. The solvent was evaporated. Than the product was purified using flash chromatography (eluent CHCl₃). Yield - 7.67 g (63%); **ESI-MS [M+1]:** 160.

### 2g) 5-(4-Methylcarbamoyl-phenylethynyl)-2-trifluoromethoxy-benzoic acid methyl ester:

A suspension of 4-Ethynyl-N-methyl-benzamide (2.5 g), 5-Bromo-2-trifluoromethoxy-benzoic acid methyl ester (6.2 g), TBAF (1 M in THF, 47.1 ml), Pd(PPh₃)₂Cl₂ (551 mg) in THF (40 ml) and ethanol (25 ml) was stirred under reflux overnight. The solvent was distilled off under reduced pressure, ethyl acetate and water were added to the reaction and the phases were separated. The organic phase was dried over sodium sulphate and the solvent distilled off. The title compound was obtained in 30% yield after flash chromatography. **¹H-NMR (DMSO-d₆):** 8.56 m (1H); 8.10 d (*J* = 2.1 Hz, 1 H); 7.92 m (3H); 7.70 d (*J* = 8.3 Hz, 2H); 7.63 m (1 H); 3.89 s (3H); 2.80 d (*J* = 4.3 Hz, 3H).

### 2h) 5-(4-Methylcarbamoyl-phenylethynyl)-2-trifluoromethoxy-benzoic acid: 5-(4-Methylcarbamoyl-phenylethynyl)-2-trifluoromethoxy-benzoic acid methyl ester

(1.78 g) and KOH (10% in MeOH, 28 mL) in MeOH (28 mL) were stirred at ambient temperature overnight. The solvent was distilled off under reduced pressure, the reaction mixture was diluted with water (200 mL) and acidified with conc. HCl. The title compound was obtained in 91 % yield after extraction with ethyl acetate and removal of the solvent. **¹H-NMR (DMSO-d₆):** 13.67 s (1 H); 8.53 m (1 H); 8.03 d (*J* = 2.1 Hz, 1 H); 7.85 m (3H); 7.64 d (*J* = 8.3 Hz, 2H); 7.53 m (1 H); 2.76 d (*J* = 4.3 Hz, 3H).

### 2i) N-[2-Cyano-1-(1H-indol-3-ylmethyl)-ethyl]-5-(4-methylcarbamoyl-phenylethynyl)-2-trifluoromethoxy-benzamide

A solution of 5-(4-Methylcarbamoyl-phenylethynyl)-2-trifluoromethoxy-benzoic acid (100 mg), 3-Amino-4-(1H-indol-3-yl)-butyronitrile (55 mg), HOBt x water (46 mg) and EDCI (58 mg) in DMF (5 ml) was stirred overnight at ambient temperature. The reaction mixture was poured into water, extracted with ethyl acetate (3x). The combined organic layers were washed with brine and the solvent removed under vacuum. The title compound was obtained in 25 % yield after flash chromatography. **¹H-NMR (DMSO-d₆):** 10.90 s (1H); 8.87 d (*J* = 8.1 Hz, 1 H); 8.53 m (1 H); 7.87 d (*J* = 8.7 Hz, 2H); 7.73 dd (*J* = 8.7 Hz / 2.3 Hz, 1 H); 7.64 d (*J* = 8.5 Hz, 2H); 7.57 d (*J* = 7.5 Hz, 1 H); 7.53 d (*J* = 2.1 Hz, 1 H); 7.49 m (1 H); 7.32 d (*J* = 7.9 Hz, 1 H); 7.20 d (*J* = 2.3 Hz, 1 H); 7.05 m (1 H); 6.97 m (1 H); 4.38 m (1 H); 2.98 m (2H); 2.78 m (1 H); 2.77 d (*J* = 4.3 Hz, 3H); 2.69 m (1 H).

The following compounds were obtained in analogy to the preparation methods described in detail:

| **Ex.** | **Product; regents** | **Method analo- gous to** | **¹H-NMR** | **Structure** |
|---|---|---|---|---|
| **3** | 3'-Chloro-4-isopropoxy-biphenyl-3,4'-dicarboxylic acid 3-{[2-cyano-1-(1H-indol-3-ylmethyl)-ethyl]-amide} 4'-methylamide; | 1 | **¹H-NMR (CDCl₃):** 8.59 d (*J* = 7.8 Hz, 1 H); 8.47 d (*J* = 2.5 Hz, 1 H); 8.16 s (1 H); 7.79 d (*J* = 8.1 Hz, 1 H); 7.72 d (*J* = 7.8 Hz, 1 H); 7.66 m (1H); 7.57 dd (*J* = 8.1 Hz / 1.8 Hz, 1 H); 7.39 d (*J* = 8.1 Hz, 1 H); 7.23 m (1 H); 7.18 m (2H); 7.07 d (*J* = 8.8 Hz, 1 H); 6.35 m (1 H); 4.81 m (2H); 3.34 m (1 H); 3.19 m (1H); 3.05 d (*J* = 4.8 Hz, 3H); 2.88 m (1 H); 2.57 m (1 H); 1.44 m (6H). | 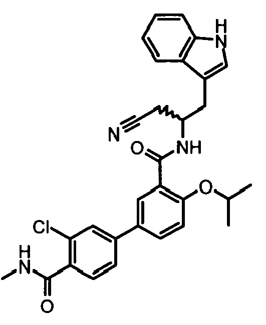 |
| | | | | |
| | *3-Amino-4-(1H-indo*/*-3-yl)-butyronitrile* | | | |
| | | | | |
| | *and* | | | |
| | | | | |
| | *3'-Chloro-4-isoprop-oxy-4'-methylcarba-moyl-biphenyl-3-carboxylic acid* | | | |
| **4** | 5-(4-Carbamoyl-2-methylphenylethynyl)-N-[2-cyano-1-(1H-indol-3-ylmethyl)-ethyl]-2-trifluoro-methoxy-benzamide; | **2** | **¹H-NMR (DMSO-d₆):** 10.91 s (1 H); 8.89 d (*J* = 7.9 Hz, 1 H); 8.00 s (1 H); 7.82 s (1H); 7.74 m (3H); 7.54 m (5H); 7.32 d (*J* = 7.9 Hz, 1H); 7.19 d (*J* = 2.1 Hz, 1 H); 7.04 m (1 H); 6.97 m (1H); 4.39 m (1H); 2.99 m (2H); 2.80 m (1 H); 2.72 m (1H); 2.48 s (3H). | 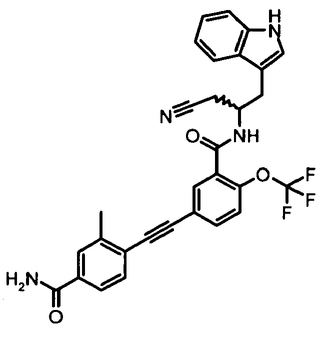 |
| | | | | |
| | *3-Amino-4-(1H-indol-3-yl)-butyronitrile* | | | |
| | | | | |
| | *and* | | | |
| | | | | |
| | *5-(4-Carbamoyl-2-methyl-phenylethyn-yl)-2-trifluoromethoxy-benzoic acid* | | | |

### Example 5

### 3'-Chloro-4-isopropoxy-biphenyl-3,4'-dicarboxylic acid 4'-amide 3-{[2-cyano-1-(1H-indol-3-ylmethyl)-ethyl]-amide}

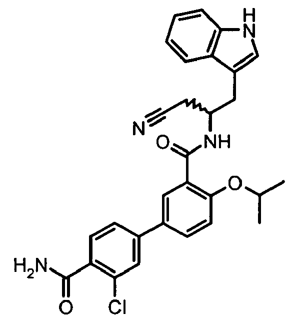

### 5a) 5-lodo-2-isopropoxy-benzoic acid methyl ester

The title compound was prepared as described in example 1 a.

### 5b) 5-lodo-2-isopropoxy-benzoic acid

5-lodo-2-isopropoxy-benzoic acid methyl ester (10g) and KOH (10% in MeOH, 30 mL) in MeOH (200 mL) were stirred at ambient temperature overnight. The reaction mixture was diluted with water (200 mL) and acidified with conc. HCl. The title compound was obtained in 97% yield after filtration of the precipitate and drying in vacuum. **¹H-NMR (DMSO-d₆):** 12.77 s (1 H); 7.78 d (*J* = 2.3 Hz, 1 H); 7.70 dd (*J* = 2.3 Hz / 8.8 Hz, 1 H); 6.94 d (*J* = 8.8 Hz, 1 H); 4.59 m (1 H); 1.21 d (*J* = 6.1 Hz, 1 H).

### 5c) 3-Amino-4-(1H-indol-3-yl)-butyronitrile

The title compound was prepared as described in example 1d-1f.

### 5d) N-[2-Cyano-1-(1H-indol-3-ylmethyl)-ethyl]-5-iodo-2-isopropoxy-benzamide

5-lodo-2-isopropoxy-benzoic acid (1.64 g), 3-Amino-4-(1H-indol-3-yl)-butyronitrile (1.17 g), EDCl (1.23 g), HOBt (0.87 g) and triethylamine (1.11 mL) in DMF (20 mL) were stirred at ambient temperature for 48 hours. The reaction mixture was poured into water, extracted with ethyl acetate (3x). The combined organic layers were washed with brine and the solvent removed under vacuum. The title compound was obtained in 54 % yield after flash chromatography. **¹H-NMR (DMSO-d₆):** 10.89 s (1 H); 8.31 d (*J* = 8.3 Hz, 1 H); 7.88 d (*J* = 2.5 Hz, 1 H); 7.69 dd (*J* = 8.7 Hz / 2.5 Hz, 1 H); 7.54 d (*J* = 7.7 Hz, 1 H); 7.31 d (*J* = 7.9 Hz, 1 H); 7.16 d (*J* = 2.3 Hz, 1 H); 7.04 m (1 H); 6.95 m (2H); 4.67 m (1 H); 4.50 m (1 H); 3.00 m (2H); 2.84 m (1 H); 2.71 m (1 H); 1.18 m (6H).

### 5e) 3'-Chloro-4-isopropoxy-biphenyl-3,4'-dicarboxylic acid 4'-amide 3-{[2-cyano-1-(1H-indol-3-ylmethyl)-ethyl]-amide} A solution of N-[2-Cyano-1-(1H-indol-3-ylmethyl)-ethyl]-5-iodo-2-isopropoxy-benzamide

5-lodo-2-isopropoxy-benzoic acid (100 mg), 3-Chloro-4-carbamoyl benzene boronic acid (41 mg) and Pd(PPh₃)₄ (12 mg) in ethanol (0.5 mL), toluene (1.5 mL) and an aqueous sodium carbonate solution (2M, 0.31 mL) were heated in a microwave reactor for 20 minutes at 120°C. The solvent was evaporated and after flash chromatography the title compound was obtained in 48% yield. **¹H-NMR (DMSO-d₆):** 10.90 s (1 H); 8.39 d (*J* = 8.1 Hz, 1 H); 7.97 d (*J* = 2.6 Hz, 1 H); 7.86 s (1 H); 7.80 dd (*J* = 8.7 Hz / 2.6 Hz, 1 H); 7.68 d (*J* = 1.7 Hz, 1 H); 7.57 m (3H); 7.48 d (*J* = 8.1 Hz, 1 H); 7.32 d (*J* = 8.1 Hz, 1 H); 7.23 d (*J* = 8.7 Hz, 1 H); 7.18 d (*J* = 2.3 Hz, 1 H); 7.04 m (1 H); 6.95 m (1 H); 4.78 m (1 H); 4.54 m (1 H); 3.04 m (2H); 3.01 m (1 H); 2.74 m (1 H); 1.23 m (6H).

The following compounds were obtained in analogy to the preparation methods described in detail:

| | **Product; Ex. reagents** | **Method analogous to** | **¹H-NMR** | **Structure** |
|---|---|---|---|---|
| **6** | 3'-Chloro-4-isopropoxy-4'-(morpholine-4-carbonyl)-biphenyl-3-carboxylic acid [2-cyano-1-(1H-indol-3-ylmethyl)-ethyl]-amide; | **5** | **¹H-NMR (DMSO-d₆):** 10.90 s (1H); 8.39 d (*J* = 8.1 Hz, 1 H); 7.97 d (*J* = 2.5 Hz, 1 H); 7.80 dd (*J* = 8.7 Hz / 2.6 Hz, 1 H); 7.72 d (*J* = 1.7 Hz, 1 H); 7.59 m (2H); 7.42 d (*J* = 7.9 Hz, 1H); 7.31 d (*J* = 7.9 Hz, 1 H); 7.23 d (*J* = 9.0 Hz, 1H); 7.18 d (*J* = 2.3 Hz, 1 H); 7.04 m (1H); 6.95 m (1H); 4.78 m (1H); 4.54 m (1 H); 3.63 m (4H); 3.52 m (2H); 3.14 m (2H); 3.04 m (2H); 2.88 m (1H); 2.74 m (1 H); 1.25 m (6H). | 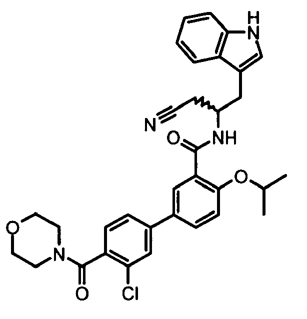 |
| | *N-[2-Cyano-1-(1H-indol-3-ylmethyl)-ethyl]-5-iodo-2-iso-propoxy-benzamide* | | | |
| | *and* | | | |
| | *N-Morpholinyl 2-chloro-4-borono-benzamide* | | | |
| **7** | 4-Isopropoxy-3',4'-dimethoxy-biphenyl-3-carboxylic acid [2-cyano-1-(1H-indol-3-ylmethyl)-ethyl]-amide; | **5** | **¹H-NMR (DMSO-d₆):** 10.90 s (1H); 8.42 d (*J* = 8.3 Hz, 1 H); 7.95 d (*J* = 2.6 Hz, 1 H); 7.68 dd (*J* = 8.7 Hz / 2.6 Hz, 1H); 7.58 d (*J* = 7.9 Hz, 1 H); 7.31 d (*J* = 8.1 Hz, 1 H); 7.19 m (2H); 7.11 s (1 H); 7.08-6.93 m (4H); 4.75 m (1H); 4.52 m (1H); 3.80 s (3H); 3.75 s (3H); 3.03 m (2H); 2.88 m (1H); 2.77 m (1H); 1.24 m (6H). | 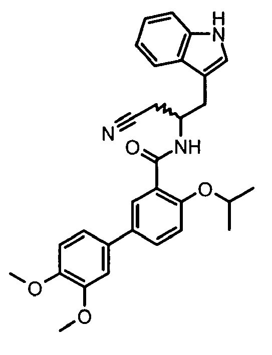 |
| | *N-[2-Cyano-1-(1H-indol-3-ylmethyl)-ethyl]-5-iodo-2-iso-propoxy-benzamide* | | | |
| | *and* | | | |
| | *3, 4-Dimethoxyphenyl boronic acid* | | | |

### Example 8

### 5-(4-Carbamoyl-phenylethynyl)-N-[2-cyano-1-(1H-indol-3-ylmethyl)-ethyl]-2-isopropoxy-benzamide

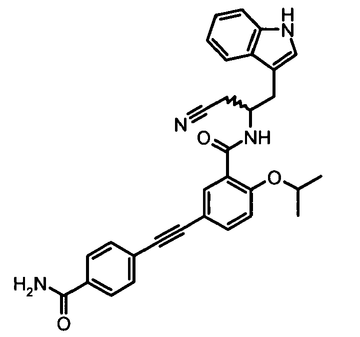

### 8a) N-[2-Cyano-1-(1H-indol-3-ylmethyl)-ethyl]-5-iodo-2-isopropoxy-benzamide

The title compound was prepared as described in examples 5a-5b.

### 8b) 4-Ethynyl-benzamide

The title compound was prepared in analogy to the examples 2d-2f.

### 8c) 5-(4-Carbamoyl-phenylethynyl)-N-[2-cyano-1-(11H-indol-3-ylmethyl)-ethyl]-2-isopropoxy-benzamide

A mixture of N-[2-Cyano-1-(1H-indol-3-ylmethyl)-ethyl]-5-iodo-2-isopropoxy-benzamide (100 mg), 4-Ethynyl-benzamide (30 mg), Pd(PPh₃)₂Cl₂ (7.2 mg), TBAF (0.62 ml, 1M in THF) in THF (2 ml) and ethanol (0.5 ml) was heated in a microwave oven at 110°C for 20 minutes. The reaction mixture was diluted with ethyl acetate and extracted with water. The solvent of the organic layer was removed under reduced pressure and the title compound was obtained after flash chromatography in 33% yield. **¹H-NMR (DMSO-d₆):** 10.90 s (1H); 8.34 d (*J* = 8.1 Hz, 1 H); 8.02 s (1H); 7.87 d (*J* = 8.1 Hz, 2H); 7.80 d (*J* = 2.3 Hz, 1 H); 7.60 m (4H); 7.42 s (1 H); 7.32 d (*J* = 8.1 Hz, 1 H); 7.19 m (2H); 7.05 m (1 H); 6.96 m (1 H); 4.77 m (1 H); 4.52 m (1 H); 3.01 m (2H); 2.86 m (1 H); 2.75 m (1 H); 1.22 m (6H).

The following compounds were obtained in analogy to the preparation methods described in detail:

| **Ex.** | **Product; reagents** | **Method analogous to** | **1H-NMR** | **Structure** |
|---|---|---|---|---|
| **9** | N-[2-Cyano-1-(1H-indol-3-ylmethyl)-ethyl]-2-isopropoxy-5-(4-methylcarbamoyl-phenylethynyl)-benzamide; | **8** | **¹H-NMR (DMSO-d₆):** 10.90 s (1H); 8.49 m (1 H); 8.34 d (*J* = 8.1 Hz, 1 H); 7.83 d (*J* = 8.3 Hz, 2H); 7.80 d (*J* = 2.3 Hz, 1 H); 7.60 m (4H); 7.32 d (*J* = | 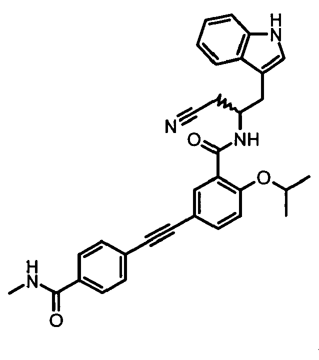 |
| | *N-[2-Cyano-1-(1H-indol-3-ylmethyl)-ethyl]-5-iodo-2-iso-propoxy-benzamide* | | 8.1 Hz, 1 H); 7.18 m (2H); 7.04 m (1H); 6.96 m (1 H); 4.78 m (1H); 4.52 m (1 H); 3.01 m (2H); 2.86 m (1 H); | |
| | *and* | | 2.75 d (*J* = 4.3 Hz, 1 H); 2.72 m (1H); 1.22 m (6H). | |
| | 4-Ethynyl-N-methyl-benzamide | | | |
| **10** | N-[2-Cyano-1-(1H-indol-3-ylmethyl)-ethyl]-2-isopropoxy-5-(3-methylcarbamoyl-phenylethynyl)-benzamide; | **8** | **¹H-NMR (DMSO-d₆):** 10.90 s (1H); 8.51 m (1 H); 8.34 d (*J* = 8.1 Hz, 1 H); 7.96 m (1 H); 7.80 m (2H); 7.65 m (1H); 7.61 dd (*J* = 2.3 Hz / 8.8 Hz, 1 H); 7.57 | 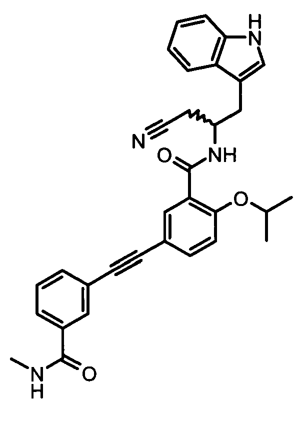 |
| | *N-[2-Cyano-1-(1H-indol-3-ylmethyl)-ethyl]-5-iodo-2-iso-propoxy-benzamide* | | d (*J* = 7.6 Hz, 1 H); 7.49 m (1 H); 7.32 d (*J* = 8.1 Hz, 1 H); 7.18 m (2H); 7.05 m (1 H); 6.96 m (1H); 4.76 m | |
| | *and* | | (1 H); 4.52 m (1H); 3.01 m (2H); 2.86 m (1H); 2.75 d | |
| | 3-Ethynyl-N-methyl-benzamide | | (*J* = 4.3 Hz, 1 H); 2.73 m (1 H); 1.22 m (6H). | |
| **11** | N-[2-Cyano-1-(1H-indol-3-ylmethyl)-ethyl]-2-isopropoxy-5-(2-methylcarbamoyl-phenylethynyl)-benzamide; | **8** | **¹H-NMR (DMSO-d₆):** 10.90 s (1H); 8.39 d (*J* = 8.1 Hz, 1 H); 7.83 d (*J* = 2.5 Hz, 1 H); 7.75 d (*J* = 7.6 Hz, 1 H); 7.55 m (4H); 7.44 m (1H); 7.31 d (*J*= 8.1 Hz, 1 H); 7.22 d (*J* = 8.6 Hz, 1 H); 7.19 d (*J* = 2.3 Hz, 1 H); 7.04 m (1 H); 6.94 m (1H); 4.78 m (1 H); 4.53 m (1H); 3.20 s (3H); 3.03 m (2H); 2.87 m (2H); 2.76 m (2H); 1.25 m (6H). | 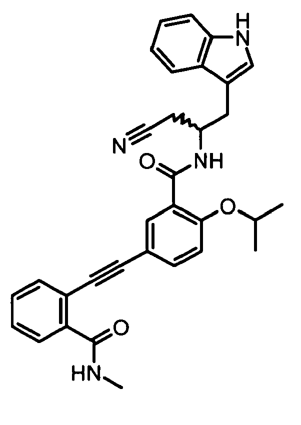 |
| | *N-[2-Cyano-1-(1H-indol-3-ylmethyl)-ethyl]-5-iodo-2-iso-propoxy-benzamide* | | | |
| | *and* | | | |
| | 2-Ethynyl-N-methyl-benzamide | | | |

### Example 12

### 4'-Morpholin-4-yl-4-trifluoromethoxy-biphenyl-3-carboxylic acid [2-cyano-1-(1H-indol-3-ylmethyl)-ethyl]-amide

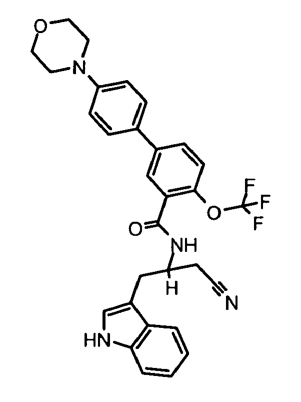

### 12a) 5-Bromo-2-trifluoromethoxy-benzoic acid

The title compound was prepared as described in example 2b.

### 12b) 3-Amino-4-(1H-indol-3-yl)-butyronitrile

The title compound was prepared as described in examples 1d-1f.

### 12c) N-[2-Cyano-1-(1H-indol-3-ylmethyl)-ethyl]-5-bromo-2-trifluoromethoxy-benzamide

A solution of 5-Bromo-2-trifluoromethoxy-benzoic acid (2.5 g), 3-Amino-4-(1H-indol-3-yl)-butyronitrile (1.75 g), HOBt x water (1.48 g) and EDCl (1.85 g) in DMF (20 ml) was stirred overnight at ambient temperature. The reaction mixture was poured into water, extracted with ethyl acetate (3x). The combined organic layers were washed with brine and the solvent removed under vacuum. The title compound was obtained in 46 % yield after flash chromatography. **¹H-NMR (DMSO-d₆):** 10.89 s (1 H); 8.87 d (*J* = 7.9 Hz, 1 H); 7.76 dd (*J* = 2.5 Hz / 8.7 Hz, 1 H); 7.56 d (*J* = 7.9 Hz, 1 H); 7.50 d (*J* = 2.6 Hz, 1 H); 7.39 dd (*J* = 1.5 Hz / 8.9 Hz, 1 H); 7.32 d (*J* = 8.1 Hz, 1H); 7.18 d (*J* = 2.3 Hz, 1 H); 7.05 m (1 H); 6.97 m (1 H); 4.35 m (1 H); 2.97 m (2H); 2.78 m (1 H); 2.68 m (1 H).

### 12d) 4'-Morpholin-4-yl-4-trifluoromethoxy-biphenyl-3-carboxylic acid [2-cyano-1-(1H-indol-3-ylmethyl)-ethyl]-amide

To 0.1 mmol of N-[2-Cyano-1-(1H-indol-3-ylmethyl)-ethyl]-5-bromo-2-trifluoromethoxy-benzamide (0.2 M in THF) in a microwave vessel are added 1.5 eq of 4-Morpholin-4-yl-benzene boronic acid (0.4 M in THF), 3 eq of K₂CO₃ (1M in H₂O), 0.1 eq Pd(OAc)₂ (0.0375 M in THF), 0.2 eq Tri(o-tolylphosphin) (0.5 M in THF) and 100 µl of H₂O. The mixture is heated to 130°C under stirring in a microwave oven for 1 h. After cooling, the solution is evaporated, redissolved in DMSO, filtered and subjected to preparative HPLC chromatography. HPLC purification: Machine: Analytical 4 channel MUX system with CTC Pal injector, Waters 1525 Pumps, Waters 2488 UV detector and Waters ZQ 2000 single quad MS detector. Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1 ): 550, retention time: 4.07 min.

The following compounds were obtained in analogy to the preparation methods described in detail:

| **Ex.** | **Product; reagents** | **Method analogous to** | **HPLC-US** | **Structure** |
|---|---|---|---|---|
| **13** | 4'-Methanesulfonyl-amino-methyl)-4-trifluoromethoxy-bi- phenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1 % (1.75'). Molecular peak (ESI, M+1): 572 Retention time: 3.89 min. | 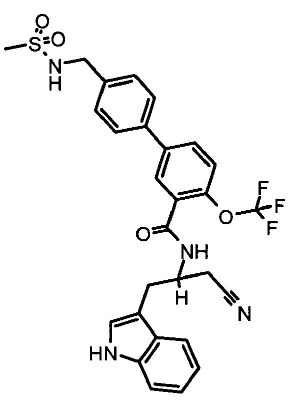 |
| | *N-[2-Cyano-1-(1H-indol-3-ylmethyl)-ethyl]-5-iodo-2-tri-fluoromethoxy-benzamide* | | | |
| | *and* | | | |
| | *4-(Methylsulphonyl-aminomethyl)-benzene boronic acid* | | | |
| **14** | N-{3'-[2-Cyano-1-(1H-indol-3-ylmethyl)-ethylcarbamoyl]-4'-trifluoromethoxy-biphenyl-3-yl}-succinamic acid; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 580 Retention time: 3.71 min. | 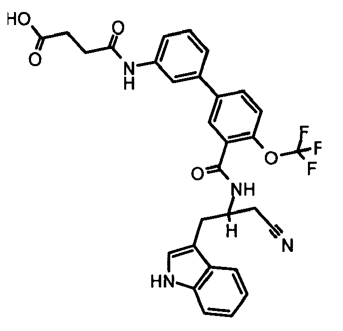 |
| | *N-[2-Cyano-1-(1H-indol-3-ylmethyl)-ethyl]-5-iodo-2-tri-fluoromethoxy-benzamide* | | | |
| | *and* | | | |
| | *3-(3-Carboxypropionylamino)-phenyl boronic acid* | | | |
| **15** | 4'-(Pyrrolidine-1-carbonyl)-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1 % (1.75'). Molecular peak (ESI, M+1): 562 Retention time: 3.93 min. | 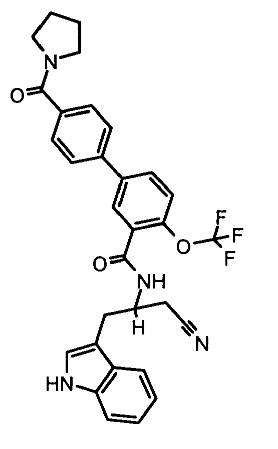 |
| | *N-[2-Cyano-1-(1H-indol-3-ylmethyl)-ethyl]-5-iodo-2-trifluoromethoxy-benzamide* | | | |
| | *and* | | | |
| | *N-Pyrrolidinyl 4-borono-benzamide* | | | |
| **16** | N-[2-Cyano-1-(1H-indol-3-ylmethyl)-ethyl]-5-(1-methyl-1H-indol-5-yl)-2-trifluoromethoxy-benzamide; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1 % (1.75'). Molecular peak (ESI, M+1): 518 Retention time: 4.43 min. | 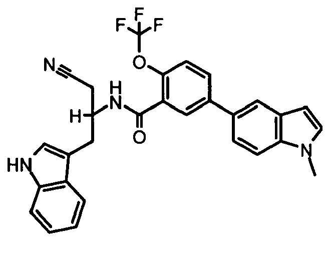 |
| | *N-[2-Cyano-1-(1H-indol-3-ylmethyl)-ethyl]-5-iodo-2-trifluoromethoxy-benzamide* | | | |
| | *and* | | | |
| | *1-Methylindol-5-boronic acid* | | | |
| **17** | 4-Trifluoromethoxy-biphenyl-3,4'-dicarboxylic acid 3-{[2-cyano-1-(1H-indol-3-ylmethyl)-ethyl]-amide} 4'-[(2-methoxy-ethyl)-amide]; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 566 Retention time: 3.74 min. | 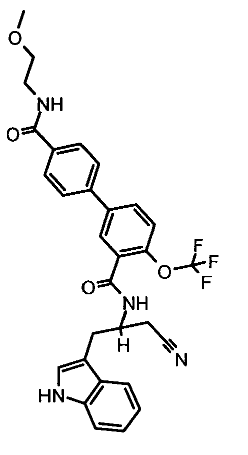 |
| | *N-[2-Cyano-1-(1H-indol-3-ylmethyl)-ethyl]-5-iodo-2-trifluoromethoxy-benzamide* | | | |
| | *and* | | | |
| | *4-(Methoxyethyl)-carbamoyl-benzene boronic acid* | | | |
| **18** | 3'-(Pyrrolidine-1-carbonyl)-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 562 Retention time: 3.96 min. | 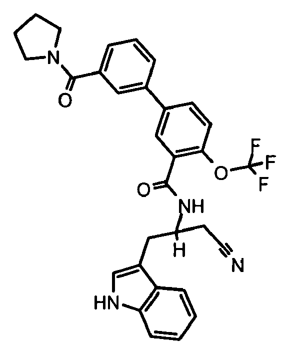 |
| | *N-[2-Cyano-1-(1H-indol-3-ylmethyl)-ethyl]-5-iodo-2-triifluoromethoxy-benzamide* | | | |
| | *and* | | | |
| | *N-Pyrrolidinyl 3-borono-benzamide* | | | |
| **19** | 4-Trifluoromethoxy-biphenyl-3,3'-di-carboxylic acid 3-{[2-cyano-1-(1H-indol-3-ylmethyl)-ethyl]-amide} 3'-ethylamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 536 Retention time: 3.93 min. | 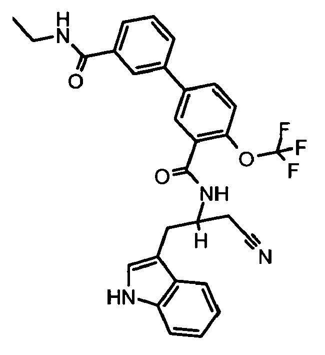 |
| | *N-[2-Cyano-1-(1H-indol-3-ylmethyl)-ethyl]-5-iodo-2-trifluoromethoxy-benzamide* | | | |
| | *and* | | | |
| | *3-Ethylcarbamoyl-benzene boronic acid* | | | |
| **20** | 4-Trifluoromethoxy-biphenyl-3,3'-dicarboxylic acid 3-{[2-cyano-1-(1H-indol-3-ylmethyl)-ethyl]-amide} 3'-(isobutylamide); | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 564 Retention time: 4.24 min. | 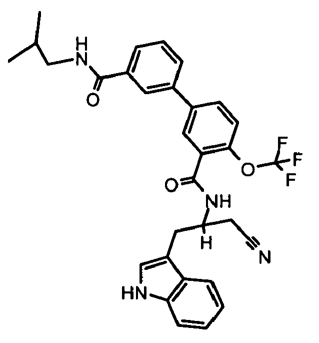 |
| | *N-[2-Cyano-1-(1H-indol-3-ylmethyl)-ethyl]-5-iodo-2-trifluoromethoxy-benzamide* | | | |
| | *and* | | | |
| | *3-Isobutylcarbamoylbenzene boronic acid* | | | |
| **21** | 4-Trifluoromethoxy-biphenyl-3,3'-dicarboxylic acid 3-{[2-cyano-1-(1H-indol-3-ylmethyl)-ethyl]-amide} 3'-cyclopentylamide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 576 Retention time: 4.31 min. | 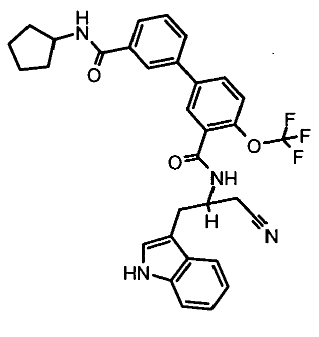 |
| | *N-[2-Cyano-1-(1H-indol-3-ylmethyl)-ethyl]-5-iodo-2-trifluoromethoxy-benzamide* | | | |
| | *and* | | | |
| | *3-Cyclopentylcarbamoyl-benzene boronic acid* | | | |
| **22** | 3'-(Methanesulfonyl-amino-methyl)-4-trifluoromethoxy-bi-phenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 572 Retention time: 3.90 min. | 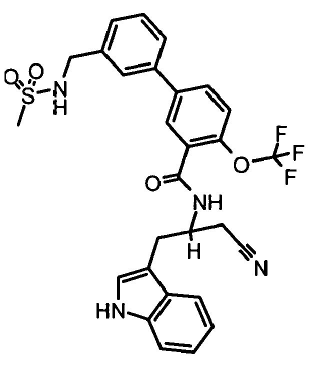 |
| | *N-[2-Cyano-1-(1H-indol-3-ylmeth yl)-ethyl]-5-iodo-2-trifluoromethoxybenzamide* | | | |
| | *and* | | | |
| | *3-Methylsulfonylaminomethyl-benzene boronic acid* | | | |
| **23** | 4'-(Acetylaminomethyl)-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 536 Retention time: 3.72 min. | 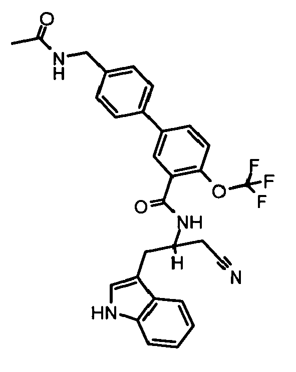 |
| | *N-[2-Cyano-1-(1H-indol-3-ylmethyl)-ethyl]-5-iodo-2-trifluoromethoxy-benzamide* | | | |
| | *and* | | | |
| | *4-Acetamidomethylbenzene boronic acid* | | | |
| **24** | 3'-(Acetylaminomethyl)-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 536 Retention time: 3.71 min. | 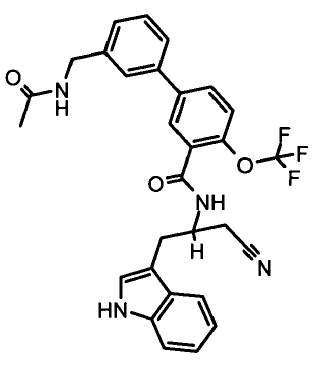 |
| | *N-[2-Cyano-1-(1H-indol-3-ylmethyl)-ethyl]-5-iodo-2-trifluoromethoxybenzamide* | | | |
| | *and* | | | |
| | *3-Acetamidomethylbenzene boronic acid* | | | |
| **25** | 4'-Methylsulfamoyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1 % (1.75'). Molecular peak (ESI, M+1): 558 Retention time: 3.91 min. | 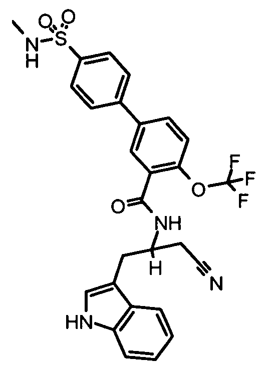 |
| | *N-[2-Cyano-1-(1H-indol-3-ylmethyl)-ethyl]-5-iodo-2-trifluoromethoxybenzamide* | | | |
| | *and* | | | |
| | *4-Methylaminosulfonyl-benzene boronic acid* | | | |
| **26** | 3-Chloro-3'-[2-cyano-1-(1H-indol-3-yl-methyl)-ethylcarbamoyl]-4'-trifluoromethoxy-biphenyl-4-carboxylic acid methyl ester; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 557 Retention time: 4.37 min. | 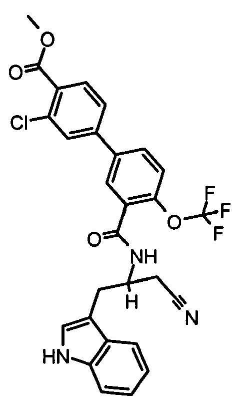 |
| | *N-[2-Cyano-1-(1H-indol-3-ylmethyl)-ethyl]-5-iodo-2-trifluoromethoxy-benzamide* | | | |
| | *and* | | | |
| | *3-Chloro-4-methyloxycarbonyl-benzene boronic acid* | | | |
| **27** | 4'-Ethylsulfamoyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1% (1.75'). Molecular peak (ESI, M+1): 572 Retention time: 4.05 min. | 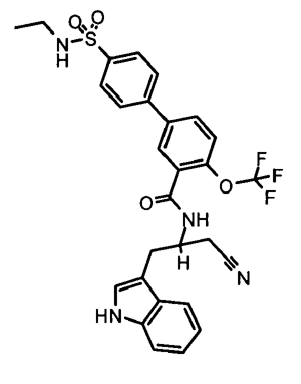 |
| | *N-[2-Cyano-1-(1H-indol-3-ylmethyl)-ethyl]-5-iodo-2-trifluoromethoxybenzamide* | | | |
| | *and* | | | |
| | *4-Ethylaminosulfonylbenzene boronic acid* | | | |
| **28** | 3'-Chloro-4-trifluoro-methoxy-biphenyl-3,4'-dicarboxylic acid 3-{[2-cyano-1-(1H-indol-3-ylmethyl)-ethyl]-amide}4'-isopropylamide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 584 Retention time: 4.09 min. | 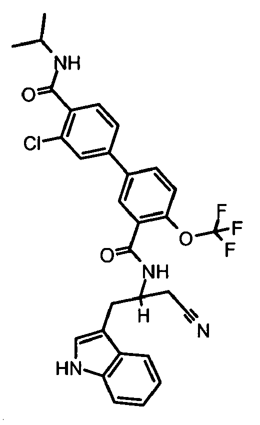 |
| | *N-[2-Cyano-1-(1H-indol-3-ylmethyl)-ethyl]-5-iodo-2-trifluoromethoxybenzamide* | | | |
| | *and* | | | |
| | *3-Chloro-4-isopropylcarbamoyl-phenylboronic acid* | | | |
| **29** | 3'-Chloro-4-trifluoro-methoxy-biphenyl-3,4'-dicarboxylic acid 3-{[2-cyano-1-(1H-indol-3-ylmethyl)-ethyl]-amide}4'-cyclopropylamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 582 Retention time: 3.90 min. | 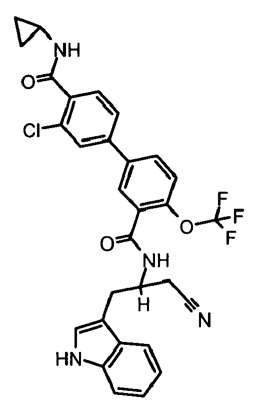 |
| | *N-[2-Cyano-1-(1H-indol-3-ylmethyl)-ethyl]-5-iodo-2-trifluoromethoxy-benzamide* | | | |
| | *and* | | | |
| | *3-Chloro-4-cyclopropylcarbamoylphenyl-boronic acid* | | | |
| **30** | 3'-Chloro-4-trifluoro-methoxy-biphenyl-3,4'-dicarboxylic acid 4'-tert-butylamide 3-{[2-cyano-1-(1H-indol-3-ylmethyl)-ethyl]-amide}; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 598 Retention time: 4.33 min. | 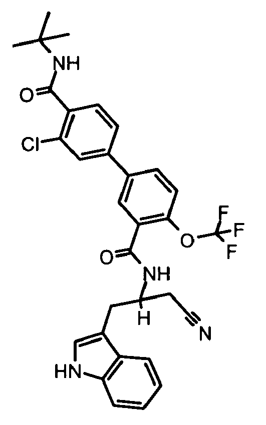 |
| | *N-[2-Cyano-1-(1H-indol-3-ylmethyl)-ethyl]-5-iodo-2-trifluoromethoxybenzamide* | | | |
| | *and* | | | |
| | *3-Chloro-4-tertbutylcarbamoyl-phenylboronic acid* | | | |
| **31** | 3'-Chloro-4-trifluoro-methoxy-biphenyl-3,4'-dicarboxylic acid 3-{[2-cyano-1-(1H-indol-3-ylmethyl)-ethyl]-amide} 4'-dimethylamide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1 % (1.75'). Molecular peak (ESI, M+1): 570 Retention time: 3.90 min. | 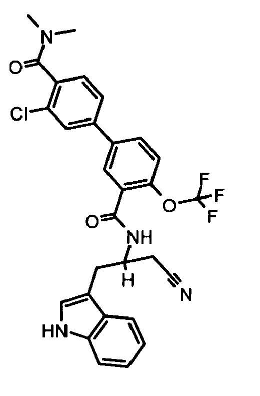 |
| | *N-[2-Cyano-1-(1H-indol-3-ylmethyl)-ethyl]-5-iodo-2-trifluoromethoxybenzamide* | | | |
| | *and* | | | |
| | *3-Chloro-4-dimethylcarbamoyl-phenylboronic acid* | | | |
| **32** | 3'-Chloro-4-trifluoromethoxy-biphenyl-3,4'-dicarboxylic acid 3-{[2-cyano-1-(1H-indol-3-ylmethyl)-ethyl]-amide} 4'-diethylamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1 % (1.75'). Molecular peak (ESI, M+1): 598 Retention time: 4.23 min. | 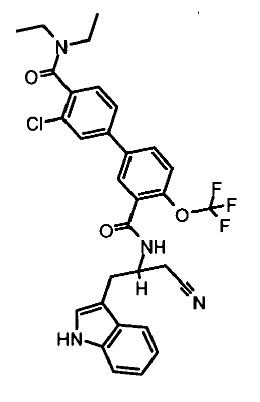 |
| | *N-[2-Cyano-1-(1H-indol-3-ylmeth yl)-ethyl]-5-iodo-2-trifluoromethoxybenzamide* | | | |
| | *and* | | | |
| | *3-Chloro-4-diethylcarbamoyl-phenylboronic acid* | | | |
| **33** | 3'-Chloro-4'-(morpholine-4-carbonyl)-4-tri-fluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 612 Retention time: 3.91 min. | 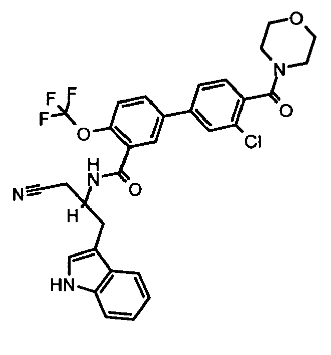 |
| | *N-[2-Cyano-1-(1H-indol-3-ylmethyl)-ethyl]-5-iodo-2-trifluoromethoxybenzamide* | | | |
| | *and* | | | |
| | *N-Morpholinyl 2-chloro-4-boronobenzamide* | | | |
| **34** | 3'-Chloro-4'-(piperidine-1-carbonyl)-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 610 Retention time: 4.29 min. | 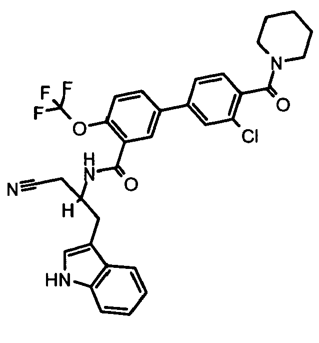 |
| | *N-[2-Cyano-1-(1H-indol-3-ylmethyl)-ethyl]-5-iodo-2-trifluoromethoxybenzamide* | | | |
| | *and* | | | |
| | *N-Piperidinyl 2-chloro-4-boronobenzamide* | | | |
| **35** | 3'-Chloro-4'-(pyrrolidine-1-carbonyl)-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1% (1.75'). Molecular peak (ESI, M+1): 596 Retention time: 4.01 min. | 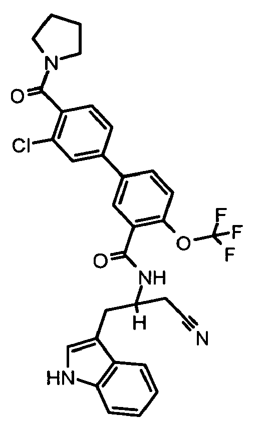 |
| | *N-[2-Cyano-1-(1H-indol-3-ylmethyl)-ethyl]-5-iodo-2-trifluoromethoxybenzamide* | | | |
| | *and* | | | |
| | *N-Pyrrolidinyl 2-chloro-4-boronobenzamide* | | | |
| **36** | 5'-Ethoxy-3'-fluoro-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 526 Retention time: 4.60 min. | 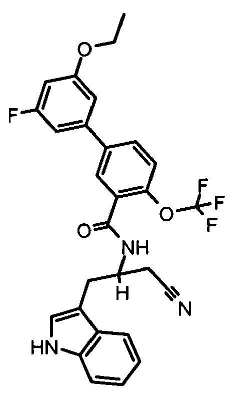 |
| | *N-[2-Cyano-1-(1H-indol-3-ylmethyl)-ethyl]-5-iodo-2-trifluoromethoxybenzamide* | | | |
| | *and* | | | |
| | *3-Fluoro-5-ethoxyphenyl boronic acid* | | | |
| **37** | 5'-Fluoro-4-trifluoromethoxy-biphenyl-3,3'-dicarboxylic acid 3-{[2-cyano-1-(1H-indol-3-ylmethyl)-ethyl]-amide} 3'-methylamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 539 Retention time: 3.86 min. | 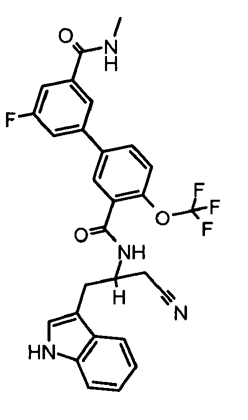 |
| | *N-[2-Cyano-1-(1H-indol-3-ylmethyl)-ethyl]-5-iodo-2-trifluoromethoxybenzamide* | | | |
| | *and* | | | |
| | *3-Fluoro-5-methylcarbamoyl-phenyl boronic acid* | | | |
| **38** | 3'-[2-Cyano-1-(1H-indol-3-ylmethyl)-ethylcarbamoyl]-3-methoxy-4'-trifluoromethoxy-biphenyl-4-carboxylic acid methyl ester; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1% (1.75'). Molecular peak (ESI, M+1): 553 Retention time: 4.14 min. | 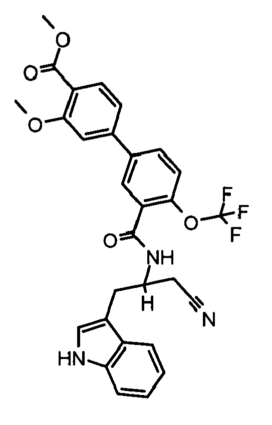 |
| | *N-[2-Cyano-1-(1H-indol-3-ylmethyl)-ethyl]-5-iodo-2-trifluoromethoxybenzamide* | | | |
| | *and* | | | |
| | *3-Methoxy-4-methyloxycarbonyl-phenyl boronic acid* | | | |
| **39** | 3'-Fluoro-5'-methoxy-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 512 Retention time: 4.42 min. | 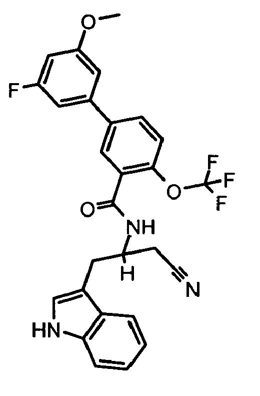 |
| | *N-[2-Cyano-1-(1H-indol-3-ylmethyl)-ethyl]-5-iodo-2-trifluoromethoxybenzamide* | | | |
| | *and* | | | |
| | *3-Fluoro-5-methoxyphenyl boronic acid* | | | |
| **40** | 4'-Acetylamino-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 522 Retention time: 3.80 min. | 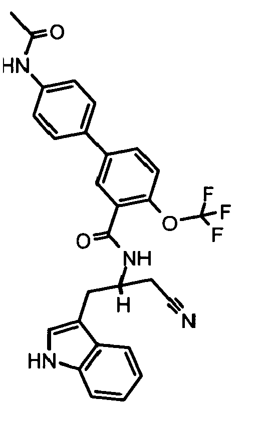 |
| | *N-[2-Cyano-1-(1H-indol-3-ylmethyl)-ethyl]-5-iodo-2-trifluoromethoxybenzamide* | | | |
| | *and* | | | |
| | *4-Acetamidophenylboronic acid* | | | |
| **41** | N-[2-Cyano-1-(1H-indol-3-ylmethyl)-ethyl]-5-quinolin-3-yl-2-trifluoromethoxybenzamide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 516 Retention time: 3.54 min. | 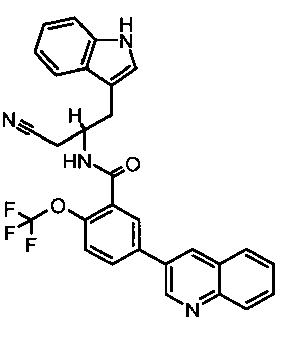 |
| | *N-[2-Cyano-1-(1H-indol-3-ylmethyl)-ethyl]-5-iodo-2-trifluoromethoxybenzamide* | | | |
| | *and* | | | |
| | *Quinoline-3-boronic acid* | | | |
| **42** | 3',5'-Dimethyl-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 493 Retention time: 4.72 min. | 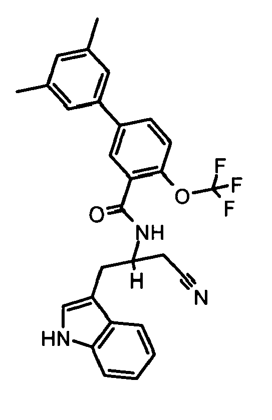 |
| | *N-[2-Cyano-1-(1H-indol-3-ylmethyl)-ethyl]-5-iodo-2-trifluoromethoxybenzamide* | | | |
| | *and* | | | |
| | *3,5-Dimethyl-phenyl boronic acid* | | | |
| **43** | 4-Trifluoromethoxybiphenyl-3,3'-dicarboxylic acid 3'-amide 3-{[2-cyano-1-(1 H-indol-3-ylmethyl)-ethyl]-amide}; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 507 Retention time: 3.65 min. | 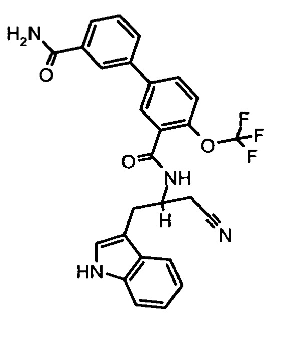 |
| | *N-[2-Cyano-1-(1H-indol-3-ylmethyl)-ethyl]-5-iodo-2-trifluoromethoxybenzamide* | | | |
| | *and* | | | |
| | *3-Carbamoyl-phenyl boronic acid* | | | |
| **44** | 3'-Cyano-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 489 Retention time: 4.20 min. | 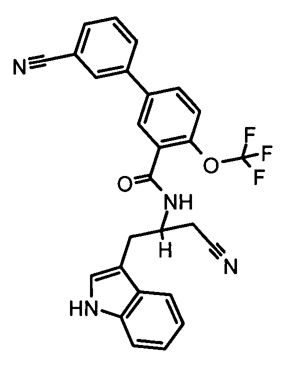 |
| | *N-[2-Cyano-1-(1H-indol-3-ylmethyl)-ethyl]-5-iodo-2-trifluoromethoxybenzamide* | | | |
| | *and* | | | |
| | *3-Cyano-phenyl boronic acid* | | | |
| **45** | 5-Benzo[1,3]dioxol-5-yl-N-[2-cyano-1-(1H-indol-3-ylmethyl)-ethyl]-2-trifluoromethoxy-benzamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1 % (1.75'). Molecular peak (ESI, M+1): 508 Retention time: 4.28 min. | 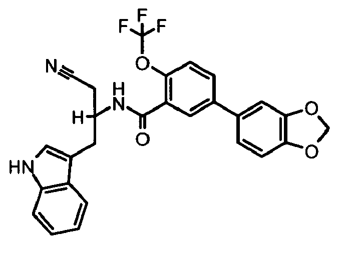 |
| | *N-[2-Cyano-1-(1H-indol-3-ylmethyl)-ethyl]-5-iodo-2-trifluoromethoxybenzamide* | | | |
| | *and* | | | |
| | *Benzo[1,3]dioxole-5-boronic acid* | | | |
| **46** | 3'-Acetylamino-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1 % (1.75'). Molecular peak (ESI, M+1): 522 Retention time: 3.79 min. | 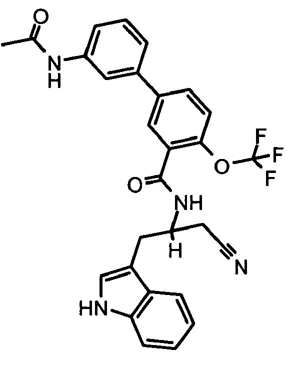 |
| | *N-[2-Cyano-1-(1H-indol-3-ylmethyl)-ethyl]-5-iodo-2-trifluoromethoxybenzamide* | | | |
| | *and* | | | |
| | *3-Acetamido-phenyl boronic acid* | | | |
| **47** | 3'-Methanesulfonylamino-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 558 Retention time: 3.93 min. | 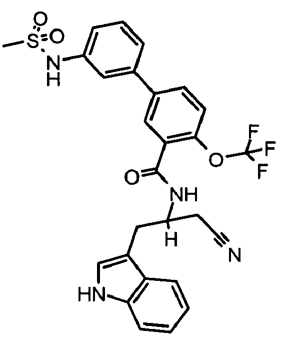 |
| | *N-[2-Cyano-1-(1H-indol-3-ylmethyl)-ethyl]-5-iodo-2-trifluoromethoxybenzamide* | | | |
| | *and* | | | |
| | *3-Methylsulfonylamino-phenyl boronic acid* | | | |
| **48** | 4'-Chloro-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 499 Retention time: 4.59 min. | 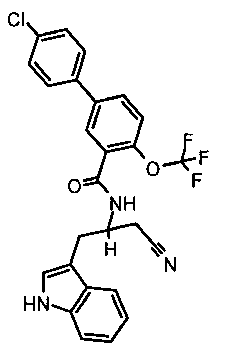 |
| | *N-[2-Cyano-1-(1H-indol-3-ylmethyl)-ethyl]-5-iodo-2-trifluoromethoxybenzamide* | | | |
| | *and* | | | |
| | *4-Chloro-phenyl boronic acid* | | | |
| **49** | 2'-Methyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 478 Retention time: 4.69 min. | 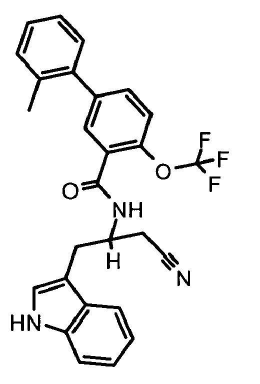 |
| | *N-[2-Cyano-1-(1H-indol-3-ylmethyl)-ethyl]-5-iodo-2-trifluoromethoxybenzamide* | | | |
| | *and* | | | |
| | *2-Methyl-phenyl boronic acid* | | | |
| **50** | 2'-Fluoro-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1 % (1.75'). Molecular peak (ESI, M+1): 482 Retention time: 4.34 min. I | 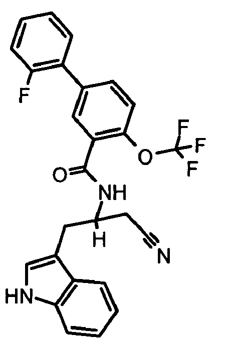 |
| | *N-[2-Cyano-1-(1H-indol-3-ylmethyl)-ethyl]-5-iodo-2-trifluoromethoxybenzamide* | | | |
| | *and* | | | |
| | *2-Fluoro-phenyl boronic acid* | | | |
| **51** | 4-Trifluoromethoxybiphenyl-3,3'-dicarboxylic acid 3-{[2-cyano-1-(1H-indol-3-ylmethyl)-ethyl]-amide} 3'-methylamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1 % (1.75'). Molecular peak (ESI, M+1): 522 Retention time: 3.76 min. | 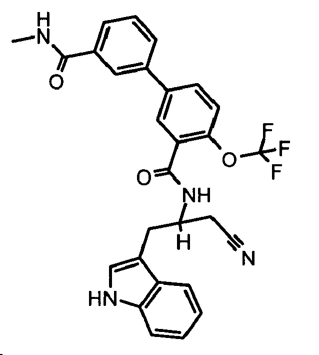 |
| | *N-[2-Cyano-1-(1H-indol-3-ylmethyl)-ethyl]-5-iodo-2-trifluoromethoxybenzamide* | | | |
| | *and* | | | |
| | *3-Methylcarbamoylphenyl boronic acid* | | | |
| **52** | N-[2-Cyano-1-(1H-indol-3-ylmethyl)-ethyl]-5-quinolin-6-yl-2-trifluoromethoxybenzamide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1 % (1.75'). Molecular peak (ESI, M+1): 516 Retention time: 3.31 min. | 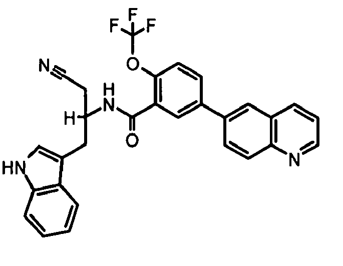 |
| | *N-[2-Cyano-1-(1H-indol-3-ylmethyl)-ethyl]-5-iodo-2-trifluoromethoxybenzamide* | | | |
| | *and* | | | |
| | *Quinoline-6-boronic acid* | | | |
| **53** | 3'-Hydroxy-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1 % (1.75'). Molecular peak (ESI, M+1): 480 Retention time: 3.90 min. | 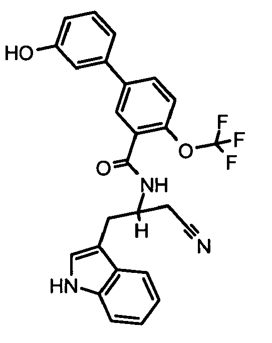 |
| | *N-[2-Cyano-1-(1H-indol-3-ylmethyl)-ethyl]-5-iodo-2-trifluoromethoxybenzamide* | | | |
| | *and* | | | |
| | *4-Hydroxy-phenyl boronic acid* | | | |
| **54** | 4-Trifluoromethoxy-4'-trifluoromethyl-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1 % (1.75'). Molecular peak (ESI, M+1): 532 Retention time: 4.62 min. | 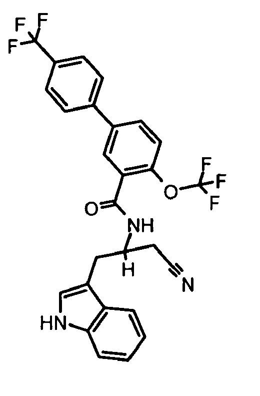 |
| | *N-[2-Cyano-1-(1H-indol-3-ylmethyl)-ethyl]-5-iodo-2-trifluoromethoxybenzamide* | | | |
| | *and* | | | |
| | *4-Trifluoromethylphenyl boronic acid* | | | |
| **55** | 3'-Hydroxymethyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 494 Retention time: 3.87 min. | 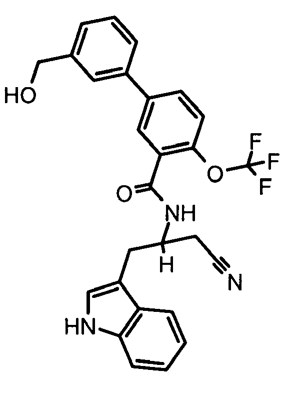 |
| | *N-[2-Cyano-1-(1H-indol-3-ylmethyl)-ethyl]-5-iodo-2-trifluoromethoxybenzamide* | | | |
| | *and* | | | |
| | *3-Hydroxymethylphenyl boronic acid* | | | |
| **56** | 4-Trifluoromethoxy-[1,1';3',1"]terphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1 % to 99% (5') to 99% (1') to 1% (0.25') to 1% (1.75'). Molecular peak (ESI, M+1): 541 Retention time: 4.84 min. | 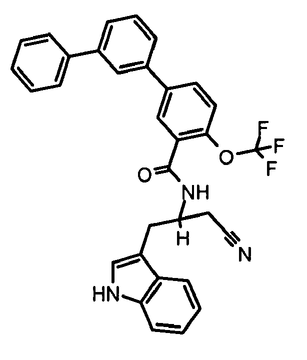 |
| | *N-[2-Cyano-1-(1H-indol-3-ylmethyl)-ethyl]-5-iodo-2-trifluoromethoxybenzamide* | | | |
| | *and* | | | |
| | *3-Biphenyl-boronic acid* | | | |
| **57** | 3'-Fluoro-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 482 Retention time: 4.36 min. | 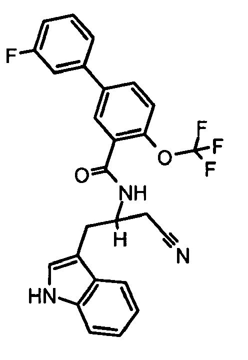 |
| | *N-[2-Cyano-1-(1H-indol-3-ylmethyl)-ethyl]-5-iodo-2-trifluoromethoxybenzamide* | | | |
| | *and* | | | |
| | *3-Fluorophenyl boronic acid* | | | |
| **58** | 3'-Acetyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 506 Retention time: 4.17 min. | 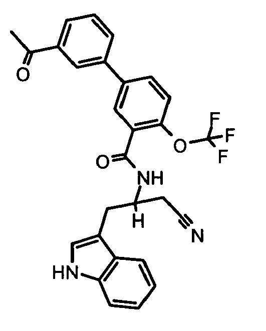 |
| | *N-[2-Cyano-1-(1H-indol-3-ylmethyl)-ethyl]-5-iodo-2-trifluoromethoxybenzamide* | | | |
| | *and* | | | |
| | *3-Acetylphenyl boronic acid* | | | |
| **59** | 3'-Amino-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 479 Retention time: 3.29 min. | 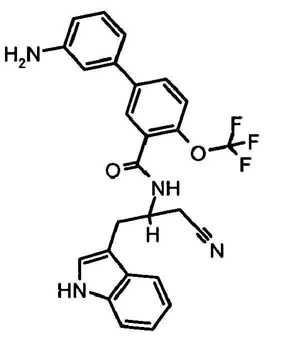 |
| | *N-[2-Cyano-1-(1H-indol-3-ylmethyl)-ethyl]-5-iodo-2-trifluoromethoxybenzamide* | | | |
| | *and* | | | |
| | *3-Aminophenyl boronic acid* | | | |
| **60** | 4'-Acetyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 506 Retention time: 4.09 min. | 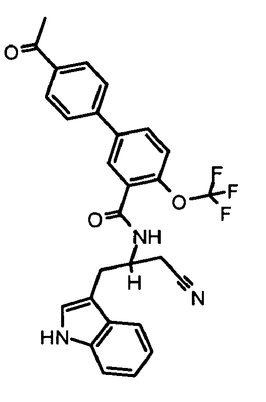 |
| | *N-[2-Cyano-1-(1H-indol-3-ylmethyl)-ethyl]-5-iodo-2-trifluoromethoxybenzamide* | | | |
| | *and* | | | |
| | *4-Acetylphenyl boronic acid* | | | |
| **61** | 4'-Methylsulfanyl-4-trifluoromethoxybiphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1 % (1.75'). Molecular peak (ESI, M+1): 511 Retention time: 4.48 min. | 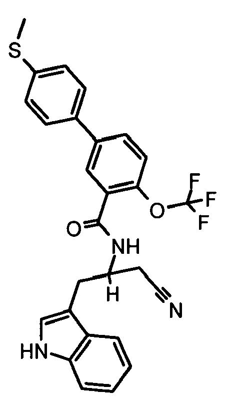 |
| | *N-[2-Cyano-1-(1H-indol-3-ylmethyl)-ethyl]-5-iodo-2-trifluoromethoxybenzamide* | | | |
| | *and* | | | |
| | *4-Methylsulfanylphenyl boronic acid* | | | |
| **62** | 4-Trifluoromethoxy-3'-trifluoromethylbiphenyl-3-carboxylic acid [2-cyano-1-(1H-indol-3-ylmethyl)-ethyl]-amide; | **12** | Column Purospher Star RP C184.6 x 125 5 µm; detection wavelength 214 nm; flow rate 2 ml/min; eluents A): 0.1 % TFA in H₂O, B): 0.1% TFA in ACN; gradients based on B: 1% to 99% (5') to 99% (1') to 1 % (0.25') to 1 % (1.75'). Molecular peak (ESI, M+1): 532 Retention time: 4.60 min. | 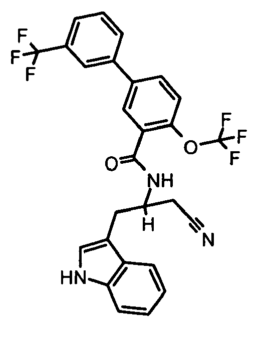 |
| | *N-[2-Cyano-1-(1H-indol-3-ylmethyl)-ethyl]-5-iodo-2-trifluoromethoxybenzamide* | | | |
| | *and* | | | |
| | *3- Trifluoromethylphenyl boronic acid* | | | |
| **63** | 4'-Hydroxy-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1% TFA in ACN; gradients based on B: 1% to 99% (5') to 99% (1') to 1% (0.25') to 1% (1.75'). Molecular peak (ESI, M+1): 480 Retention time: 3.92 min. | 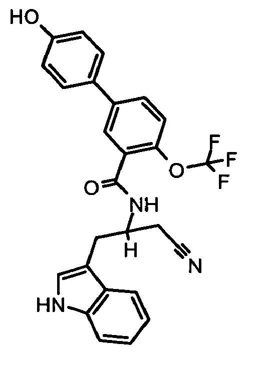 |
| | *N-[2-Cyano-1-(1H-indol-3-ylmethyl)-ethyl]-5-iodo-2-trifluoromethoxybenzamide* | | | |
| | *and* | | | |
| | *4-Hydroxyphenyl boronic acid* | | | |
| **64** | N-[2-Cyano-1-(1H-indol-3-ylmethyl)-ethyl]-5-naphthalen-1-yl-2-trifluoromethoxybenzamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1 % (0.25') to 1 % (1.75'). Molecular peak (ESI, M+1): 515 Retention time: 4.65 min. | 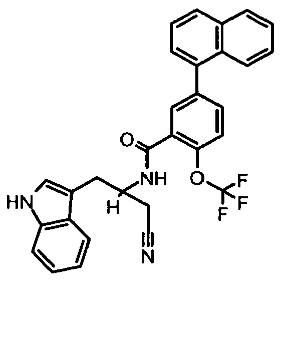 |
| | *N-[2-Cyano-1-(1H-indol-3-ylmethyl)-ethyl]-5-iodo-2-trifluoromethoxybenzamide* | | | |
| | *and* | | | |
| | *Naphthalen-1-boronic acid* | | | |
| **65** | 4'-Methyl-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 478 Retention time: 4.51 min. | 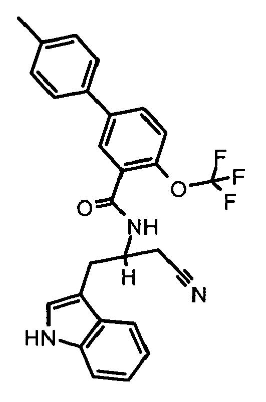 |
| | *N-[2-Cyano-1-(1H-indol-3-ylmethyl)-ethyl]-5-iodo-2-trifluoromethoxybenzamide* | | | |
| | *and* | | | |
| | *4-Methylphenyl boronic acid* | | | |
| **66** | 3'-Chloro-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1% (1.75'). Molecular peak (ESI, M+1): 499 Retention time: 4.51 min. | 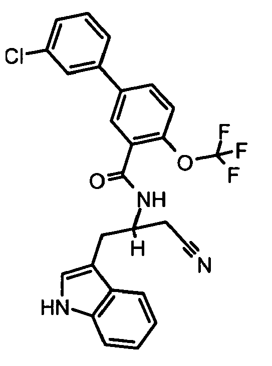 |
| | *N-[2-Cyano-1-(1H-indol-3-ylmethyl)-ethyl]-5-iodo-2-trifluoromethoxybenzamide* | | | |
| | *and* | | | |
| | *3-Chlorophenyl boronic acid* | | | |
| **67** | N-[2-Cyano-1-(1H-indol-3-ylmethyl)-ethyl]-5-naphthalen-2-yl-2-trifluoromethoxybenzamide; | **12** | Column Purospher Star RP C18 4.6x125 5µm; detection wavelength 214 nm; flow rate 2 ml/min; eluents A): 0.1% TFA in H₂O, B): 0.1 % TFA in ACN; gradients based on B: 1% to 99% (5') to 99% (1') to 1% (0.25') to 1 % (1.75'). Molecular peak (ESI, M+1): 515 Retention time: 4.66 min. | 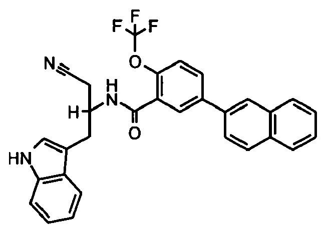 |
| | *N-[2-Cyano-1-(1H-indol-3-ylmethyl)-ethyl]-5-iodo-2-trifluoromethoxybenzamide* | | | |
| | *and* | | | |
| | *Naphthalen-2-boronic acid* | | | |
| **68** | 3'-Methoxy-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 494 Retention time: 4.34 min. | 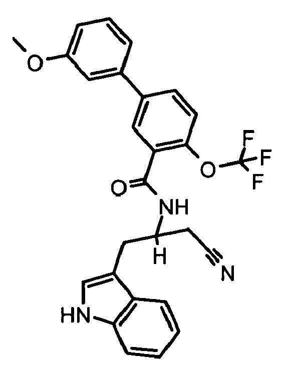 |
| | *N-[2-Cyano-1-(1H-indol-3-ylmethyl)-ethyl]-5-iodo-2-trifluoromethoxybenzamide* | | | |
| | *and* | | | |
| | *3-Methoxyphenyl boronic acid* | | | |
| **69** | 4'-Methoxy-4-trifluoromethoxy-biphenyl-3-carboxylic acid [2-cyano-1-(1H-indol-3-ylmethyl)-ethyl]-amide; | **12** | Column Purospher Star RP C18 4.6x125 5µm; detection wavelength 214 nm; flow rate 2 ml/min; eluents A): 0.1 % TFA in H₂O, B): 0.1 % TFA in ACN; gradients based on B: 1% to 99% (5') to 99% (1') to 1% (0.25') to 1% (1.75'). Molecular peak (ESI, M+1): 494 Retention time: 4.30 min. | 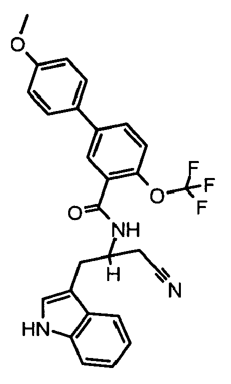 |
| | *N-[2-Cyano-1-(1H-indol-3-ylmethyl)-ethyl]-5-iodo-2-trifluoromethoxybenzamide* | | | |
| | *and* | | | |
| | *4-Methoxyphenyl boronic acid* | | | |
| **70** | N-[2-Cyano-1-(1H-indol-3-ylmethyl)-ethyl]-5-thiophen-3-yl-2-trifluoromethoxybenzamide; | **12** | Column Purospher Star RP C18 4.6x125 5µm; detection wavelength 214 nm; flow rate 2 ml/min; eluents A): 0.1 % TFA in H₂O, B): 0.1% TFA in ACN; gradients based on B: 1% to 99% (5') to 99% (1') to 1% (0.25') to 1% (1.75'). Molecular peak (ESI, M+1): 470 Retention time: 4.29 min. | 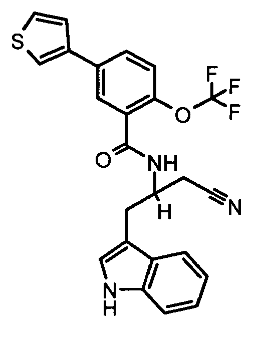 |
| | *N-[2-Cyano-1-(1H-indol-3-ylmethyl)-ethyl]-5-iodo-2-trifluoromethoxybenzamide* | | | |
| | *and* | | | |
| | *Thiopene-3-boronic acid* | | | |

The present invention further relates to the aryl halides of the formulae XIII, XIV or XVI as intermediates of the process according to the invention for preparing the compounds according to the invention, namely:
N-[2-Cyano-1-(1H-indol-3-ylmethyl)-ethyl]-5-iodo-2-isopropoxy-benzamide;
N-[2-Cyano-1-(1H-indol-3-ylmethyl)-ethyl]-5-bromo-2-trifluoromethoxy-benzamide.

## Claims

1. Compounds of the formula I 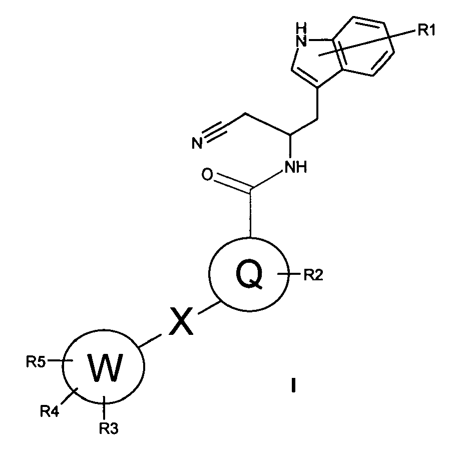 in which
R1 is hydrogen, fluorine or C₁-C₆-alkyl; where the hydrocarbon chains therein may optionally be substi- tuted one or more times by fluorine;
R2 is hydrogen, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃- C₆-alkenylene, hydroxy-C₃-C₆-alkynylene, C₁-C₆-alkyloxy, C₁₋C₆- alkyloxy-C₁-C₆-alkylene, C₃-C₇-cycloalkyloxy, C₃-C₇-cycloalkyl-C₁-C₆- alkylenoxy, C₃-C₇-cycloalkyloxy-C₁-C₆-alkylene, C₁-C₆-alkyloxy-C₃-C₆- alkenylene, C₁-C₆-alkyloxy-C₃-C₆-alkynylene, C₁-C₆-alkylamino-C₁-C₆- alkylene, di(C₁-C₆-alkyl)amino-C₁-C₆-alkylene; where the hydrocarbon chains therein may optionally be substituted one or more times by fluorine, cyano or hydroxy;
R3, R4, R5 may be independently of one another hydrogen, hydroxy, halogen, nitro, amino, cyano, phenyl, C₁-C₆-alkyl, C₂-C₆-alkenyl or C₂-C₆-alkynyl, C₃- C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the radicals: 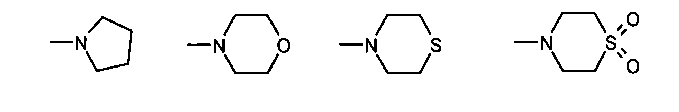 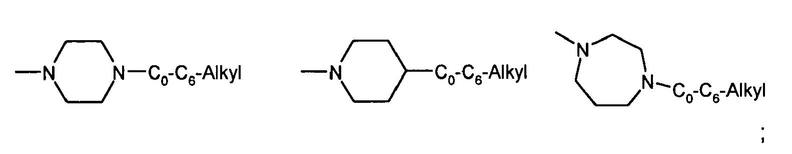 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl,
C₁-C₆-alkylcarbonyl, C₃-C₇-cycloalkylcarbonyl,
carboxy, carboxamido [-C(O)NH₂], C₁-C₆-alkyloxycarbonyl,
C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido,
-N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
(C₁-C₆-alkyl)oxy-C₂-C₆-alkylene-aminocarbonyl, carboxy-C₁-C₆-alkylene-carbonylamido, (C₁-C₆-alkyl)sulfonylmido,
or the radicals: 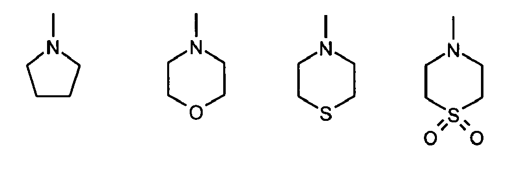 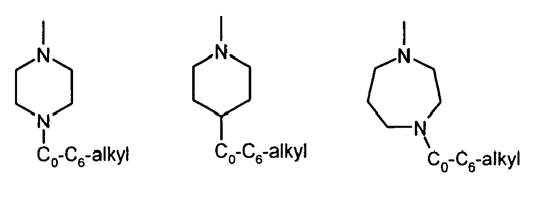 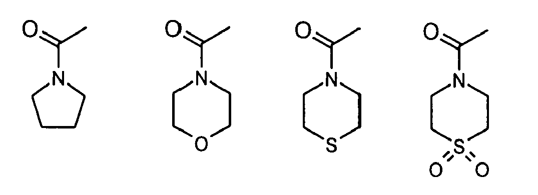 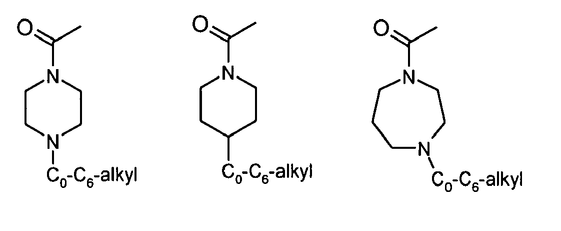 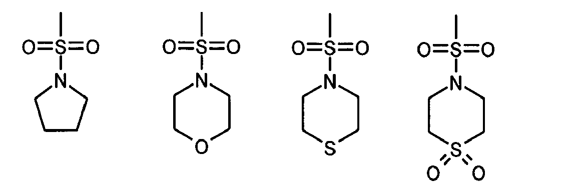 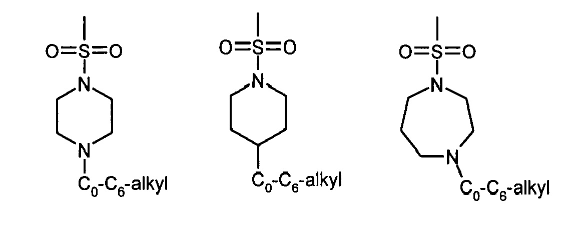 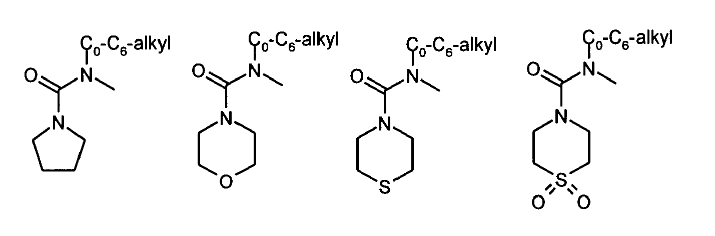 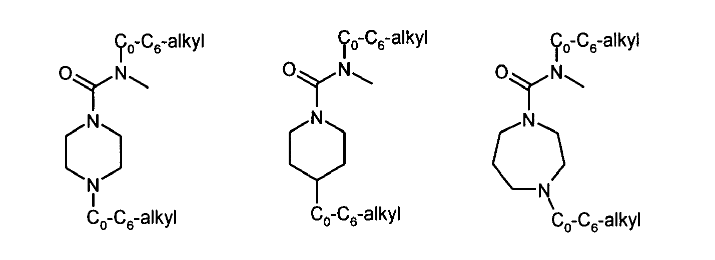 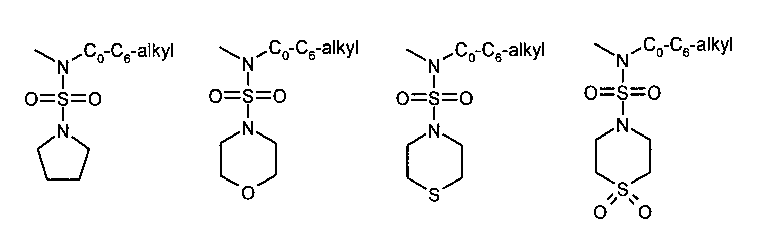 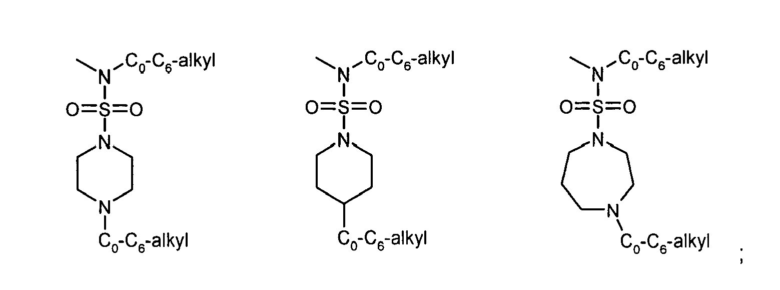
R3 and R4 may together form heterocycloalkyl, cycloalkyl;
Q is an aryl or heteroaryl group or the group 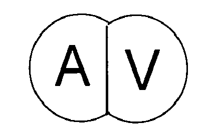 in which
A is a monocyclic aryl or a monocyclic heteroaryl group;
V is a cycloalkylen, cycloalkenylen, heterocycloalkylen or heterocycloalkenylen group;
X is a bond or an ethinylen group;
W is an aryl or heteroaryl group;
where
R1 substitutes one or more positions of the aryl or heteroaryl ring in the indole residue;
R2 substitutes one or more positions of the aryl or heteroaryl ring in the radical Q and or the radical V.

2. Compounds according to claim 1, namely cyanomethyl substituted N-acyl tryptamines of the formula II 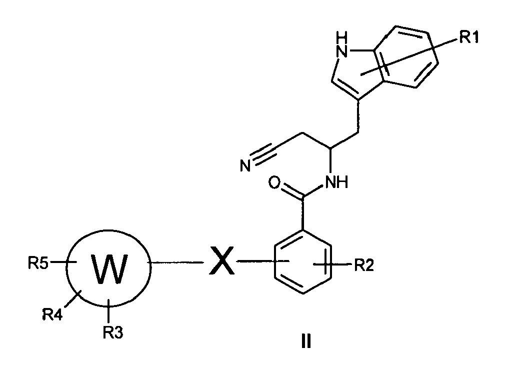 in which the radicals R1 to R5, X and W have the same meaning as defined in claim 1.

3. Compounds according to claim 1, namely cyanomethyl substituted N-acyl tryptamines of the formula III 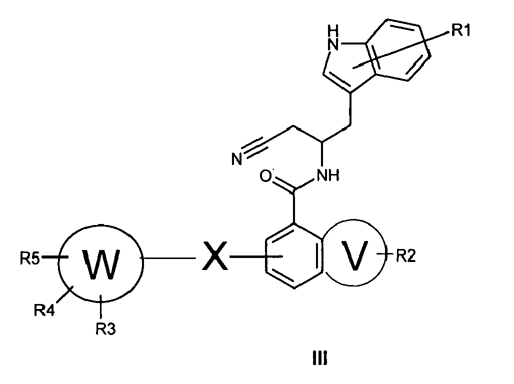 in which the radicals R1 to R5, X, W and V have the same meaning as defined in claim 1.

4. Compounds according to claim 1 or 2, namely cyanomethyl substituted N-acyl tryptamines of the formula IV 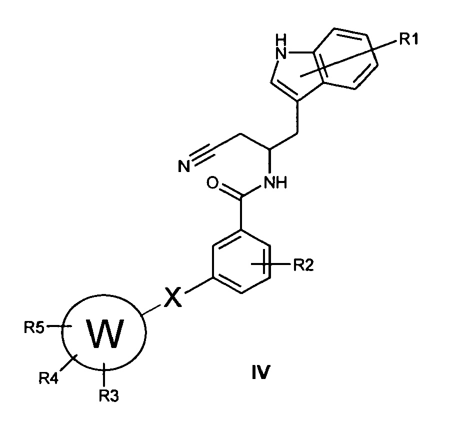 in which the radicals R1 to R5, X and W have the same meaning as defined in claim 1.

5. Compounds according to claim 1 or 3, namely cyanomethyl substituted N-acyl tryptamines of the formula V 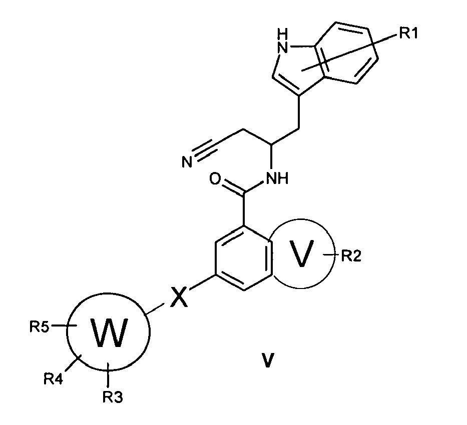 in which the radicals R1 to R5, X, W and V have the same meaning as defined in claim 1.

6. Compounds according to any of the preceding claims, namely
| | |
|---|---|
| **1** | 5'-Fluoro-4-isopropoxy-biphenyl-3,3'-dicarboxylic acid 3-{[2-cyano-1-(1H-indol-3-yl-methyl)-ethyl]-amide} 3'-methylamide; |
| **2** | N-[2-Cyano-1-(1H-indol-3-ylmethyl)-ethyl]-5-(4-methylcarbamoyl-phenylethynyl)-2-trifluoromethoxy-benzamide; |
| **3** | 3'-Chloro-4-isopropoxy-biphenyl-3,4'-dicarboxylic acid 3-{[2-cyano-1-(1H-indol-3-yl-methyl)-ethyl]-amide} 4'-methylamide; |
| **4** | 5-(4-Carbamoyl-2-methylphenylethynyl)-N-[2-cyano-1-(1H-indol-3-ylmethyl)-ethyl]-2-trifluoromethoxy-benzamide; |
| **5** | 3'-Chloro-4-isopropoxy-biphenyl-3,4'-dicarboxylic acid 4'-amide 3-{[2-cyano-1-(1H-indol-3-ylmethyl)-ethyl]-amide}; |
| **6** | 3'-Chloro-4-isopropoxy-4'-(morpholine-4-carbonyl)-biphenyl-3-carboxylic acid [2-cyano-1-(1H-indol-3-ylmethyl)-ethyl]-amide; |
| **7** | 4-Isopropoxy-3',4'-dimethoxy-biphenyl-3-carboxylic acid [2-cyano-1-(1H-indol-3-yl-methyl)-ethyl]-amide; |
| **8** | 5-(4-Carbamoyl-phenylethynyl)-N-[2-cyano-1-(1H-indol-3-ylmethyl)-ethyl]-2-isoprop-oxy-benzamide |
| **9** | N-[2-Cyano-1-(1H-indol-3-ylmethyl)-ethyl]-2-isopropoxy-5-(4-methylcarbamoyl-phenyl-ethynyl)-benzamide; |
| **10** | N-[2-Cyano-1-(1H-indol-3-ylmethyl)-ethyl]-2-isopropoxy-5-(3-methylcarbamoyl-phenyl-ethynyl)-benzamide; |
| **11** | N-[2-Cyano-1-(1H-indol-3-ylmethyl)-ethyl]-2-isopropoxy-5-(2-methylcarbamoyl-phenyl-ethynyl)-benzamide; |
| **12** | 4'-Morpholin-4-yl-4-trifluoromethoxy-biphenyl-3-carboxylic acid [2-cyano-1-(1H-indol-3-ylmethyl)-ethyl]-amide |
| **13** | 4'-Methanesulfonylamino-methyl)-4-trifluoromethoxy-biphenyl-3-carboxylic acid [2-cyano-1-(1H-indol-3-ylmethyl)-ethyl]-amide; |
| **14** | N-{3'-[2-Cyano-1-(1H-indol-3-ylmethyl)-ethylcarbamoyl]-4'-trifluoromethoxy-biphenyl-3-yl}-succinamic acid; |
| **15** | 4'-(Pyrrolidine-1-carbonyl)-4-trifluoromethoxy-biphenyl-3-carboxylic acid [2-cyano-1-(1 H-indol-3-ylmethyl)-ethyl]-amide; |
| **16** | N-[2-Cyano-1-(1H-indol-3-ylmethyl)-ethyl]-5-(1-methyl-1H-indol-5-yl)-2-trifluoromethoxy-benzamide; |
| **17** | 4-Trifluoromethoxy-biphenyl-3,4'-dicarboxylic acid 3-{[2-cyano-1-(1H-indol-3-yl-methyl)-ethyl]-amide} 4'-[(2-methoxy-ethyl)-amide]; |
| **18** | 3'-(Pyrrolidine-1-carbonyl)-4-trifluoromethoxy-biphenyl-3-carboxylic acid [2-cyano-1-(1 H-indol-3-ylmethyl)-ethyl]-amide; |
| **19** | 4-Trifluoromethoxy-biphenyl-3,3'-dicarboxylic acid 3-{[2-cyano-1-(1H-indol-3-yl-methyl)-ethyl]-amide} 3'-ethylamide; |
| **20** | 4-Trifluoromethoxy-biphenyl-3,3'-dicarboxylic acid 3-{[2-cyano-1-(1H-indol-3-yl-methyl)-ethyl]-amide} 3'-(isobutyl-amide); |
| **21** | 4-Trifluoromethoxy-biphenyl-3,3'-dicarboxylic acid 3-{[2-cyano-1-(1H-indol-3-yl-methyl)-ethyl]-amide} 3'-cyclopentylamide; |
| **22** | 3'-(Methanesulfonylamino-methyl)-4-trifluoromethoxy-biphenyl-3-carboxylic acid [2-cyano-1-(1H-indol-3-ylmethyl)-ethyl]-amide; |
| **23** | 4'-(Acetylamino-methyl)-4-trifluoromethoxy-biphenyl-3-carboxylic acid [2-cyano-1-(1H-indol-3-ylmethyl)-ethyl]-amide; |
| **24** | 3'-(Acetylamino-methyl)-4-trifluoromethoxy-biphenyl-3-carboxylic acid [2-cyano-1-(1H-indol-3-ylmethyl)-ethyl]-amide; |
| **25** | 4'-Methylsulfamoyl-4-trifluoromethoxy-biphenyl-3-carboxylic acid [2-cyano-1-(1H-indol-3-ylmethyl)-ethyl]-amide; |
| **26** | 3-Chloro-3'-[2-cyano-1-(1H-indol-3-ylmethyl)-ethylcarbamoyl]-4'-trifluoromethoxy-biphenyl-4-carboxylic acid methyl ester; |
| **27** | 4'-Ethylsulfamoyl-4-trifluoromethoxy-biphenyl-3-carboxylic acid [2-cyano-1-(1H-indol-3-ylmethyl)-ethyl]-amide; |
| **28** | 3'-Chloro-4-trifluoromethoxy-biphenyl-3,4'-dicarboxylic acid 3-{[2-cyano-1-(1H-indol-3-ylmethyl)-ethyl]-amide} 4'-isopropylamide; |
| **29** | 3'-Chloro-4-trifluoromethoxy-biphenyl-3,4'-dicarboxylic acid 3-{[2-cyano-1-(1H-indol-3-ylmethyl)-ethyl]-amide} 4'-cyclopropylamide; |
| **30** | 3'-Chloro-4-trifluoromethoxy-biphenyl-3,4'-dicarboxylic acid 4'-tert-butylamide 3-{[2-cyano-1-(1H-indol-3-ylmethyl)-ethyl]-amide}; |
| **31** | 3'-Chloro-4-trifluoromethoxy-biphenyl-3,4'-dicarboxylic acid 3-{[2-cyano-1-(1H-indol-3-ylmethyl)-ethyl]-amide} 4'-dimethylamide; |
| **32** | 3'-Chloro-4-trifluoromethoxy-biphenyl-3,4'-dicarboxylic acid 3-{[2-cyano-1-(1H-indol-3-ylmethyl)-ethyl]-amide} 4'-diethylamide; |
| **33** | 3'-Chloro-4'-(morpholine-4-carbonyl)-4-trifluoromethoxy-biphenyl-3-carboxylic acid [2-cyano-1-(1H-indol-3-ylmethyl)-ethyl]-amide; |
| **34** | 3'-Chloro-4'-(piperidine-1-carbonyl)-4-trifluoromethoxy-biphenyl-3-carboxylic acid [2-cyano-1-(1H-indol-3-ylmethyl)-ethyl]-amide; |
| **35** | 3'-Chloro-4'-(pyrrolidine-1-carbonyl)-4-trifluoromethoxy-biphenyl-3-carboxylic acid [2-cyano-1-(1H-indol-3-ylmethyl)-ethyl]-amide; |
| **36** | 5'-Ethoxy-3'-fluoro-4-trifluoromethoxy-biphenyl-3-carboxylic acid [2-cyano-1-(1H-indol-3-ylmethyl)-ethyl]-amide; |
| **37** | 5'-Fluoro-4-trifluoromethoxy-biphenyl-3,3'-dicarboxylic acid 3-{[2-cyano-1-(1H-indol-3-ylmethyl)-ethyl]-amide} 3'-methylamide; |
| **38** | 3'-[2-Cyano-1-(1H-indol-3-ylmethyl)-ethylcarbamoyl]-3-methoxy-4'-trifluoromethoxy-biphenyl-4-carboxylic acid methyl ester; |
| **39** | 3'-Fluoro-5'-methoxy-4-trifluoromethoxy-biphenyl-3-carboxylic acid [2-cyano-1-(1H-indol-3-ylmethyl)-ethyl]-amide; |
| **40** | 4'-Acetylamino-4-trifluoromethoxy-biphenyl-3-carboxylic acid [2-cyano-1-(1H-indol-3-ylmethyl)-ethyl]-amide; |
| **41** | N-[2-Cyano-1-(1H-indol-3-ylmethyl)-ethyl]-5-quinolin-3-yl-2-trifluoromethoxy-benzamide; |
| **42** | 3',5'-Dimethyl-4-trifluoromethoxy-biphenyl-3-carboxylic acid [2-cyano-1-(1H-indol-3-ylmethyl)-ethyl]-amide; |
| **43** | 4-Trifluoromethoxy-biphenyl-3,3'-dicarboxylic acid 3'-amide 3-{[2-cyano-1-(1H-indol-3-ylmethyl)-ethyl]-amide}; |
| **44** | 3'-Cyano-4-trifluoromethoxy-biphenyl-3-carboxylic acid [2-cyano-1-(1H-indol-3-yl-methyl)-ethyl]-amide; |
| **45** | 5-Benzo[1,3]dioxol-5-yl-N-[2-cyano-1-(1H-indol-3-ylmethyl)-ethyl]-2-trifluoromethoxy-benzamide; |
| **46** | 3'-Acetylamino-4-trifluoromethoxy-biphenyl-3-carboxylic acid [2-cyano-1-(1H-indol-3-ylmethyl)-ethyl]-amide; |
| **47** | 3'-Methanesulfonylamino-4-trifluoromethoxy-biphenyl-3-carboxylic acid [2-cyano-1-(1 H-indol-3-ylmethyl)-ethyl]-amide; |
| **48** | 4'-Chloro-4-trifluoromethoxy-biphenyl-3-carboxylic acid [2-cyano-1-(1H-indol-3-yl-methyl)-ethyl]-amide; |
| **49** | 2'-Methyl-4-trifluoromethoxy-biphenyl-3-carboxylic acid [2-cyano-1-(1H-indol-3-yl-methyl)-ethyl]-amide; |
| **50** | 2'-Fluoro-4-trifluoromethoxy-biphenyl-3-carboxylic acid [2-cyano-1-(1H-indol-3-yl-methyl)-ethyl]-amide; |
| **51** | 4-Trifluoromethoxy-biphenyl-3,3'-dicarboxylic acid 3-{[2-cyano-1-(1H-indol-3-yl-methyl)-ethyl]-amide} 3'-methylamide; |
| **52** | N-[2-Cyano-1-(1H-indol-3-ylmethyl)-ethyl]-5-quinolin-6-yl-2-trifluoromethoxy-benzamide; |
| **53** | 3'-Hydroxy-4-trifluoromethoxy-biphenyl-3-carboxylic acid [2-cyano-1-(1H-indol-3-ylmethyl)-ethyl]-amide; |
| **54** | 4-Trifluoromethoxy-4'-trifluoromethyl-biphenyl-3-carboxylic acid [2-cyano-1-(H-indol-3-ylmethyl)-ethyl]-amide; |
| **55** | 3'-Hydroxymethyl-4-trifluoromethoxy-biphenyl-3-carboxylic acid [2-cyano-1-(1H-indol-3-ylmethyl)-ethyl]-amide; |
| **56** | 4-Trifluoromethoxy-[1,1';3',1"]terphenyl-3-carboxylic acid [2-cyano-1-(1H-indol-3-yl-methyl)-ethyl]-amide; |
| **57** | 3'-Fluoro-4-trifluoromethoxy-biphenyl-3-carboxylic acid [2-cyano-1-(1H-indol-3-yl-methyl)-ethyl]-amide; |
| **58** | 3'-Acetyl-4-trifluoromethoxy-biphenyl-3-carboxylic acid [2-cyano-1-(1H-indol-3-yl-methyl)-ethyl]-amide; |
| **59** | 3'-Amino-4-trifluoromethoxy-biphenyl-3-carboxylic acid [2-cyano-1-(1H-indol-3-yl-methyl)-ethyl]-amide; |
| **60** | 4'-Acetyl-4-trifluoromethoxy-biphenyl-3-carboxylic acid [2-cyano-1-(1H-indol-3-yl-methyl)-ethyl]-amide; |
| **61** | 4'-Methylsulfanyl-4-trifluoromethoxy-biphenyl-3-carboxylic acid [2-cyano-1-(1H-indol-3-ylmethyl)-ethyl]-amide; |
| **62** | 4-Trifluoromethoxy-3'-trifluoromethyl-biphenyl-3-carboxylic acid [2-cyano-1-(1H-indol-3-ylmethyl)-ethyl]-amide; |
| **63** | 4'-Hydroxy-4-trifluoromethoxy-biphenyl-3-carboxylic acid [2-cyano-1-(1H-indol-3-yl-methyl)-ethyl]-amide; |
| **64** | N-[2-Cyano-1-(1H-indol-3-ylmethyl)-ethyl]-5-naphthalen-1-yl-2-trifluoromethoxy-benzamide; |
| **65** | 4'-Methyl-4-trifluoromethoxy-biphenyl-3-carboxylic acid [2-cyano-1-(1H-indol-3-yl-methyl)-ethyl]-amide; |
| **66** | 3'-Chloro-4-trifluoromethoxy-biphenyl-3-carboxylic acid [2-cyano-1-(1H-indol-3-yl-methyl)-ethyl]-amide; |
| **67** | N-[2-Cyano-1-(1H-indol-3-ylmethyl)-ethyl]-5-naphthalen-2-yl-2-trifluoromethoxy-benzamide; |
| **68** | 3'-Methoxy-4-trifluoromethoxy-biphenyl-3-carboxylic acid [2-cyano-1-(1H-indol-3-yl-methyl)-ethyl]-amide; |
| **69** | 4'-Methoxy-4-trifluoromethoxy-biphenyl-3-carboxylic acid [2-cyano-1-(1H-indol-3-yl-methyl)-ethyl]-amide; |
| **70** | N-[2-Cyano-1-(1H-indol-3-ylmethyl)-ethyl]-5-thiophen-3-yl-2-trifluoromethoxy-benzamide. |

7. Process for preparing compounds of the formula I of claim 1, wherein an amine of the formula VI 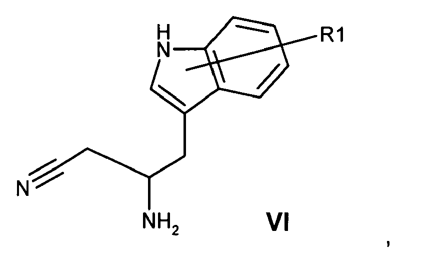 in which the radical R1 has the same meaning as defined in claim 1,
is coupled with a carboxylic acid of the formula VII 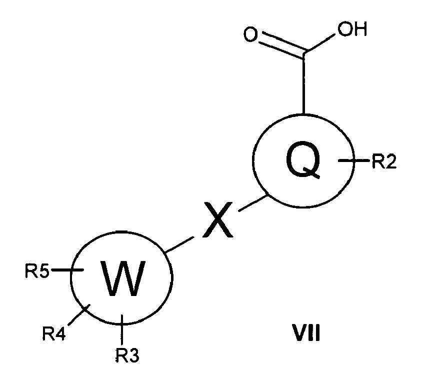 in which R2, R3, R4, R5, Q, X and W have the same meaning as defined in claim 1, in an amide forming reaction comprising
a) conversion of said carboxylic acids into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said amino alcohol.

8. Process according to claim 7 for preparing compounds of the formula II of claim 2, wherein an amine of the formula VI is coupled with a carboxylic acid of the formula VIII 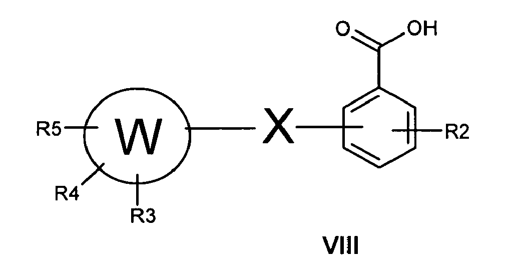 in which R2, R3, R4, R5, X and W have the same meaning as defined in claim 2.

9. Process according to claim 7 for preparing compounds of the formula III of claim 3, wherein an amine of the formula VI is coupled with a carboxylic acid of the formula IX 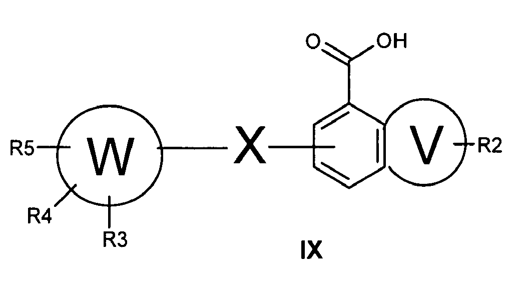 in which R2, R3, R4, R5, X, V and W have the same meaning as defined in claim 3.

10. Process according to claim 7 for preparing compounds of the formula IV of claim 4, wherein an amine of the formula VI is coupled with a carboxylic acid of the formula X 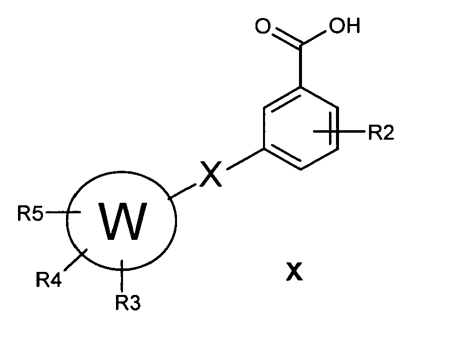 in which R2, R3, R4, R5, X and W have the same meaning as defined in claim 4.

11. Process according to claim 7 for preparing compounds of the formula V of claim 5, wherein an amine of the formula VI is coupled with a carboxylic acid of the formula XI 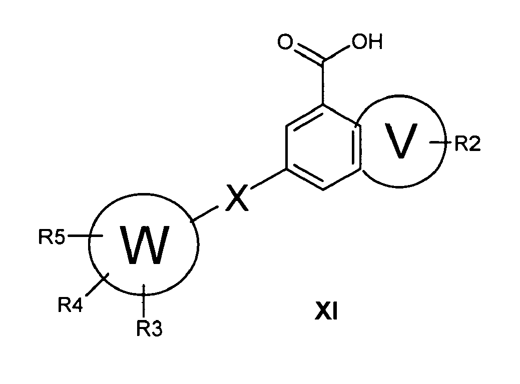 in which R2, R3, R4, R5, X, V and W have the same meaning as defined in claim 5.

12. Process for preparing compounds of the formula I of claim 1, wherein the building block of the formula XII 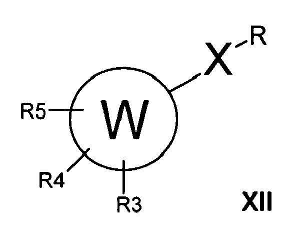 in which R3, R4, R5, W and X have the same meaning as defined in claim 1 and
R is a -B(OH)_{2,} -C≡C-H, -Zn-Hal or -Sn(alkyl)₃) group,
is coupled in a metal catalyzed cross-coupling reaction
with an aryl halide of the formula XIII 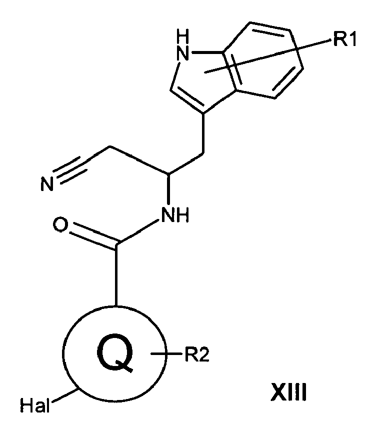 in which R1, R2 and Q have the same meaning as defined in claim 1, and
Hal stands for a chlorine, bromine or iodine.

13. Process according to claim 12 for preparing compounds of the formula II of claim 2, wherein the building block of the formula XII is coupled with an aryl halide of the formula XIV 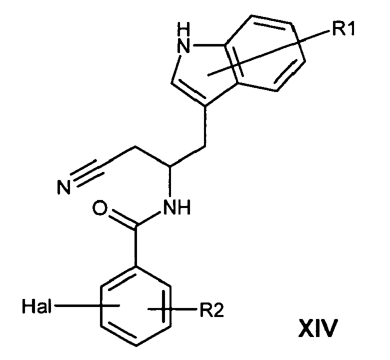 in which R1 and R2 have the same meaning as defined in claim 1, and
Hal stands for a chlorine, bromine or iodine.

14. Process according to claim 12 for preparing compounds of the formula III of claim 3, wherein the building block of the formula XII is coupled with an aryl halide of the formula XV 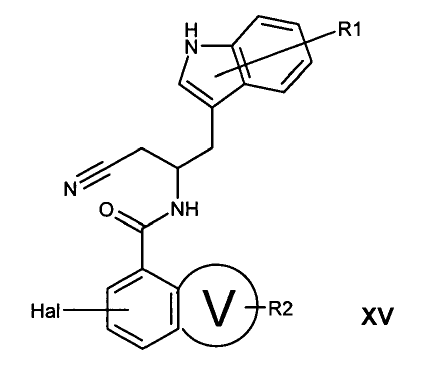 in which R1, R2 and V have the same meaning as defined in claim 1, and
Hal stands for a chlorine, bromine or iodine.

15. Process according to claim 12 for preparing compounds of the formula IV of claim 4, wherein the building block of the formula XII is coupled with an aryl halide of the formula XVI 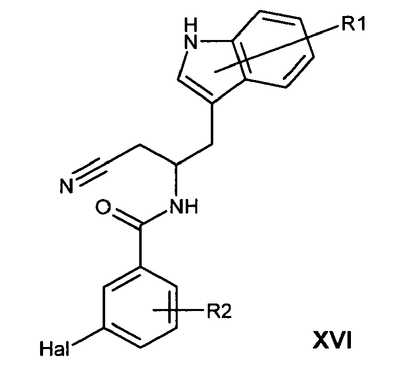 in which R1 and R2 have the same meaning as defined in claim 1, and
Hal stands for a chlorine, bromine or iodine.

16. Process according to claim 12 for preparing compounds of the formula V of claim 5, wherein the building block of the formula XII is coupled with an aryl halide of the formula XVII 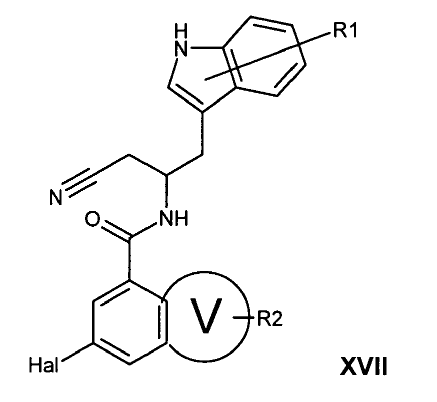 in which R1, R2 and V have the same meaning as defined in claim 1,
and
Hal stands for a chlorine, bromine or iodine.

17. Aryl halides as intermediates in a process according to any of claims 12 to 16, namely:
N-[2-Cyano-1-(1H-indol-3-ylmethyl)-ethyl]-5-iodo-2-isopropoxy-benzamide;
N-[2-Cyano-1-(1H-indol-3-ylmethyl)-ethyl]-5-bromo-2-trifluoromethoxy-benzamide.

18. Pharmaceutical compositions comprising at least one of the compounds according to any of claims 1 to 6 with pharmaceutically suitable excipients and/or carriers.

19. Use of the compounds of the general formula I according to any of claims 1 to 6 for the fertility control in men or in women.

20. Process for producing medicaments comprising at least one of the compounds of the general formula I according to any of claims 1 to 6 for the prevention and/or treatment of osteoporosis.
